# EUROPEAN PATENT APPLICATION

(11) **EP 3 466 954 A1**
(43) Date of publication of application: **10.04.2019**
(21) Application number: 17194742.7
(22) Date of filing: 04.10.2017
(51) Int. Cl.: C07D 491/06

(54) **FUSED PHENYLQUINAZOLINES BRIDGED WITH A HETEROATOM**

(71) Applicant: Idemitsu Kosan Co., Ltd., Chiyoda-ku, Tokyo 100-8321 (JP)
(72) Inventor: Nishimae, Yuichi, 4058 Basel (CH); Chebotareva, Natalia, 68220 Hagenthal le Bas (FR)
(74) Representative: Hollah, Dorothee

(57) **Abstract**

Fused phenylquinazolines bridged with a heteroatom and a process for their preparation, an electronic device comprising at least one of these fused phenylquinazolines, an emitting layer, preferably present in an electronic device, comprising at least one of said fused phenylquinazolines and use of said compounds in an electronic device as a host material, a charge transporting material, charge and/or exciton blocking material, preferably as a host material or an electron transporting material. The present invention further relates to specifically substituted phenylquinazolines bridged with a heteroatom, to a process for their preparation, to an electronic device comprising at least one of these specifically substituted phenylquinazolines, to an emitting layer, preferably present in an electronic device, comprising at least one of said specifically substituted phenylquinazolines and to the use of said specifically substituted compounds.

## Description

The present invention relates to fused phenylquinazolines bridged with a heteroatom of the general formula (I) and to a process for their preparation, to an electronic device comprising at least one of these compounds, to an emitting layer, preferably present in an electronic device, comprising at least one compound of general formula (I) and to the use of a compound according to general formula (I) in an electronic device as a host material, a charge transporting material, charge and/or exciton blocking material, preferably as a host material or an electron transporting material. The present invention further relates to specifically substituted phenylquinazolines bridged with a heteroatom of the general formula (I*), to a process for their preparation, to an electronic device comprising at least one of these compounds, to an emitting layer, preferably present in an electronic device, comprising at least one compound of general formula (I*) and to the use of a compound according to general formula (I*).

Quinazolines and their use in electronic devices are known from the related art.

EP 3 168 221 A1 relates to compounds of formula 1 and organic optoelectronic devices comprising the same:

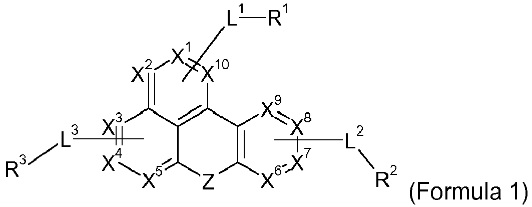

In all exemplified compounds Z is N-L⁴-R^{b}: Fused phenylquinazolines are not mentioned in EP 3 168 221 A1.

KR 20160056521 concerns compounds of formulae (1) and (2), and light emitting elements comprising said compounds as hosts.

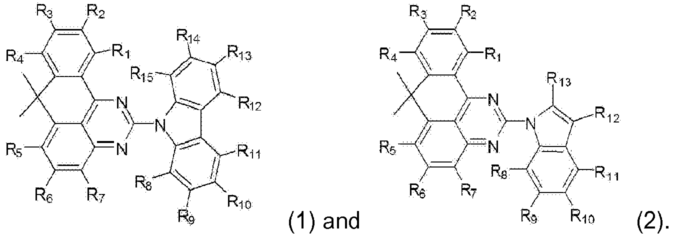

The residues in said compounds may be fused, for example as shown below: 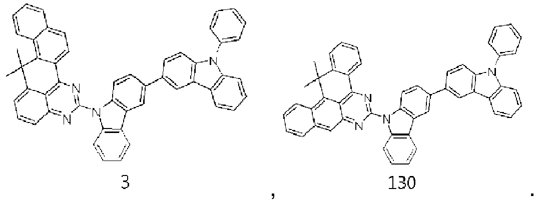 However, in the compounds according to KR 20160056521 no heteroatom other than N is present.

WO2017/109727 A1 concerns compounds of general formula (I) 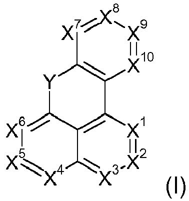 and their use in electronic devices. Under a number of none-fused phenylquinazolines, two fused phenylquinazolines are mentioned:

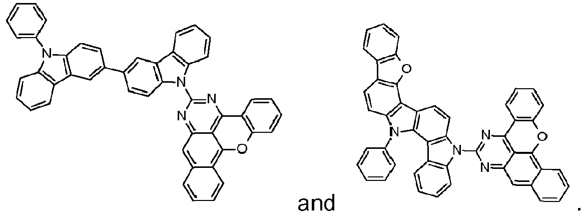

However, such fused systems are characterized by low triplet energies.

It is an object of the present invention to provide electronic devices, preferably OLEDs, comprising new materials, especially as a host material, a charge transporting material, charge and/or exciton blocking material, preferably as a host material, having a good overall performance, especially improved efficiency and/or driving voltage and preferably having a high triplet energy.

This object is solved by a compound represented by the general formula (I) 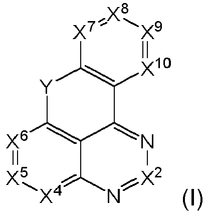 wherein
X² is CR²,
X⁴ is CR⁴ or N, preferably CR⁴,
X⁵ is CR⁵ or N, preferably CR⁵,
X⁶ is CR⁶ or N, preferably CR⁶,
X⁷ is CR⁷ or N, preferably CR⁷,
X⁸ is CR⁸ or N, preferably CR⁸,
X⁹ is CR⁹ or N, preferably CR⁹,
X¹⁰ is CR¹⁰ or N, preferably CR¹⁰,
Y is O or S,
wherein at least two adjacent groups X⁴, X⁵, X⁷, X⁸, X⁹ or X¹⁰ are not N, and at least two of R⁴, R⁵, R⁷, R⁸, R⁹ or R¹⁰, if present at adjacent carbon atoms, preferably at least two of R⁷, R⁸, R⁹ or R¹⁰, if present at adjacent carbon atoms, together form a structure according to the general formula (II) 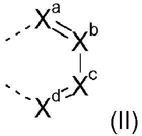 wherein
   X^{a} is CR^{a} or N, preferably CR^{a},
   X^{b} is CR^{b} or N, preferably CR^{b},
   X^{c} is CR^{c} or N, preferably CR^{c},
   X^{d} is CR^{d} or N, preferably CR^{d},
the remainder of R², R⁴, R⁵, R⁶, R⁷, R⁸, R⁹, R¹⁰, R^{a}, R^{b}, R^{c} and R^{d}
   are independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂saralkyl which is unsubstituted or substituted by at least one group E, or a C₅-C₁₂cycloalkyl group which is unsubstituted or substituted by at least one group E,
D is independently of each other -CO-, -COO-, -S-, -SO-, -SO₂-, -O-,-CR¹⁵=CR¹⁶-, -NR¹⁷-, - SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN, - SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group;
a C₁-C₂₅alkyl group, which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group;R¹⁵ and R¹⁶ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²¹ is independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C6-C18aryl group which is unsubstituted or substituted by at least one C6-C12aryl or by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₆-C₁₂aryl or by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a group of formula - (L¹)ₒ-(L²)ₚ-(L³)_{q}-(L⁴)ᵣ-R¹⁴,
wherein L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E, and
R¹⁴ is H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂saralkyl which is unsubstituted or substituted by at least one group E, a C₅-C₁₂cycloalkyl group which is unsubstituted or substituted by at least one group E, and
o is independently of each other 1, p is independently of each other 0 or 1, q is independently of each other 0 or 1 and r is independently of each other 0 or 1,
wherein the dotted lines are bonding sites.

The compounds of formula (I) are suitable as material for electronic devices, preferably OLEDs, especially as a host material, a charge transporting material, charge and/or exciton blocking material, preferably as a host material, having a good overall performance, especially improved efficiency and/or driving voltage and preferably having a high triplet energy, preferably of > 2.0 eV.

The residues mentioned in the specification of the present application generally have the following preferred meanings, if said residues are not further specified in specific embodiments mentioned below:
Halogen is fluorine, chlorine, bromine and iodine.

C₁-C₂₅alkyl, preferably C₁-C₁₈alkyl, is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups, preferably C₁-C₁₈alkoxy groups, are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₆₀aryl, preferably C₆-C₂₄aryl, particularly preferably C₆-C₁₈aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₆-C₂₄aryloxy, which optionally can be substituted, is typically C₆-C₁₀aryloxy, which optionally can be substituted by one, or more C₁-C₈alkyl and/or C₁-C₈alkoxy groups, such as, for example, phenoxy, 1-naphthoxy, or 2-naphthoxy.

C₁-C₆₀heteroaryl, preferably C₂-C₃₀heteroaryl, particularly preferably C₂-C₁₃heteroaryl, which optionally can be substituted, represents a ring with five to seven ring atoms or a condensed ring system, wherein nitrogen, oxygen or sulfur are the possible hetero atoms, and is typically a heterocyclic group with 5 to 40 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazyl, pyrimidyl, pyrazinyl, pyridazinyl, indolizinyl, isoindolyl, indolyl, indazolyl, purinyl, quinolizinyl, quinolyl, isoquinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₇-C₂₅aralkyl, which optionally can be substituted, is for example benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₅-C₁₂cycloalkyl, which optionally can be substituted, is for example cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

Examples of the alkylene group (i.e. alkane-diyl group) having 1 to 25 carbon atoms represented include methylene group, ethylene group, n-propylene group, isopropylene group, n-butylene group, s-butylene group, isobutylene group, t-butylene group, n-pentylene group, n-hexylene group, n-heptylene group, n-octylene group, n-nonylene group, n-decylene group, n-undecylene group, n-dodecylene group, n-tridecylene group, n-tetradecylene group, n-pentadecylene group, n-hexadecylene group, n-heptadecylene group, n-octadecylene group, neopentylene group, 1-methylpentylene group, with methylene group, ethylene group, n-propylene group, isopropylene group, n-butylene group, s-butylene group, isobutylene group, t-butylene group being preferred. The alkylene group is substituted or unsubstituted.

Examples of the cycloalkylene group (i.e. cycloalkane-diyl group) having 5 to 12 carbon atoms include cyclopropylene group, cyclobutylene group, cyclopentylene group, cyclohexylene group, cyclooctylene group, and adamantylene group, with cyclopentylene group, and cyclohexylene group being preferred. The cycloalkylene group is substituted or unsubstituted.

The arylene group is preferably a divalent aromatic hydrocarbon group having 6 to 60 ring carbon atoms may be a non-condensed divalent aromatic hydrocarbon group or a condensed divalent aromatic hydrocarbon group. Specific examples thereof include phenylene group, naphthylene group, phenanthrylene group, biphenyl-diyl group, terphenyl-diyl group, quaterphenyldiyl group, fluoranthen-diyl group, triphenylenylene-diyl group, phenanthrene-diyl group, fluorene-diyl group, spirofluorene-diyl group, 9,9-diphenylfluorene-diyl group, 9,9'-spirobi[9H-fluorene]-2-diyl group, 9,9-dimethylfluorene-diyl group, benzo[c]phenanthrene-diyl group, benzo[a]triphenylene-diyl group, naphtho[1,2-c]phenanthrene-diyl group, naphtho[1,2-a]triphenylenylene-diyl group, dibenzo[a,c]triphenylenylene-diyl group, and benzo[b]fluoranthene-diyl group, with phenylene group, naphthylene group, biphenyl-diyl group, terphenyl-diyl group, phenanthryl-diyl group, triphenylenylen-diyl group, fluorene-diyl group, spirobifluorene-diyl group, and fluoranthene-diyl group being preferred, and 1,2-phenylene group, 1,3-phenylene group, 1,4-phenylene group, 1,4-naphthylene group, 1,8-naphthylene group, 2,6-naphthylene group, 2,7-naphthylene group, biphenyl-2,2'-diyl group, biphenyl-2,3'-diyl group, biphenyl-2,4'-diyl group, biphenyl-2,5'-diyl group, biphenyl-2,6'-diyl group, biphenyl-3,3'-diyl group, biphenyl-3,4'-diyl group, biphenyl-3,5'-diyl group, biphenyl-3,6'-diyl group, biphenyl-4,4'-diyl group, biphenyl-4,5'-diyl group, biphenyl-4,6'-diyl group, biphenyl-5,5'-diyl group, biphenyl-5,6'-diyl group, biphenyl-6,6'-diyl group, phenanthrene-9,10-diyl group, phenanthrene-2,3-diyl group, phenanthrene-2,7-diyl group, phenanthrene-2,8-diyl group, phenanthrene-2,6-diyl group, phenanthrene-2,9-diyl group, phenanthrene-2,10-diyl group, phenanthrene-3,9-diyl group, phenanthrene-3,10-diyl group, triphenylene-2,3-diyl group, triphenylene-2,5-diyl group, triphenylene-2,6-diyl group, triphenylene-2,7-diyl group, triphenylene-2,8-diyl group, 9,9-dimethylfluorene-2,7-diyl group, 9,9-dimethylfluorene-3,7-diyl group, 9,9-dimethylfluorene-1,4-diyl group, fluoranthene-3,9-diyl group, fluoranthene-3,8-diyl group, fluoranthene-3,4-diyl group, fluoranthene-3,5-diyl group, fluoranthene-3,6-diyl group, fluoranthene-2,9-diyl group, fluoranthene-2,8-diyl group, fluoranthene-2,4-diyl group, fluoranthene-2,5-diyl group, fluoranthene-2,6-diyl group, fluoranthene-1,9-diyl group, fluoranthene-1,8-diyl group, fluoranthene-1,4-diyl group, fluoranthene-1,5-diyl group, fluoranthene-1,6-diyl group being more preferred. The arylene group is substituted or unsubstituted.

The heteroarylene group is preferably a divalent heterocyclic group having 5 to 30 ring atoms may be a non-condensed heterocyclic group or a condensed heterocyclic group. Specific examples thereof include the divalent residues of pyrrole ring, isoindole ring, benzofuran ring, isobenzofuran ring, dibenzothiophene ring, isoquinoline ring, quinoxaline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the divalent residues of derivatives of these rings, with the divalent residues of dibenzofuran ring, carbazole ring, dibenzothiophene ring, and derivatives of these divalent rings being preferred, and the dibenzofuran-diyl group, 9-phenylcarbazole-diyl group and dibenzothiophene-diyl group being more preferred. The heteroarylene group is substituted or unsubstituted.

The term "spiro group" is known by a person skilled in the art and is in general a group that forms a twisted structure of two or more rings (a ring system), in which 2 or 3 rings, preferably two rings, are linked together by one common atom. Typical spiro groups are for example: 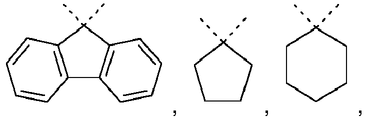 wherein the dotted lines are bonding sites. The spiro groups are unsubstituted or substituted.

The abovementioned groups are substituted or unsubstituted. Possible preferred optional substituents of the above-mentioned groups are C₁-C₈alkyl, a hydroxyl group, a mercapto group, C₁-C₈alkoxy, C₁-C₈alkylthio, halogen, halo-C₁-C₈alkyl, C₆-C₂₄aryl, C₂-C₃₀heteroaryl, or a cyano group.

The optional substituents mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

The "carbon number of a to b" in the expression of "X group having a to b carbon atoms" is the carbon number of the unsubstituted X group and does not include the carbon atom(s) of an optional substituent.

The hydrogen atom referred to herein includes isotopes different from neutron numbers, i.e., light hydrogen (protium), heavy hydrogen (deuterium) and tritium. Therefore, one or more or all of the hydrogen atoms in the residues mentioned above and below may be replaced by protium, deuterium or tritium, preferably by deuterium.

### The compounds of formula (I)

The present invention relates to compound of the general formula (I) 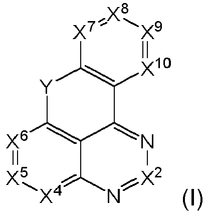 wherein X², X⁴, X⁵, X⁶, X⁷, X⁸, X⁹, X¹⁰ and Y have the meanings as mentioned above. Preferred embodiments are explained in the following.

Y in the general formula (I) is O or S, preferably O.

Therefore the present invention preferably relates to compounds according to the following general formulae (Ia) and (Ib), more preferably to compounds according to the following general formula (Ia), 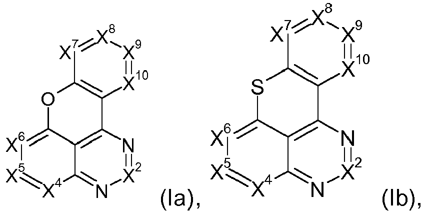 wherein independently of each other X², X⁴, X⁵, X⁶, X⁷, X⁸, X⁹, X¹⁰ and R¹¹ have the same meanings as mentioned above.

At least two adjacent groups X⁴, X⁵, X⁷, X⁸, X⁹ or X¹⁰ are not N. Preferably, 0, 1 or 2 of X⁴, X⁵, X⁶, X⁷, X⁸, X⁹, X¹⁰ in the compounds of formula (I) are N, more preferably, 0 of X⁴, X⁵, X⁶, X⁷, X⁸, X⁹, X¹⁰ are N, i.e. X⁴, X⁵, X⁶, X⁷, X⁸, X⁹, X¹⁰ are CR⁴, CR⁵, CR⁶, CR⁷, CR⁸, CR⁹ and CR¹⁰.

More preferred compounds of formula (I) are therefore compounds of the following general formulae (Ia1) and (Ib1), preferably (Ia1):

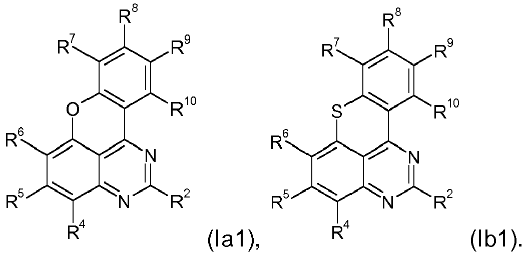

*In formula (I):*
At least two of R⁴, R⁵, R⁷, R⁸, R⁹ or R¹⁰, if present at adjacent carbon atoms, preferably at least two of R⁷, R⁸, R⁹ or R¹⁰, if present at adjacent carbon atoms, together form a structure according to the general formula (II) 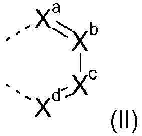 wherein
   X^{a} is CR^{a} or N, preferably CR^{a},
   X^{b} is CR^{b} or N, preferably CR^{b},
   X^{c} is CR^{c} or N, preferably CR^{c},
   X^{d} is CR^{d} or N, preferably CR^{d},
the remainder of R², R⁴, R⁵, R⁶, R⁷, R⁸, R⁹, R¹⁰, R^{a}, R^{b}, R^{c} and R^{d}
are independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂saralkyl which is unsubstituted or substituted by at least one group E, or a C₅-C₁₂cycloalkyl group which is unsubstituted or substituted by at least one group E,
wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a group of formula - (L¹)ₒ-(L²)ₚ-(L³)_{q}-(L⁴)ᵣ-R¹⁴.

D is -CO-, -COO-, -S-, -SO-, -SO₂-, -O-,-CR¹⁵=CR¹⁶-, -NR¹⁷-, -SiR²²R²³-, -POR²⁵-, -C≡C-, preferably -O-, -NR¹⁷-, -SiR²²R²³-.

E is -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₂₅alkyl group, which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; preferably E is independently of each other selected from -NR¹⁷R¹⁸, -CN, -SiR²²R²³R²⁴ , -POR²⁵R^{26'}, a C₁-C₁₈ alkyl group, a C6-C18aryl group which is unsubstituted or substituted by at least one C6-C12aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one C₁-C₁₈alkyl group, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group.

R¹⁵ and R¹⁶ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group which is interrupted by at least one O, preferably H.

R¹⁷ and R¹⁸ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H or a C₁-C₁₈ alkyl group.

R¹⁷ and R¹⁸ together form a five or six membered aliphatic, aromatic or heteroaromatic ring, preferably five or six membered aliphatic ring.

R¹⁹ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²⁰ is H or a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈ alkyl group or a C₁-C₁₈alkyl group, which is interrupted by at least one O, preferably H, a C₆-C₁₈aryl group which is unsubstituted or a C₁-C₁₈alkyl group.

R²¹ is independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²², R²³ and R²⁴ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group.

R²⁵, R^{26'} and R²⁷ are independently of each other H, a C₁-C₁₈alkyl group, a C₆-C₁₈aryl group which is unsubstituted or substituted by at least one C₆-C₁₂aryl or by at least one C₁-C₁₈alkyl group, a C₇-C₂saralkyl which is unsubstituted or substituted by at least one group E, a C₅-C₁₂cycloalkyl group which is unsubstituted or substituted by at least one group E, preferably H, a C₆-C₁₈aryl group which is unsubstituted or a C₁-C₁₈alkyl group.

Preferably, in formula (I), X⁴ is CR⁴, X⁵ is CR⁵, X⁶ is CR⁶, X⁷ is CR⁷, X⁸ is CR⁸, X⁹ is CR⁹ and X¹⁰ is CR¹⁰.

Preferably at least two of R⁷, R⁸, R⁹ or R¹⁰, if present at adjacent carbon atoms, together form a structure according to the general formula (II) 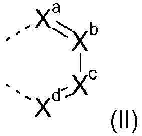 wherein
X^{a} is CR^{a} or N, preferably CR^{a},
X^{b} is CR^{b} or N, preferably CR^{b},
X^{c} is CR^{c} or N, preferably CR^{c},
X^{d} is CR^{d} or N, preferably CR^{d},
more preferably a structure according to the general formula (IIa) 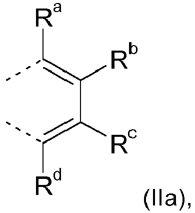 wherein R^{a}, R^{b}, R^{c} and R^{d} are defined above and below.

More preferably, the compound of formula (I) is represented by the following formulae (III), (IV), (V) or (VI) 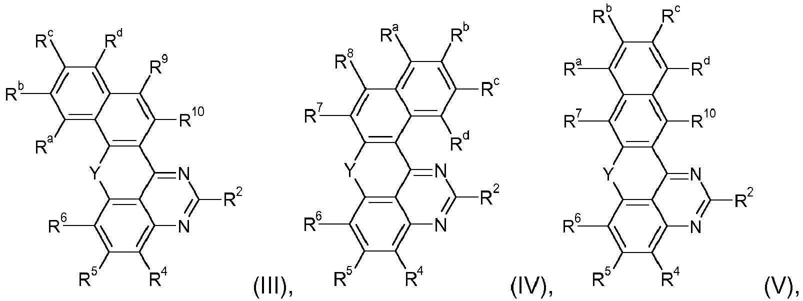 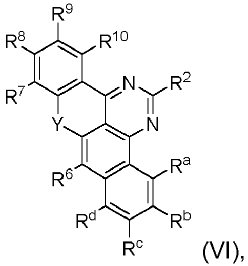 wherein the residues R², R⁴, R⁵, R⁶, R⁷, R⁸, R⁹, R¹⁰, R^{a}, R^{b}, R^{c} and R^{d} and Y are defined above and below.

Preferably, the remainder of R², R⁴, R⁵, R⁶, R⁷, R⁸, R⁹, R¹⁰, R^{a}, R^{b}, R^{c} and R^{d}
are independently of each other selected from H, a C₆-C₂₄aryl group which is unsubstituted or substituted by at least one group E, C₂-C₂₄heteroaryl group which is unsubstituted or substituted by at least one group E, a C₁-C₂₅alkyl group, which is unsubstituted or substituted by at least one group E and/or interrupted by at least one group D a C₅-C₁₂cycloalkyl group which is unsubstituted or substituted by at least one group E,
wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a group of formula - (L¹)ₒ-(L²)ₚ-(L³)_{q}-(L⁴)ᵣ-R¹⁴.

More preferably, the remainder of R², R⁴, R⁵, R⁶, R⁷, R⁸, R⁹, R¹⁰, R^{a}, R^{b}, R^{c} and R^{d} are independently of each other selected from H, a C₆-C₂₄aryl group which is unsubstituted or substituted by at least one group E or C₂-C₂₄heteroaryl group which is unsubstituted or substituted by at least one group E,
wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a group of formula - (L¹)ₒ-(L²)ₚ-(L³)_{q}-(L⁴)ᵣ-R¹⁴.

Most preferably, one of the remainder of R², R⁴, R⁵, R⁶, R⁷, R⁸, R⁹, R¹⁰, R^{a}, R^{b}, R^{c} and R^{d}, preferably R², in the compound of formula (I) is a group of formula -(L¹)ₒ-(L²)ₚ-(L³)_{q}-(L⁴)ᵣ-R¹⁴ as described below, and the remaining of R², R⁴, R⁵, R⁶, R⁷, R⁸, R⁹, R¹⁰, R^{a}, R^{b}, R^{c} and R^{d} are H.

Further most preferably, R² in the compound of formula (I) is a group of formula -(L¹)ₒ-(L²)ₚ-(L³)_{q}-(L⁴)ᵣ-R¹⁴ as described below, and R², R⁴, R⁵, R⁶, R⁷, R⁸, R⁹, R¹⁰, R^{a}, R^{b}, R^{c} and R^{d} are H.

Most preferred are therefore compounds of formula (IIIa1a), (IVa1a), (Va1a), (IIIb1a), (IVb1a) and (Vb1a), further most preferred are compounds of formula (IIIa1a), (IVa1a) and (Va1a): 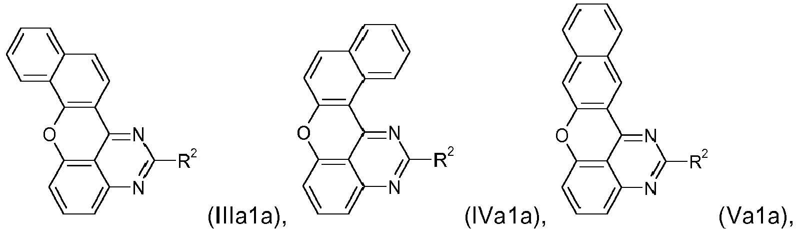 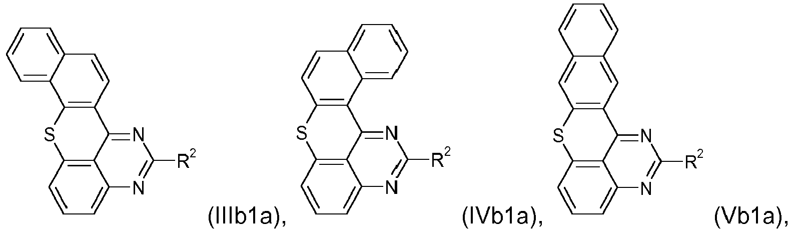 wherein R² is a group of formula -(L¹)ₒ-(L²)ₚ-(L³)_{q}-(L⁴)ᵣ-R¹⁴ as described above and below.

### The group of formula -(L¹)ₒ-(L²)ₚ-(L³)_{q}-(L⁴)ᵣ-R¹⁴

In formula (I), in the group of formula -(L¹)ₒ-(L²)ₚ-(L³)_{q}-(L⁴)ᵣ-R¹⁴, L¹, L², L³ and L⁴, o, p, q, r and R¹⁴ have the following meaning:
L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E. Preferably L¹. L², L³ and L⁴ are independently of each other selected from a C₆-C₄₀arylene group which is unsubstituted and from a C₁-C₂₄heteroarylene group which is unsubstituted.

R¹⁴ is H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂saralkyl which is unsubstituted or substituted by at least one group E, a C₅-C₁₂cycloalkyl group which is unsubstituted or substituted by at least one group E. Preferably, R¹⁴ is H or a C₁-C₂₅alkyl group which unsubstituted or substituted by at least one group E and/or interrupted by at least one group D. More preferably, R¹⁴ is H or C₁-C₈alkyl, which is linear or branched. Most preferably, R¹⁴ is H.

o is independently of each other 1, p is independently of each other 0 or 1, q is independently of each other 0 or 1 and r is independently of each other 0 or 1, preferably, o is 1, p is 0 or 1 and q and r are 0. More preferably, o is 1 and p, q and r are 0.

Most preferably, the group of formula -(L¹)ₒ-(L²)ₚ-(L³)_{q}-(L⁴)ᵣ-R¹⁴ is a N-heteroaryl group, further most preferably a N-heteroaryl group of one of the following formulae (XII), (XIII), (XV), (XVI) or (XXI): 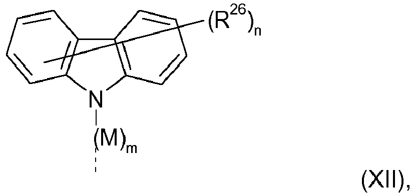 wherein
n is an integer of 0 to 8, preferably 0, 1, 2, 3 or 4, more preferably 0, 1 or 2, most preferably 1, m is an integer of 0 to 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂saralkyl which is unsubstituted or substituted by at least one group E, or a C₅-C₁₂cycloalkyl group which is unsubstituted or substituted by at least one group E, or
at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
wherein the dotted line is a bonding site;
or 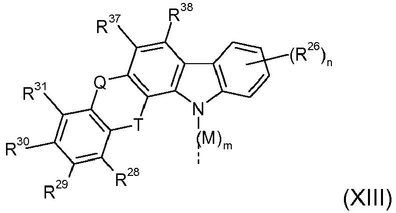
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂saralkyl which is unsubstituted or substituted by at least one group E, a C₅-C₁₂cycloalkyl group which is unsubstituted or substituted by at least one group E, preferably H, phenyl or biphenyl,
   or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂saralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 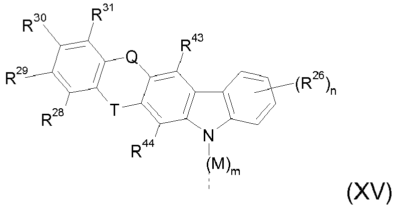
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,R²⁸, R²⁹, R³⁰, R³¹ R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂saralkyl which is unsubstituted or substituted by at least one group E, a C₅-C₁₂cycloalkyl group which is unsubstituted or substituted by at least one group E, preferably H,
   or
at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂saralkyl which is unsubstituted or substituted by at least one group E, or a C₅-C₁₂cycloalkyl group which is unsubstituted or substi-tuted by at least one group E, or R³⁴ and R³⁵ together may form a spiro group, preferably, R³⁴ and R³⁵ are independently of each other H, ethyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group, for example phenyl,
wherein the dotted line is a bonding site,
or 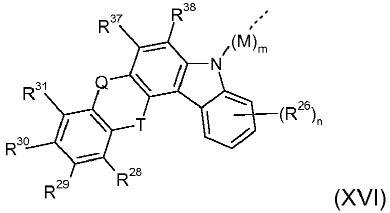
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R29, R³⁰, R31, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
   or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 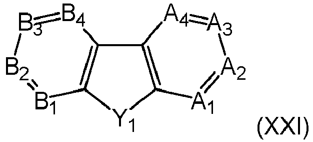 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is NR⁷⁰, CR⁷¹R⁷², O or S;
R⁶², R⁶³, R⁶⁴, R⁶⁵, R⁶⁶, R⁶⁷, R⁶⁸ and R⁶⁹ are independently of each other H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   or
at least two of R⁶², R⁶³, R⁶⁴ and R⁶⁵ may be directly bonded to the moiety represented by the general formula (XXII) via the two *-locations; 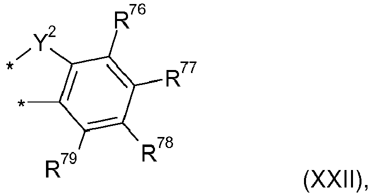 wherein
   Y² is NR⁷³, CR⁷⁴R⁷⁵, O or S,
      or
   at least two of R⁶⁶, R⁶⁷, R⁶⁸ and R⁶⁹ may be directly bonded to the moiety represented by the general formula (XXIII) via the two *-locations. 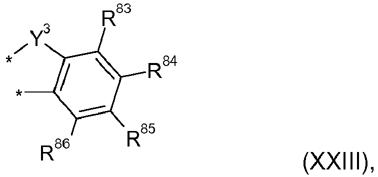
   wherein
   Y³ is NR⁸⁰, CR⁸¹R⁸², O or S,
   R⁷⁰ , R⁷³ and R⁸⁰ are independently of each other a direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   R⁷¹, R⁷², R⁷⁴, R⁷⁵, R⁸¹ and R⁸² are, independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   or R⁷¹, and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
   R⁷⁶, R⁷⁷, R⁶⁵, R⁷⁹, R⁸³, R⁶⁴, R⁸⁵ and R⁸⁶ are independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or
   R⁷⁶ and R⁷⁷, R⁷⁷ and R⁷⁸ and/or R⁷⁸ and R⁷⁹ together may form at least one C₆-C₁₈aryl or C₂-C₁₈heteroaryl ring or ring system, and/or
   R⁸³ and R⁸⁴, R⁸⁴ and R⁸⁵ and/or R⁸⁵ and R⁸⁶ together form at least one C₆-C₁₈aryl or C₂-C₁₈heteroaryl ring or ring system,
   wherein the heterocyclic group according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, wherein this respective residue R⁷⁰, R⁷³, R⁸⁰, R⁶², R⁶³, R⁶⁴, R⁶⁵, R⁶⁶, R⁶⁷, R⁶⁶, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
   m is 1, 2, 3 or 4,
   M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

*The N-heteroaryl groups according to general formulae (XII), (XIII), (XV), (XVI) or (XXI) are described in the following:*

### N-heteroaryl groups according to general formula (XXI)

In one embodiment of the present invention, the N-heteroaryl group is represented by the general formula (XXI) 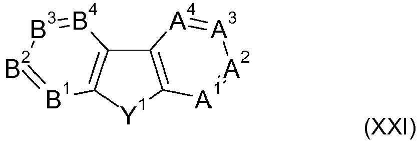 wherein
A¹ is CR⁶² or N, preferably CR⁶²,
A² is CR⁶³ or N, preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a C₆-C₄₀aryl group which is unsubstituted or substituted by at least one group E, C₁-C₃heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
   and/or
at least two of R⁶², R⁶³, R⁶⁴ and R⁶⁵, if present are directly bonded to the moiety represented by the general formula (XXI I) by the two *-locations. 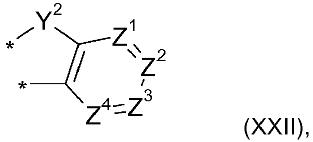
wherein
Y² is NR⁷³, CR⁷⁴R⁷⁵, O or S,
Z¹ is N or CR⁷⁶, preferably CR⁷⁶,
Z² is N or CR⁷⁷, preferably CR⁷⁷,
Z³ is N or CR⁷⁸, preferably CR⁷⁸,
Z⁴ is N or CR⁷⁹, preferably CR⁷⁹,
   and/or
R⁶², R⁶³, R⁶⁴ or R⁶⁵, if present at adjacent carbon atoms, together form at least one C₆-C₁₈aryl or C₂-C₁₈heteroaryl ring or ring system,
   and/or
at least two of R⁶⁶, R⁶⁷, R⁶⁸ and R⁶⁹, if present are directly bonded to the moiety represented by the general formula (XXIII) by the two *-locations. 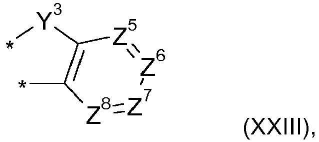
wherein
Y³ is NR⁸⁰, CR⁸¹R⁸², O or S,
Z⁵ is N or CR⁸³, preferably CR⁸³,
Z⁶ is N or CR⁸⁴, preferably CR⁸⁴,
Z⁷ is N or CR⁸⁵, preferably CR⁸⁵,
Z⁸ is N or CR⁸⁶, preferably CR⁸⁶,
   and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably phenyl, biphenyl, naphthyl, phenanthryl, triphenylenyl, fluorenyl, dibenzothienyl, dibenzofuryl, N-phenylcarbazolyl, pyridyl, pyrimidyl, triazyl and quinolyl, which are unsubstituted or substituted by at least one group E, preferably unsubstituted,
R⁷¹, R⁷², R⁷⁴, R⁷⁵, R⁸¹ and R⁸² are, independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably methyl or phenyl, which is unsubstituted or substituted by at least one group E, preferably unsubstituted,
or R⁷¹, and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁶⁵, R⁷⁹, R⁸³, R⁶⁴, R⁸⁵ and R⁸⁶ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
   and/or
at least two of R⁷⁶, R⁷⁷, R⁷⁸, R⁷⁹, R⁸³, R⁸⁴, R⁸⁵ or R⁶⁶, if present at adjacent carbon atoms, together form at least one C₆-C₁₈aryl or C₂-C₁₈heteroaryl ring or ring system,
wherein the substituent according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁶⁴, R⁸⁵ or R⁸⁶, if present, preferably R⁷⁰, R⁷³ or
R⁸⁰, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁶⁴, R⁸⁵ or R⁸⁶, preferably R⁷⁰, R⁷³ or R⁸⁰, is a direct bond, optionally interrupted by a group of formula -(M)ₘ-, in this case,
m is 0, 1, 2, 3 or 4, preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D, preferably phenylene or naphthylene, which are unsubstituted or substituted by at least one group E, preferably unsubstituted,
wherein D and E have the same meanings as mentioned above.

According to the present invention the heterocyclic group according to general formula (XXI), which may be for example a dibenzofuran, dibenzothiophene or benzofurodibenzofuran group, can be attached to the compound of general formula (I) via any atom present in the heterocyclic ring system. Therefore, any of R⁷⁰, R⁷³, R⁸⁰, R⁶², R⁶³, R⁶⁴, R⁶⁵, R⁶⁶, R⁶⁷, R⁶⁸ R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, preferably R⁷⁰, R⁷³ or R⁸⁰, if present, can be a direct bond, optionally interrupted by a group of formula -(M)ₘ-, with which the heterocyclic group according to formula (XXI) can be attached to the compound of general formula (I).

Preferably, the heterocyclic group according to general formula (XXI) is represented by any one of general formula (XXIa), (XXIB) or (XXIc) 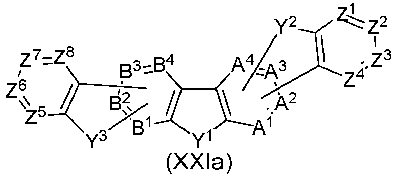 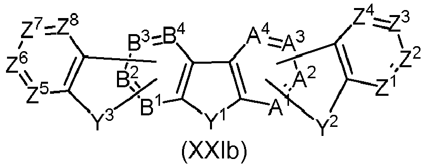 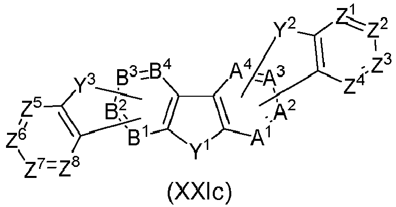 wherein A¹, A², A³, A⁴, B¹, B², B³, B⁴, Z¹, Z², Z³, Z⁴, Z⁵, Z⁶, Z⁷, Z⁸, Y¹, Y² and Y³ have the same meanings as defined above.

According to a further preferred embodiment of the present invention one of Y¹, Y² and Y³ in the compounds according general formulae (XXI), (XXIa), (XXIB) and/or (XXIc) is NR⁷⁰, NR⁷³ or NR⁸⁰ respectively.

According to a further preferred embodiment of the present invention two of Y¹, Y² and Y³ in the compound according to general formulae (XXI), (XXIa), (XXIB) and/or (XXIc) are NR⁷⁰, NR⁷³ or NR⁸⁰ respectively.

Preferably, the N-heteroaryl group according to general formula (XXI) corresponds to a N-heteroaryl group according to general formula (XXI) 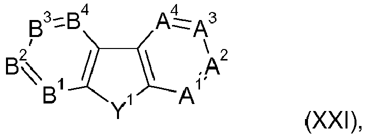
wherein A¹ is CR⁶²,
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
R⁶⁶, R⁶⁷, R⁶⁸ and R⁶⁹ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
   and
at least two of R⁶², R⁶³, R⁶⁴ and R⁶⁵ are direct bond and, if present at adjacent carbon atoms, are directly connected to the two *-locations in the moiety of general formula (XXII) 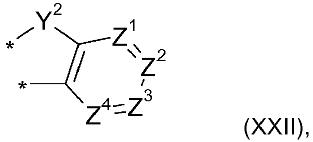 wherein
   Y² is NR⁷³, CR⁷⁴R⁷⁵, O or S,
   Z¹ is CR⁷⁶,
   Z² is CR⁷⁷,
   Z³ is CR⁷⁸,
   Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
wherein R⁷⁶, R⁷⁷, R⁷⁸ and R⁷⁹ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably hydrogen,
R⁷³, R⁷⁴ and R⁷⁵, have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰, CR⁷¹R⁷², O or S
at least two of R⁶², R⁶³, R⁶⁴ and R⁶⁵ are direct bond and, if present at adjacent carbon atoms, are directly connected to the two *-locations in the moiety of general formula (XXII) 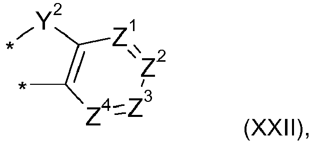 wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
   and
at least two of R⁶⁶, R⁶⁷, R⁶⁸ and R⁶⁹, if present at adjacent carbon atoms, are directly connected to the two *-locations in the moiety of general formula (XXIII) 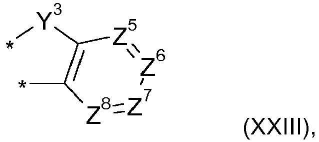 wherein
   Y³ is NR⁸⁰,
   Z⁵ is CR⁸³,
   Z⁶ is CR⁸⁴,
   Z⁷ is CR⁸⁵,
   Z⁸ is CR⁸⁶,
and the remaining of R⁶⁶, R⁶⁷, R⁶⁸ and R⁶⁹, that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁶⁵, R⁷⁹, R⁸³, R⁶⁴, R⁸⁵ and R⁸⁶ are independently of each other selected from direct bond, H, E, a C₆-C₄₀aryl group which is unsubstituted or substituted by at least one group E, C₁-C₃₉₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁰, R⁷¹, R⁷², R⁷³, R⁷⁴ and R⁷⁵ have the same meanings as mentioned above,
and the substituent according to general formula (XXI) is connected to the compound according to general formula (I) via R⁸⁰ being a direct bond, which is optionally interrupted by a group of formula -(M)ₘ-, wherein M and m have the same meanings as mentioned above, preferably m is 0 or 1.
In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
at least two of R⁶², R⁶³, R⁶⁴ and R⁶⁵ are direct bond and, if present at adjacent carbon atoms, are directly connected to the two *-locations in the moiety of general formula (XXII) 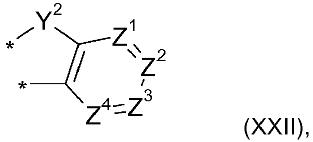 wherein
   Y² is NR⁷³, CR⁷⁴R⁷⁵, O or S,
   Z¹ is CR⁷⁶,
   Z² is CR⁷⁷,
   Z³ is CR⁷⁸
   Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
   and
at least two of R⁶⁶, R⁶⁷, R⁶⁸ and R⁶⁹, if present at adjacent carbon atoms, are directly connected to the two *-locations in the moiety of general formula (XXIII) 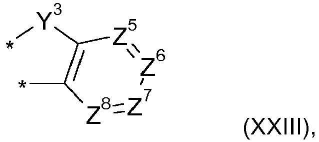 wherein
   Y³ is NR⁸⁰, CR⁸¹R⁸², O or S,
   Z⁵ is CR⁸³,
   Z⁶ is CR⁸⁴,
   Z⁷ is CR⁸⁵,
   Z⁸ is CR⁸⁶,
and the remaining of R⁶⁶, R⁶⁷, R⁶⁸ and R⁶⁹ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁶⁵, R⁷⁹, R⁸³, R⁶⁴, R⁸⁵ and R⁸⁶ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷³ R⁷⁴, R⁷⁵, R⁸⁰, R⁸¹ and R⁸² have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

### N-heteroaryl groups according to general formula (XIII)

In one embodiment of the present invention, the N-heteroaryl group is represented by the general formula (XIII) 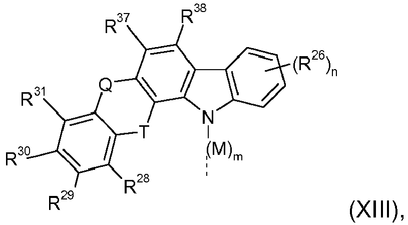
wherein M, m and R²⁶ have the meanings as mentioned above, preferably m is 0;
n is 0, 1, 2, 3 or 4, preferably n is 0;
R²⁸, R²⁹, R³⁰, R³¹, R³⁷ and R³⁸ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl, biphenyl, more preferably H;
   or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, preferably NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, wherein R³⁴ and R³⁵ together form a spiro group, preferably H, methyl, ethyl, or phenyl, wherein R³⁴ and R³⁵ together form a spiro group,
R³⁶ is a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, a C₆-C₁₈aryl group which is unsubstituted or substituted by phenyl, or a C₁-C₁₈alkyl group, more preferably a phenyl group;
wherein D and E have the same meanings as mentioned above.

Preferred compounds of formula (XIII) are compounds of the following formula (XIIIa), wherein T is a direct bond, and compounds of formula (XIIIb), wherein Q is a direct bond: 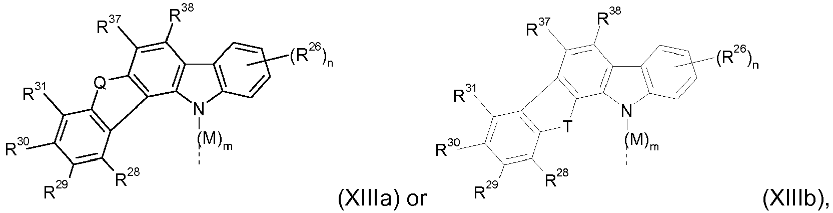 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XIII) are the following compounds: 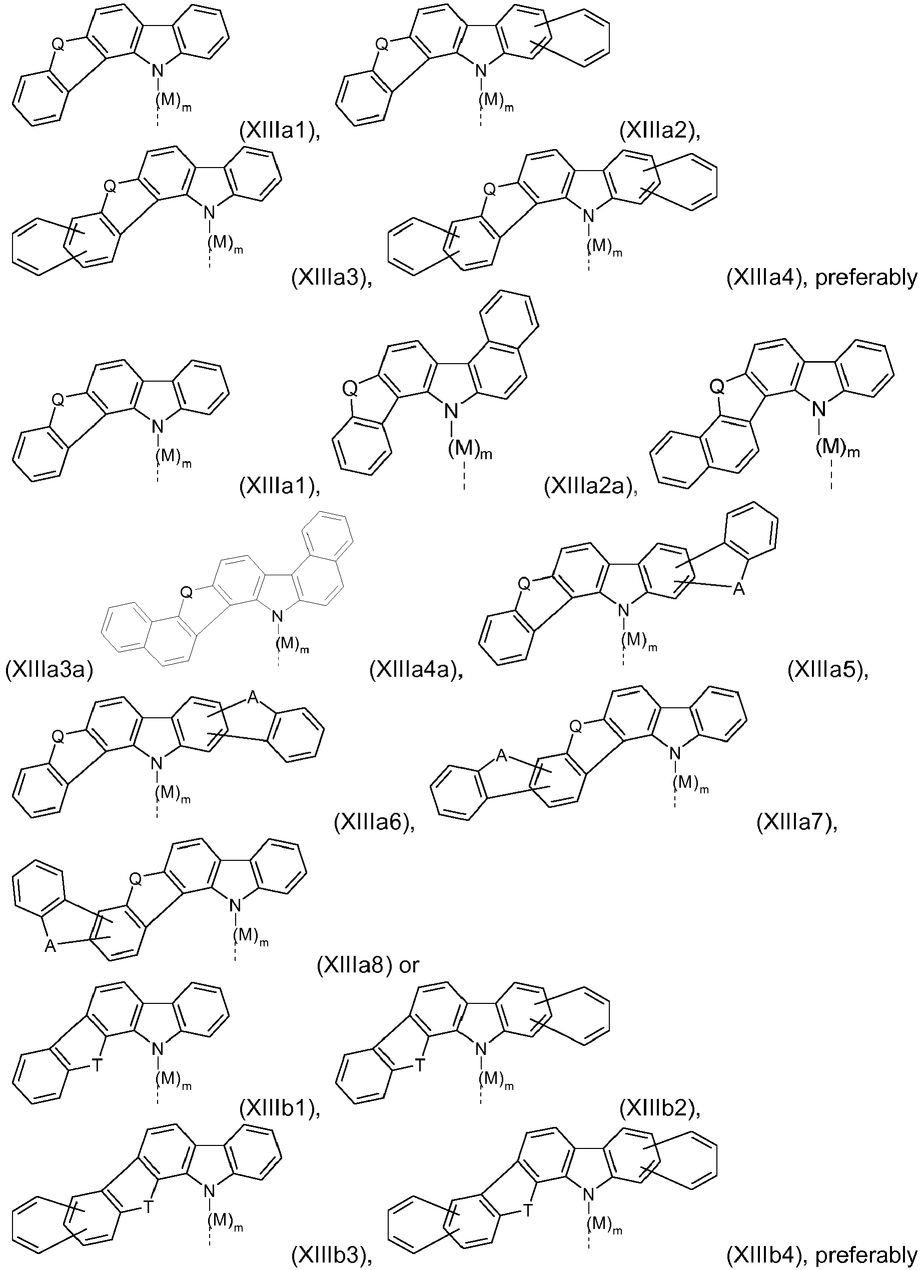 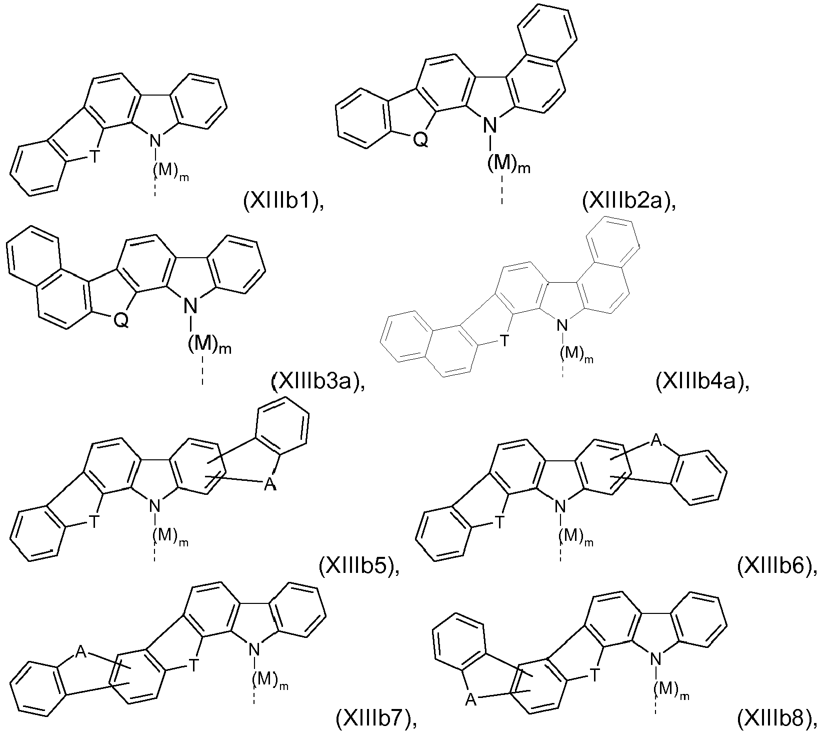 wherein
A is S, O, SiR³²R³³, CR³⁴R³⁵ or NR³⁶, preferably S, O, CR³⁴R³⁵ or NR³⁶,, and
Q and T are independently of each other S, O, SiR³²R³³, CR³⁴R³⁵ or NR³⁶, preferably NR³⁶.

According to a particularly preferred embodiment of the present invention, in the compound of formula (XIII), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XIII), Q is direct bond, T is NR³⁶, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XIII), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is NR³⁶, T is direct bond, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

Most preferred compounds of formula (XIII) are compounds of formula (XIII), wherein Q is a direct bond, especially preferred are therefore compounds (Xlllb), more preferably (XIIIb1) to (XIIIb12).

According to a further preferred embodiment, the heteroaryl group according to general formula (XIII) corresponds to general formula (XIV) 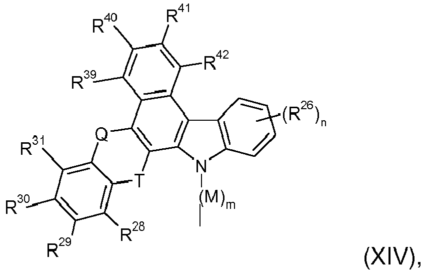
wherein R²⁶, R²⁸, R²⁹, R³⁰, R³¹, M, Q, T, n and m have the same meanings as mentioned above and
R³⁹, R⁴⁰, R⁴¹ R⁴² are independently of each other selected from H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

Preferred compounds of formula (XIV) are compounds of the following formula (XIVa), wherein T is a direct bond, and compounds of formula (XIVb), wherein Q is a direct bond: 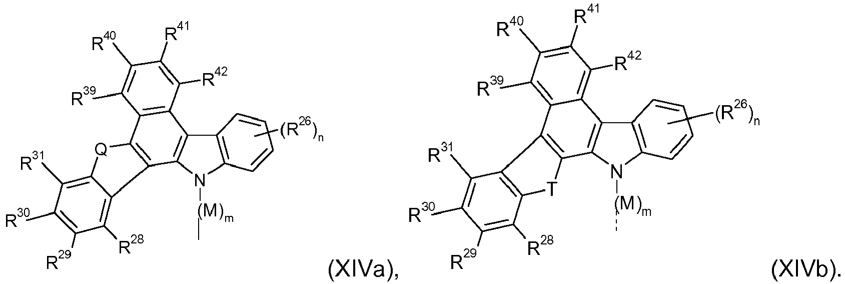 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XIV) are the following compounds: 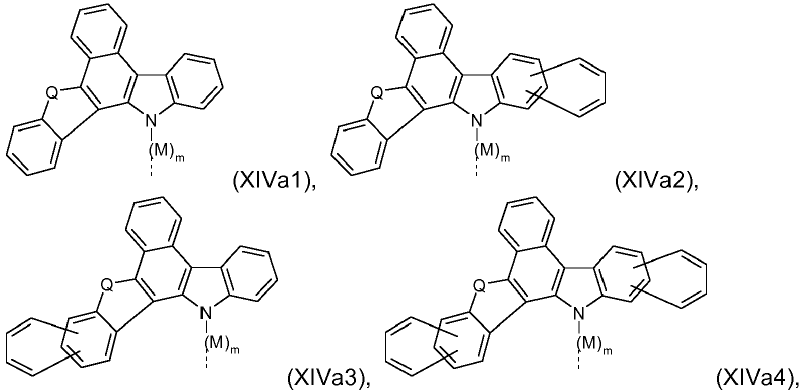 preferably 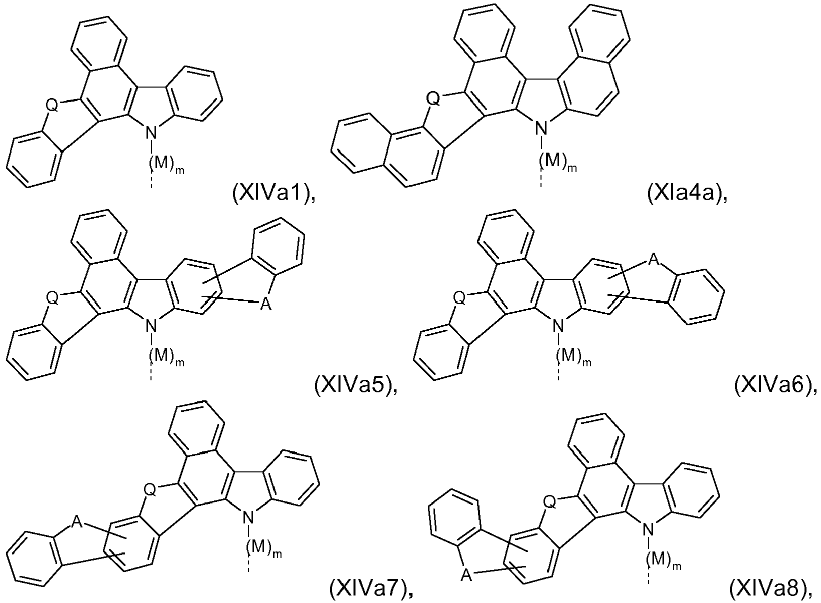 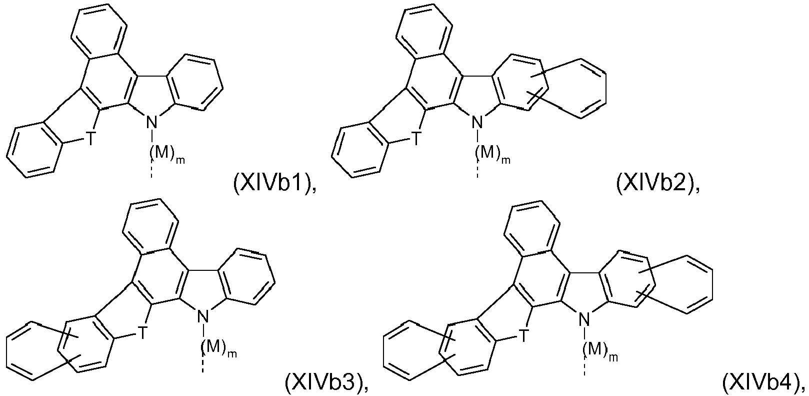 preferably 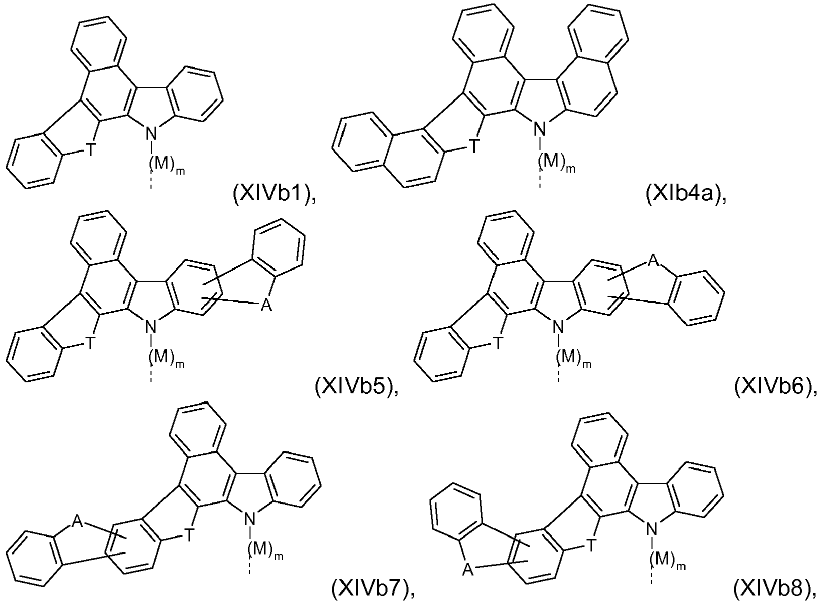 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

### N-heteroaryl groups according to general formula (XV)

In one embodiment of the present invention, the N-heteroaryl group is represented by the general formula 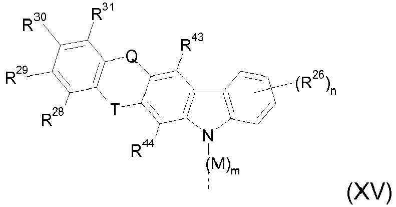
wherein n is 0, 1, 2, 3 or 4, preferably 0, 1 or 2
m is 0, 1, 2, 3 or 4, preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D, preferably, M is a C₆-C₄₀ arylene group which is unsubstituted, a C₁-C₂₄heteroarylene group which is unsubstituted or a C₁-C₂₅ alkylene group which unsubstituted, particularly preferably, M is a C₆-C₁₈ arylene group which is unsubstituted, most preferably a phenylene group,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
preferably R²⁶ is H or two R²⁶ that are present at adjacent carbon atoms together form a fused phenyl ring,
R²⁸, R²⁹, R³⁰, R³¹ R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, or at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably, R³⁴ and R³⁵ are independently of each other H, ethyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group, for example phenyl,
wherein the dotted line is a bonding site;
wherein D and E have the same meanings as mentioned above.

Preferred compounds of formula (XV) are compounds of the following formula (XVa), wherein T is a direct bond, and compounds of formula (XVb), wherein Q is a direct bond: 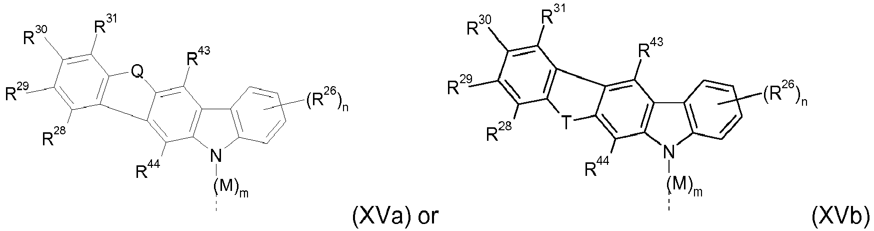 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XV) are the following compounds: 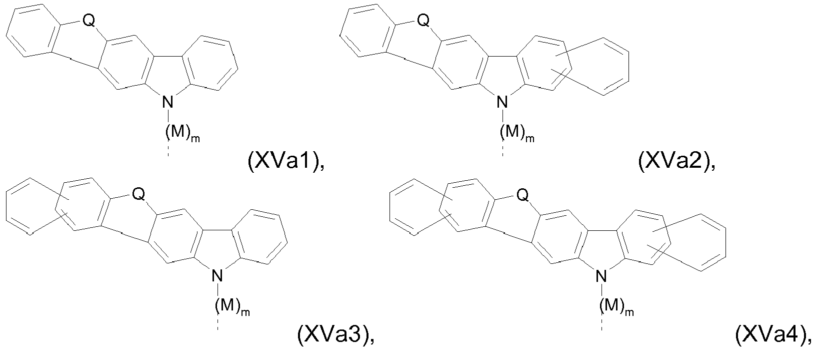 preferably 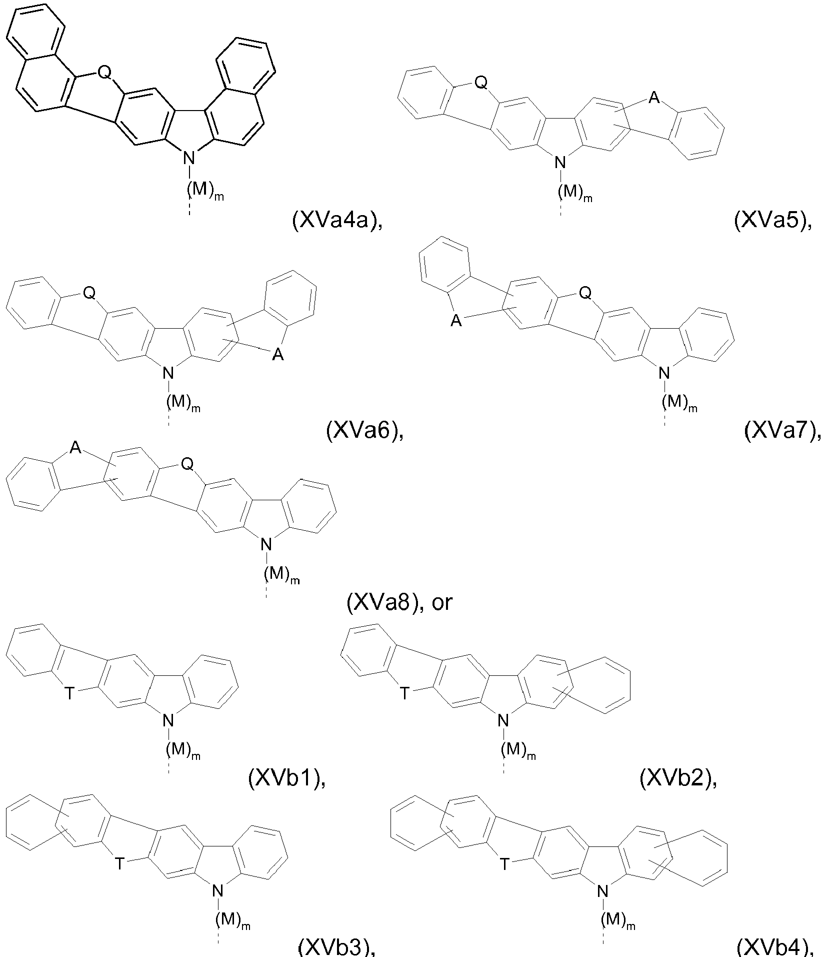 preferably 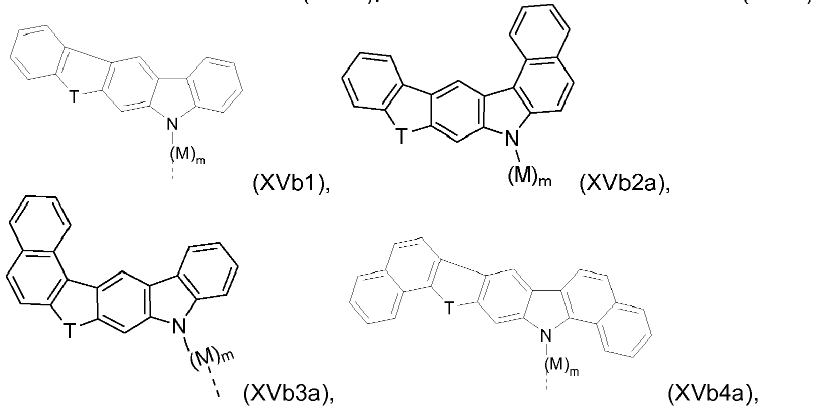 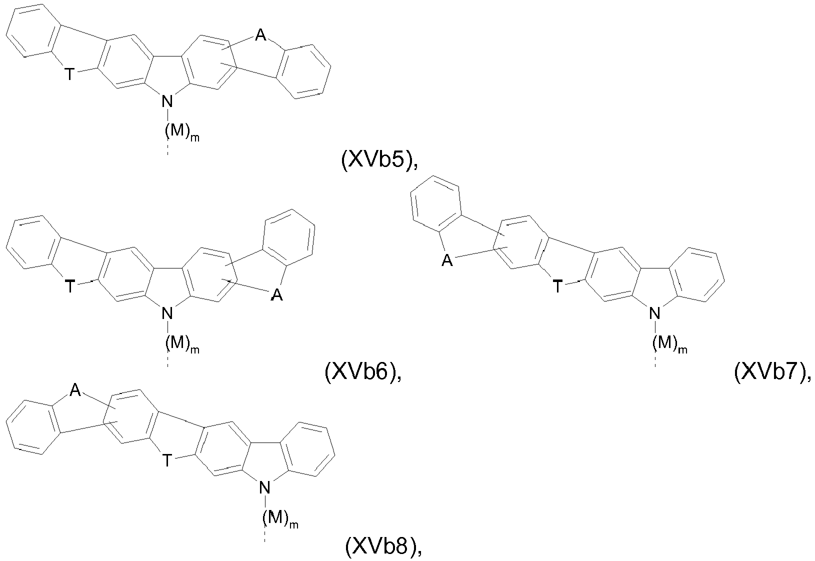 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

According to a particular preferred embodiment of the present invention in substituent according to general formula (XV), m is 0, n is 0, R²⁸, R²⁹, R³⁰, R³¹, R⁴³ and R⁴⁴ are H, Q is a direct bond, T is CR³⁴R³⁵, R³⁴ and R³⁵ are methyl.

According to a further particular preferred embodiment of the present invention in substituent according to general formula (XV), m is 0, n is 2, R²⁸, R²⁹, R³⁰, R³¹, R⁴³ and R⁴⁴ are H, two R²⁶ that are present at adjacent carbon atoms together form a fused phenyl ring, which is preferably present in position of R³⁰ and R³¹, Q is a direct bond, T is CR³⁴R³⁵, R³⁴ and R³⁵ are methyl.

### N-heteroaryl groups according to general formula (XVI)

In one embodiment of the present invention, the N-heteroaryl group is represented by the general formula (XVI) 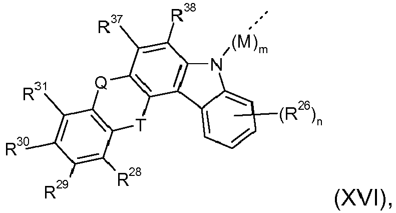
wherein n is 0, 1, 2, 3 or 4, preferably 0, 1 or 2, more preferably 0 or 1, most preferably 0, m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site; and
wherein D and E have the same meanings as mentioned above.

Preferred compounds of formula (XVI) are compounds of the following formula (XVIa), wherein T is a direct bond, and compounds of formula (XVIb), wherein Q is a direct bond: 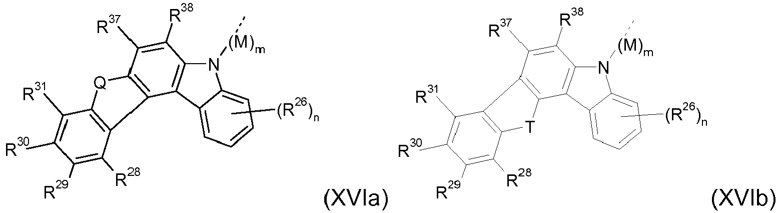 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XVI) are the following compounds: 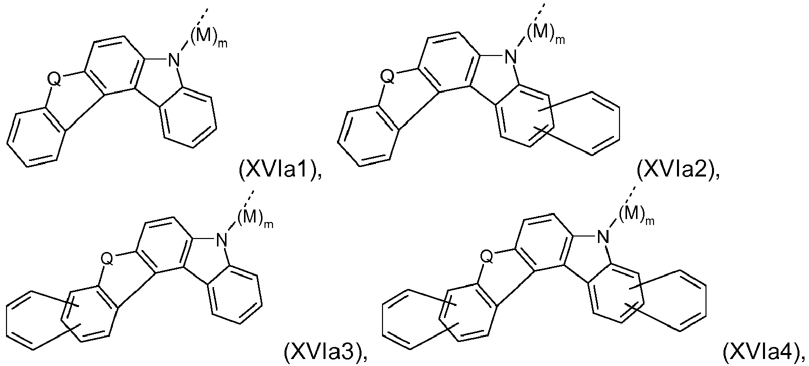 preferably 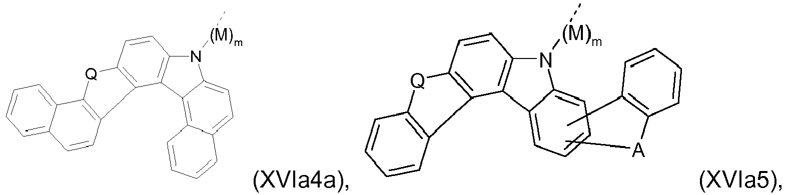 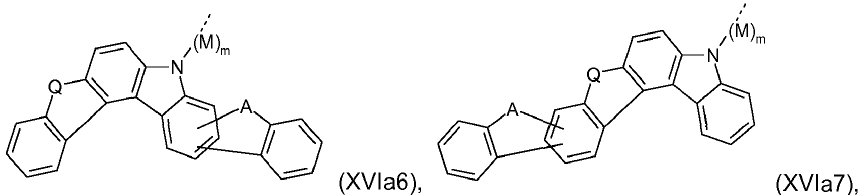 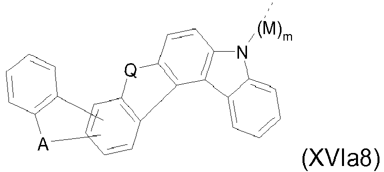 or 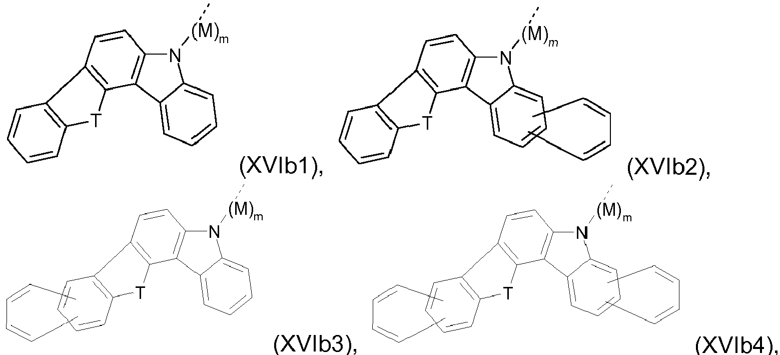 preferably 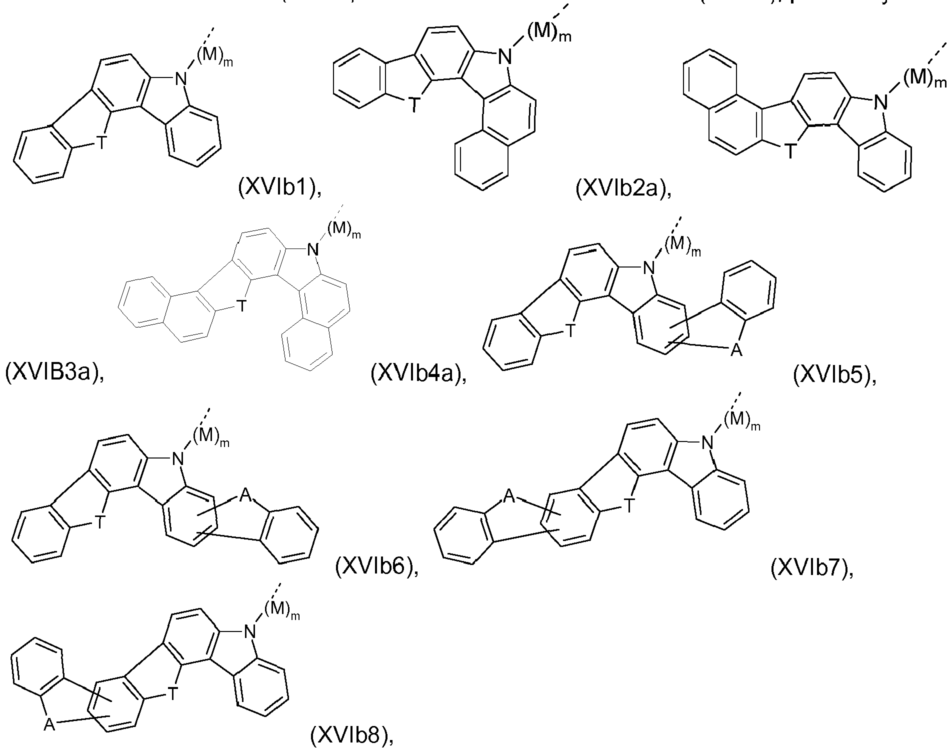 wherein
A is S, O, SiR³²R³³, CR³⁴R³⁵ or NR³⁶, preferably S, O, CR³⁴R³⁵ or NR³⁶, and
Q and T are independently of each other S, O, SiR³²R³³, CR³⁴R³⁵ or NR³⁶, preferably NR³⁶.

According to a particularly preferred embodiment of the present invention, in the compound of formula (XVI), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XVI), Q is direct bond, T is NR³⁶, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XVI), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XVI), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XVI), Q is NR³⁶, T is direct bond, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

Most preferred compounds of formula (XVI) are compounds of formula (XVI), wherein Q is a direct bond, especially preferred are therefore compounds (XVIb), more preferably (XVIb1) to (XVIb12).

According to a further preferred embodiment, the heteroaryl group according to general formula (XVI) corresponds to general formula (XVII) 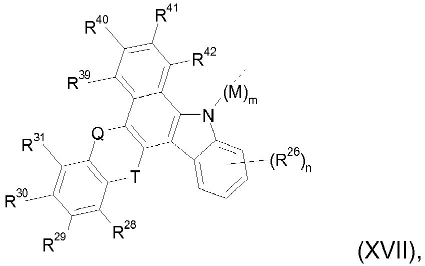
wherein R²⁶, R²⁸, R²⁹, R³⁰, R³¹, M, Q, T, n and m have the same meanings as mentioned above and
R³⁹, R⁴⁰, R⁴¹ R⁴² are independently of each other selected from H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

Preferred compounds of formula (XVII) are compounds of the following formula (XVIIa), wherein T is a direct bond, and compounds of formula (XVIIb), wherein Q is a direct bond: 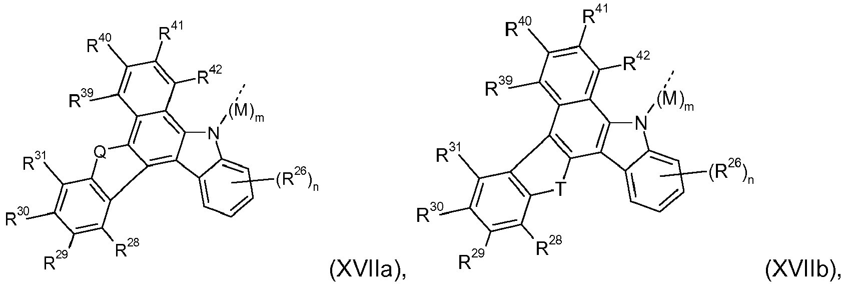 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XVII) are the following compounds: 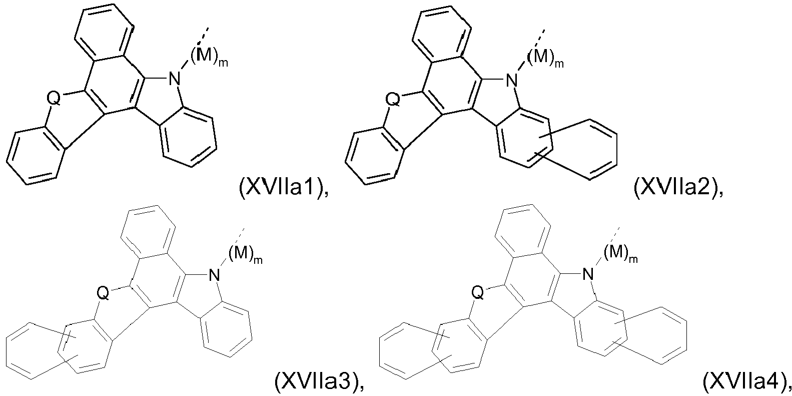 preferably 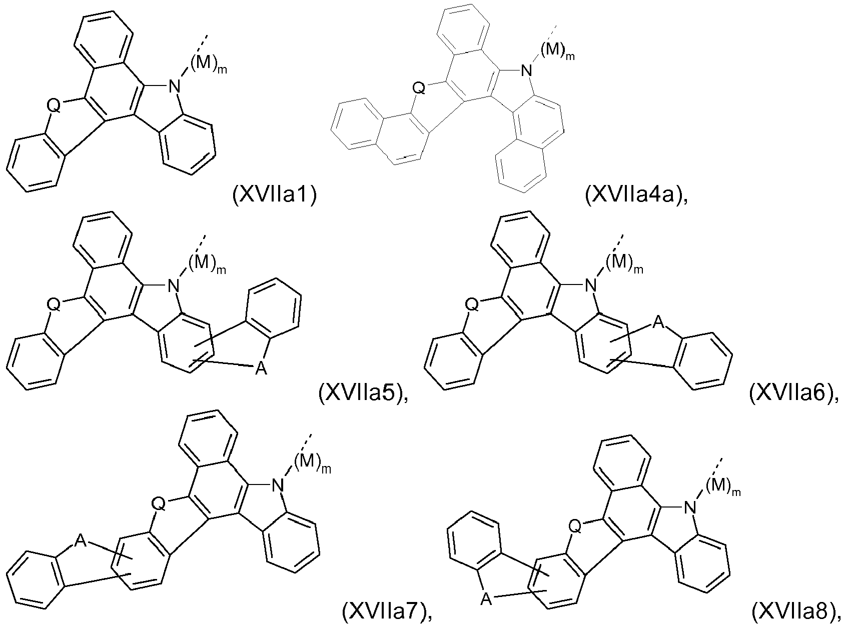 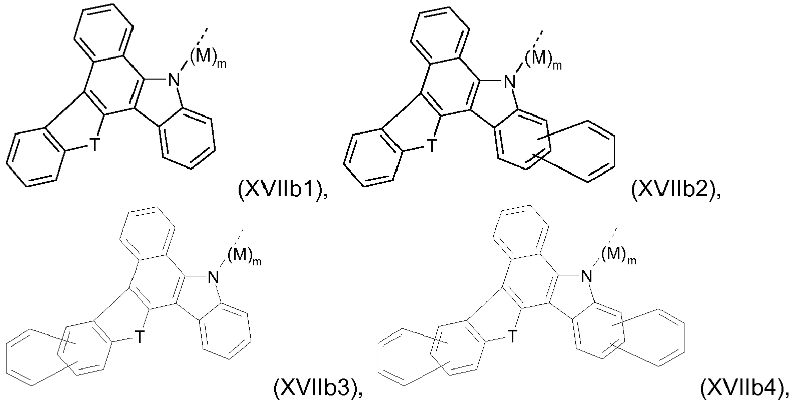 preferably 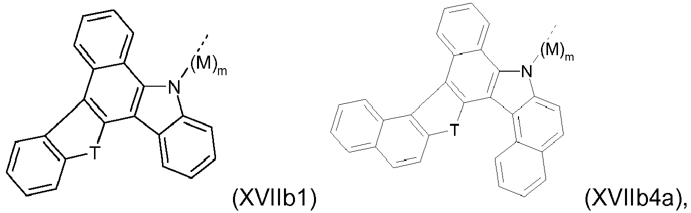 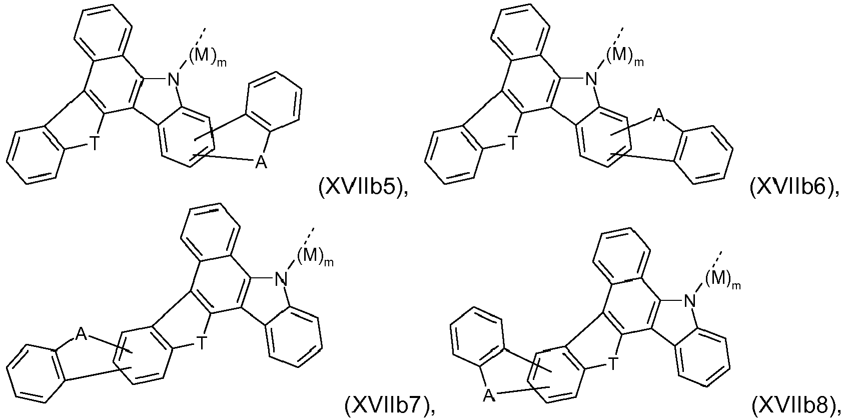 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

According to a further preferred embodiment of the present invention, R²⁶ in the N-heteroaryl groups according to general formulae (XIII), (XIV), (XV), (XVI) and (XVII) may correspond to the following formula (XVIII) 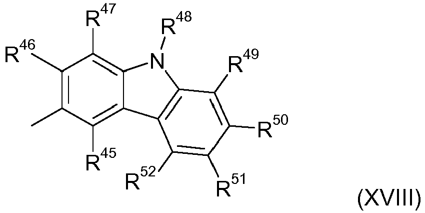
wherein R⁴⁵, R⁴⁶, R⁴⁷, R⁴⁸, R⁴⁹, R⁵⁰, R⁵¹ and R⁵² are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
at least two of R⁴⁶, R⁴⁷ R⁴⁹, R⁵⁰, R⁵¹ or R⁵², if present at adjacent carbon atoms, together form at least one C₆-C₁₈aryl or C₁-C₁₈heteroaryl ring or ring system,
wherein E and D have the same meanings as mentioned above.

Particularly preferred R⁴⁵, R⁴⁶, R⁴⁷, R⁴⁹, R⁵⁰, R⁵¹ and R⁵² are independently of each other H, E, a unsubstituted C₆-C₁₈aryl group or a C₆-C₁₈aryl group substituted with at least one group E, or a C₁-C₂₅alkyl group, which is unsubstituted or substituted by at least one group E and/or interrupted by at least one group D, preferably H, phenyl, biphenyl, naphthyl, phenanthryl or dimethylfluorenyl.

R⁴⁸ is particularly preferred H, a unsubstituted C₆-C₁₈aryl group or a C₆-C₁₈aryl group substituted with at least one group E, preferably phenyl, biphenhyl or naphthyl.

Further preferred at least two of R⁴⁶, R⁴⁷, R⁴⁹, R⁵⁰, R⁵¹ or R⁵², most preferably R⁵¹ and R⁵² if present at adjacent carbon atoms, together form at least one C₆-C₁₈aryl or C₁-C₁₈heteroaryl ring or ring system, most preferably a fused phenyl ring.

According to a particularly preferred embodiment, in general formula (XVIII) R⁴⁵, R⁴⁶, R⁴⁷, R⁴⁹, R⁵⁰, R⁵¹ and R⁵² are H and R⁴⁸ is phenyl.

According to a further particularly preferred embodiment, in general formula (XVIII) R⁴⁵, R⁴⁶, R⁴⁷, R⁴⁹ and R⁵⁰ are H, R⁵¹ and R⁵² form a fused phenyl ring and R⁴⁸ is phenyl.

In general, m is 1, 2, 3 or 4, wherein m describes the number of groups M present. If m is 0, no group M is present, but the N-heteroaryl group according to general formula (XII) is directly attached to carbon atom within the skeleton of the compound of general formula (I). Preferably, m is 0, 1, 2 or 3, more preferably, m is 0 or 1.

Preferably, M is a C₆-C₄₀ arylene group which is unsubstituted, a C₁-C₂₄heteroarylene group which is unsubstituted or a C₁-C₂₅ alkylene group which unsubstituted. Particularly preferred, M is a C₆-C₁₈ arylene group which is unsubstituted, most preferably a phenylene group.

Preferably, R²⁶ is independently of each other selected from H, a C₆-C₄₀aryl group which is unsubstituted or substituted by at least one group E and a C₁-C₃₀heteroaryl group which is unsubstituted or substituted by at least one group E.

According to a very preferred embodiment of the present invention, m is 0, and at least two of R²⁶, if present at adjacent carbon atoms, form at least one five or six membered, substituted or unsubstituted, saturated, unsaturated, aromatic or heteroaromatic ring or ring system, without further substituents R²⁶.

According to a further very preferred embodiment of the present invention, m is 1, M is M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring or ring system, without further substituents R²⁶.

The present invention therefore preferably relates the compound according to the present invention, wherein m is 1, M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring, wherein E has the meanings as mentioned above.

According to the present invention, in the compounds according to general formula (I), in particular in compounds according to general formulae (Ia) and (Ib), X⁴, X⁵, X⁶, X⁷, X⁸, X⁹ and X¹⁰ can independently of each other be N, CR⁴, CR⁵, CR⁶, CR⁷, CR⁸, CR⁹ or CR¹⁰, wherein R⁴ to R¹⁰ have the meanings as mentioned above. According to a preferred embodiment of the present invention none of X⁴, X⁵, X⁶, X⁷, X⁸, X⁹ and X¹⁰ is N, i.e. X⁴, X⁵, X⁶, X⁷, X⁸, X⁹ and X¹⁰ are CR⁴, CR⁵, CR⁶, CR⁷, CR⁸, CR⁹ respectively CR¹⁰, wherein R⁴ to R¹⁰ have the meanings as mentioned above.

The present invention therefore preferably relates to compounds according to formula (I), preferably (Ia) and (Ib), more preferably (Ia1) and (Ib1) as mentioned above, wherein independently of each other R², R⁴, R⁵, R⁶, R⁷, R⁸, R⁹, R¹⁰ and R¹¹ have the same meanings as mentioned above. Most preferably, R², R⁴, R⁵, R⁶, R⁷, R⁸, R⁹, and R¹⁰ are H, wherein one of R², R⁴, R⁵, R⁶, R⁷, R⁸, R⁹, R¹⁰ in the compound of formula (I) is a N-heteroaryl group according to general formulae (XII), (XIII), (XIIIa), (XIIIb), (XIV), (XIVb), (XV), (XVa), (XVb), (XVI), (XVIa), (XVIb), (XVII), (XVIIb), (XXI), (XXIa), (XXIb) or (XXIc). Further most preferably, R⁴, R⁵, R⁶, R⁷, R⁸, R⁹ and R¹⁰ are H and R² is a N-heteroaryl group according to one of the formulae (XII), (XIII), (XIIIa), (Xlllb), (XIV), (XIVb), (XV), (XVa), (XVb), (XVI), (XVIa), (XVIb), (XVII), (XVIIb), (XXI), (XXIa), (XXIb) or (XXIc).

Particularly preferred molecules according to general formula (I) according to the present invention are shown in the following.

Particularly preferred compounds of formula (I), wherein Y is O:

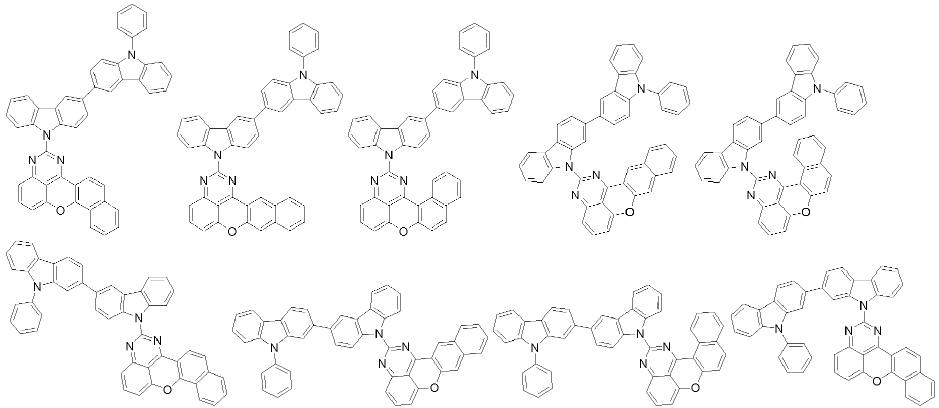

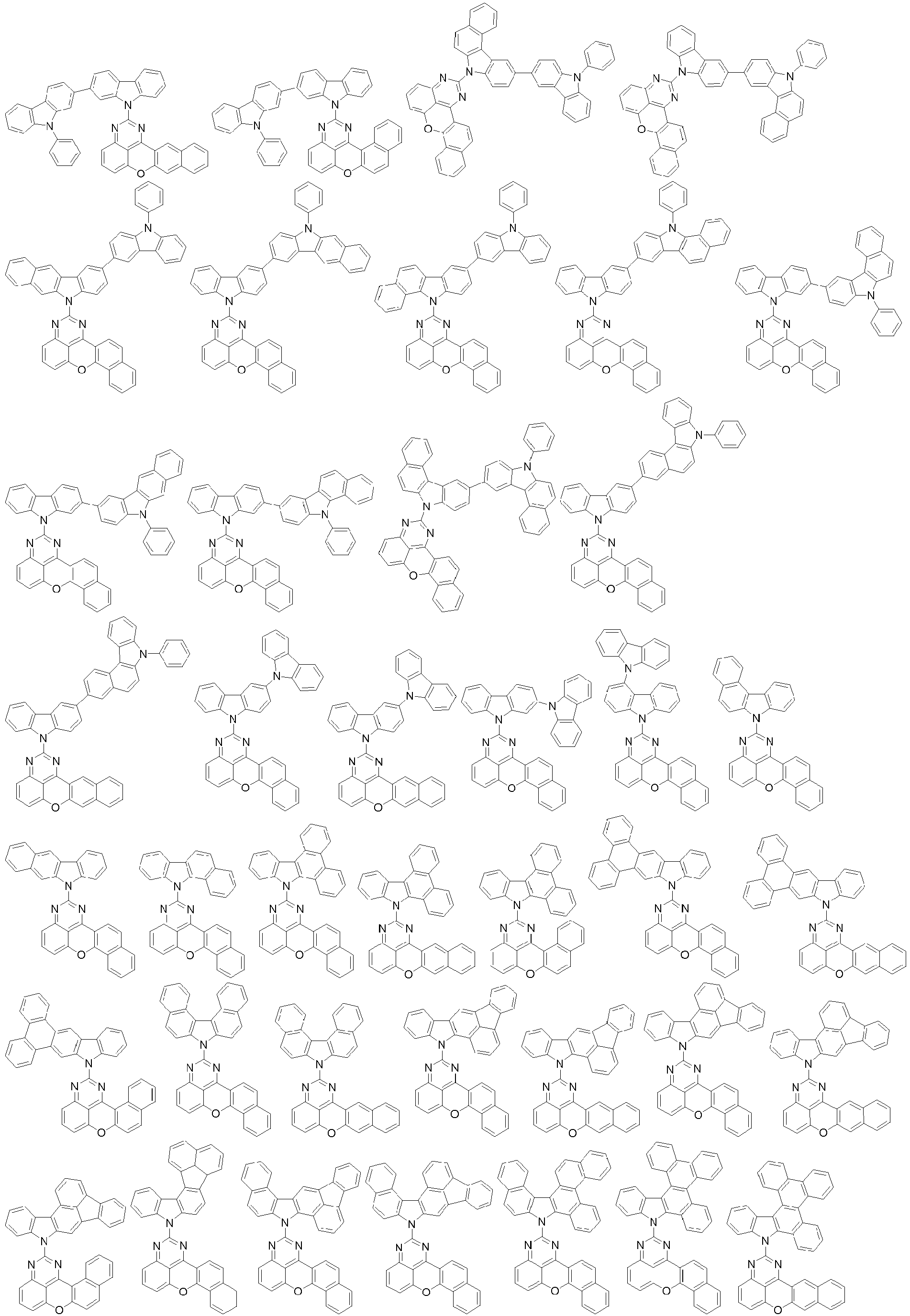

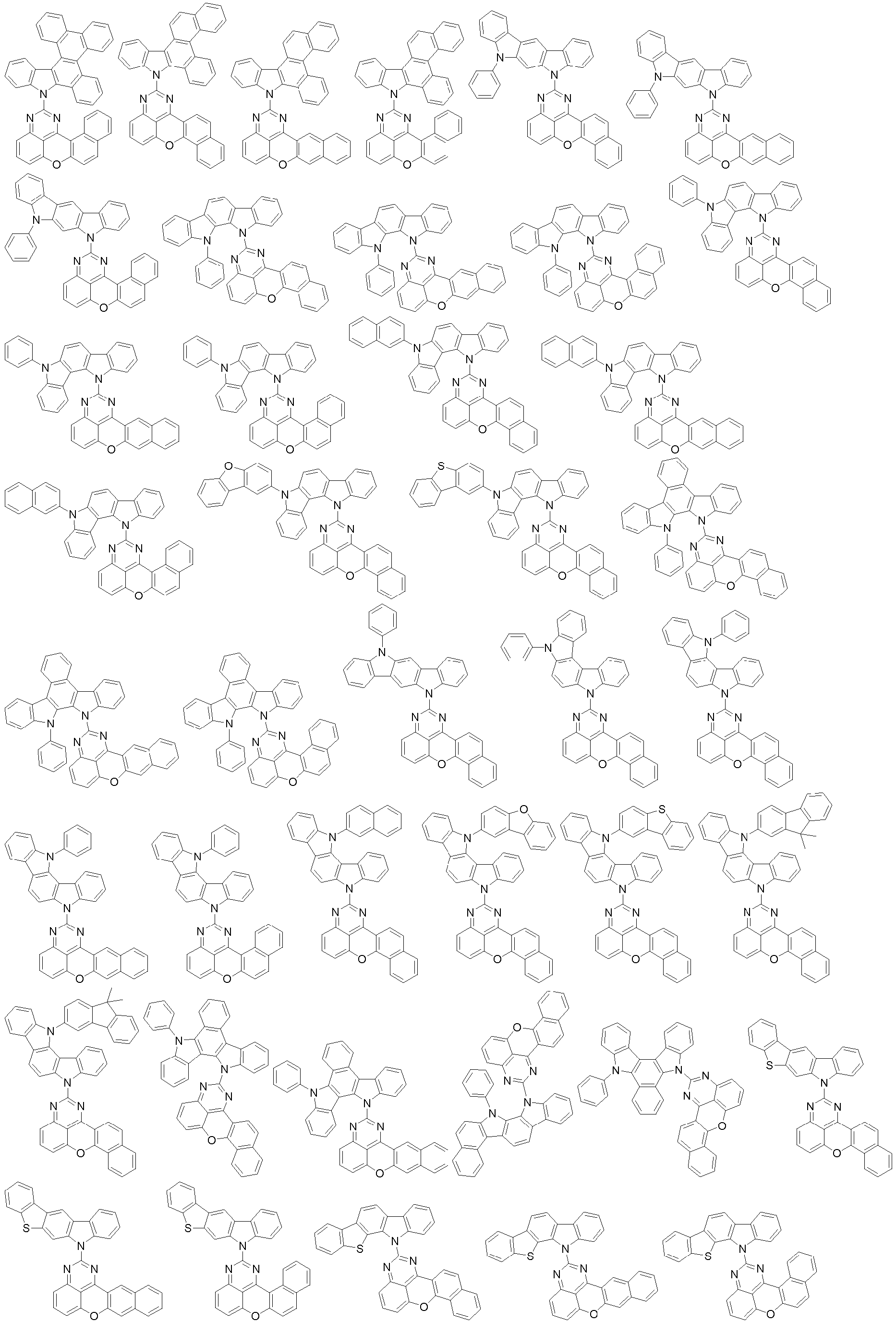

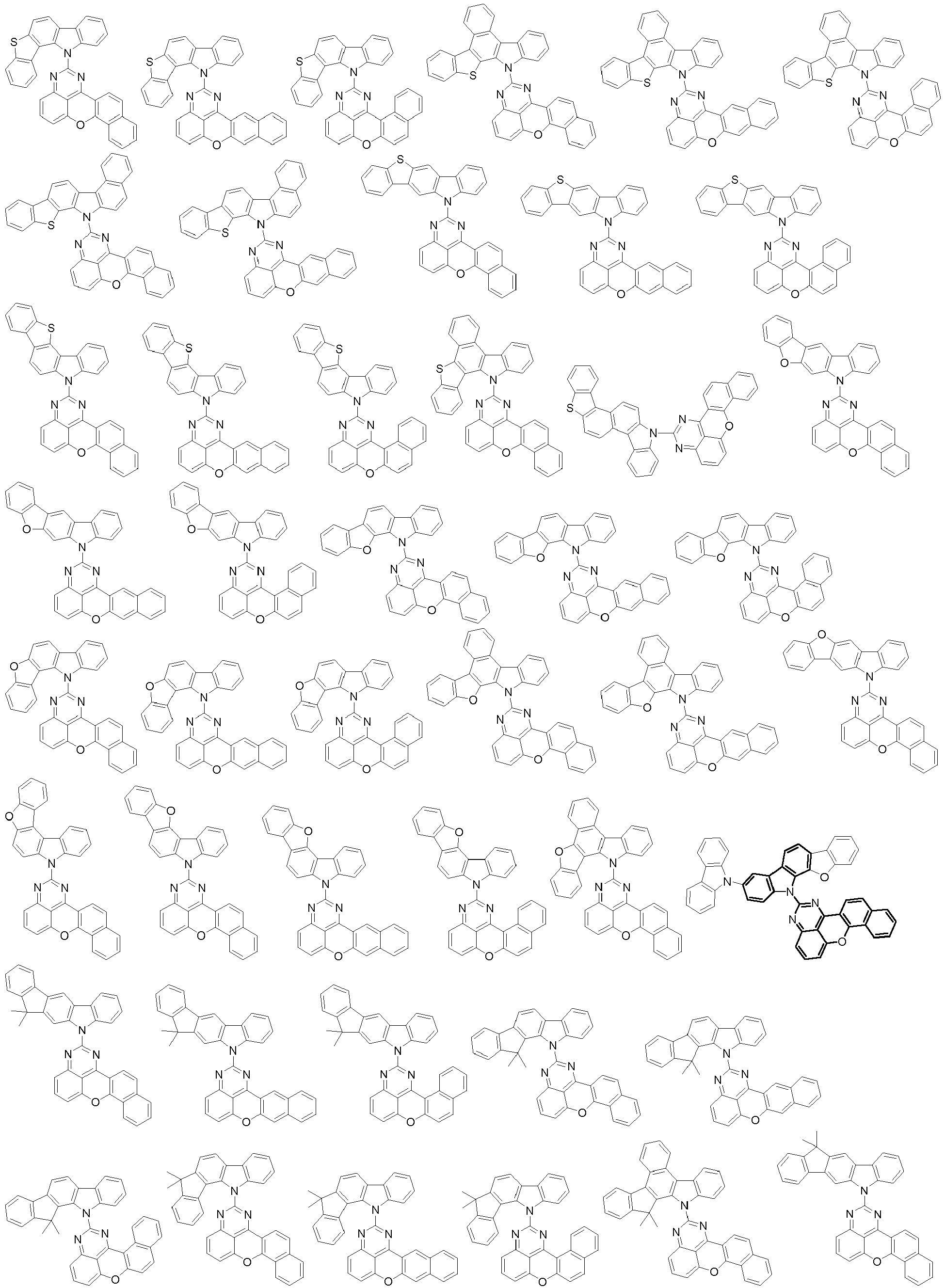

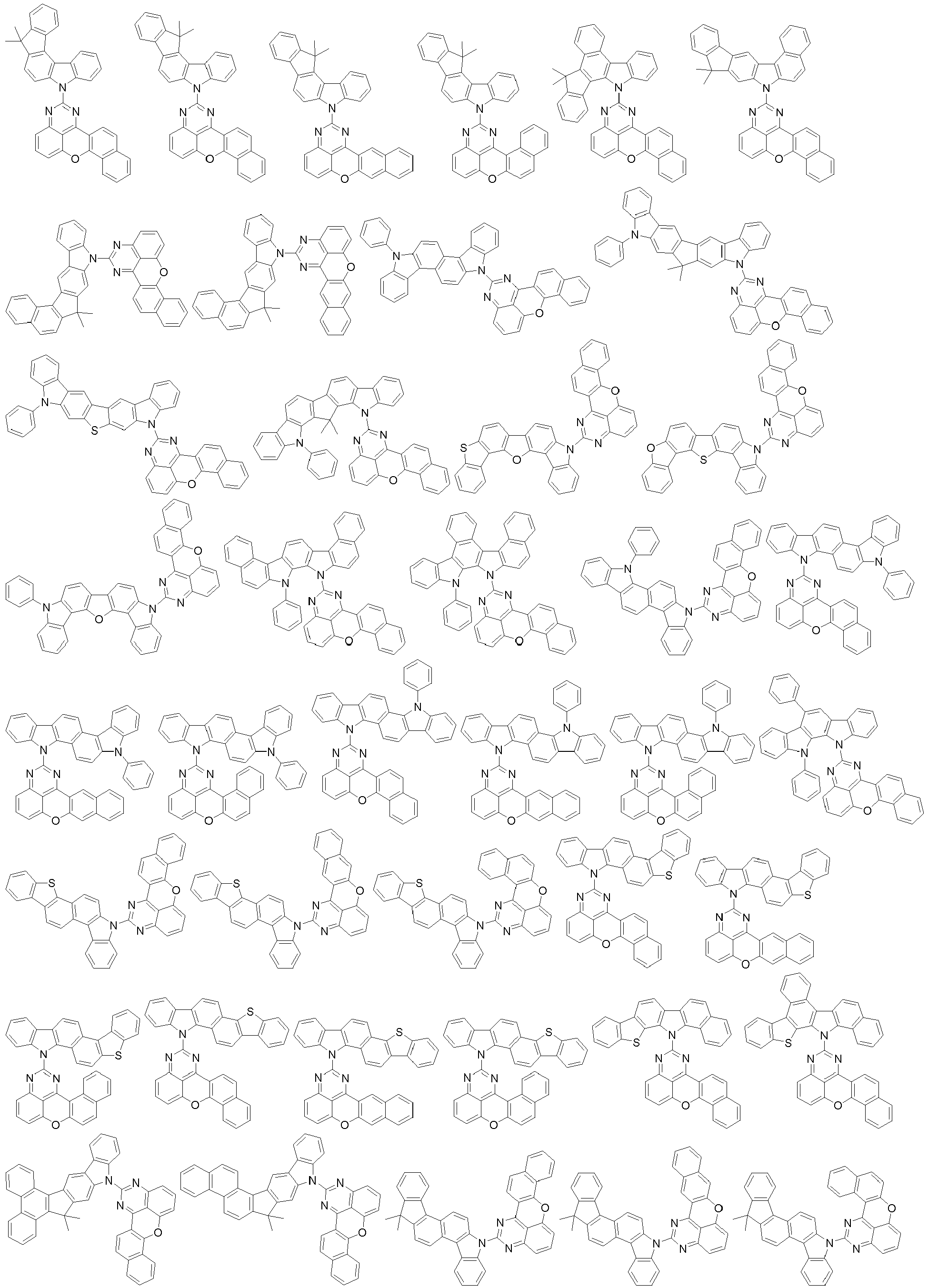

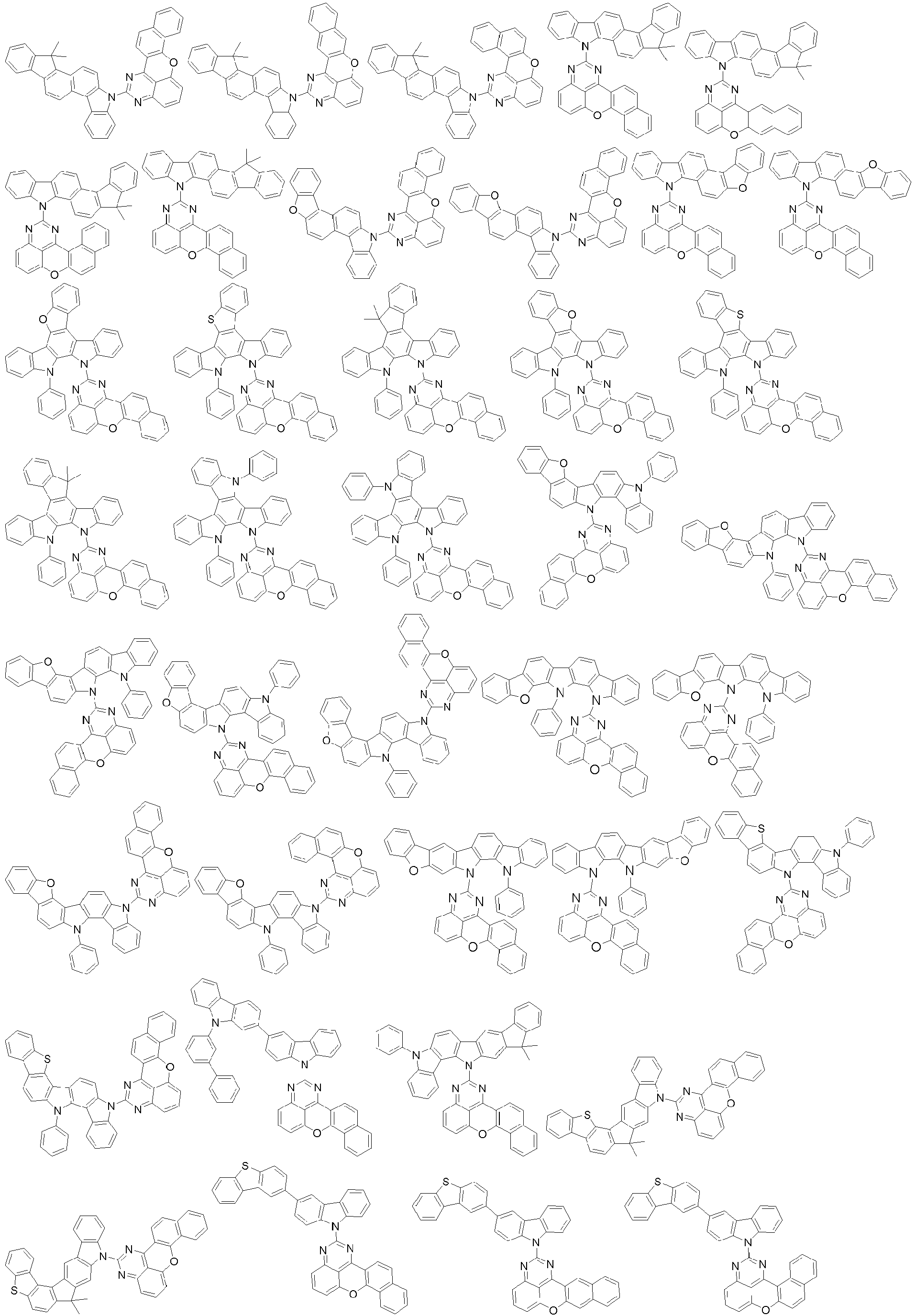

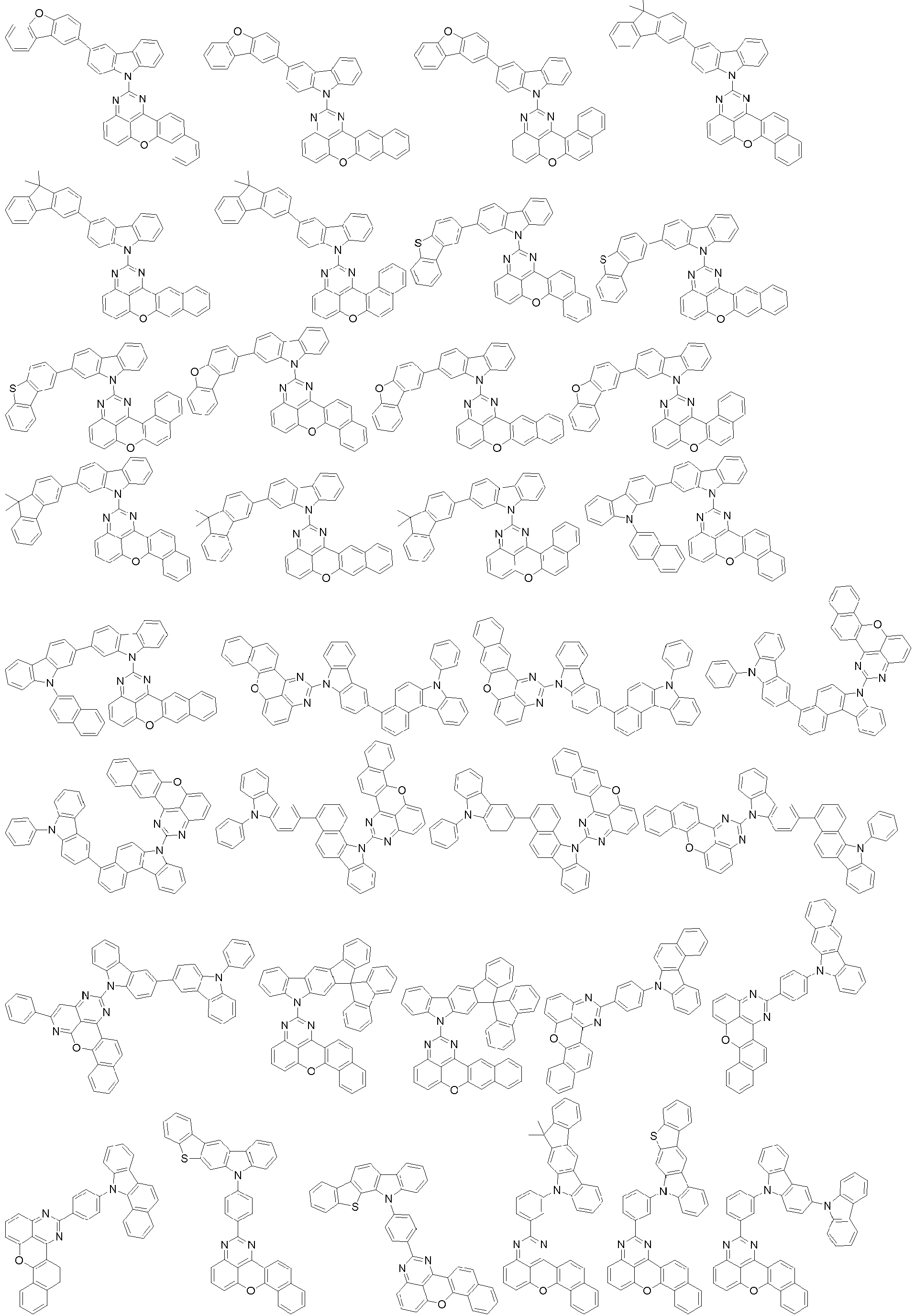

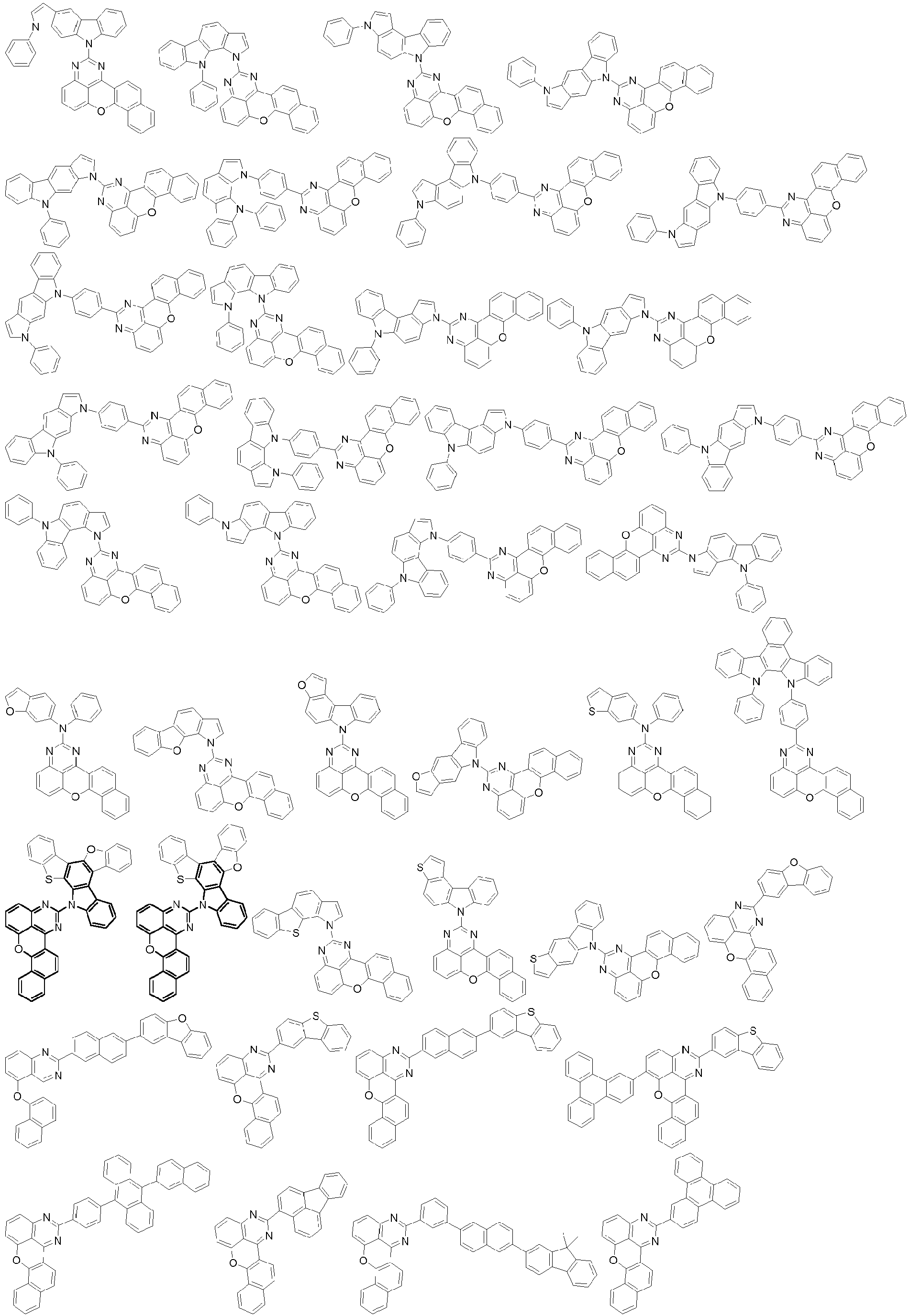

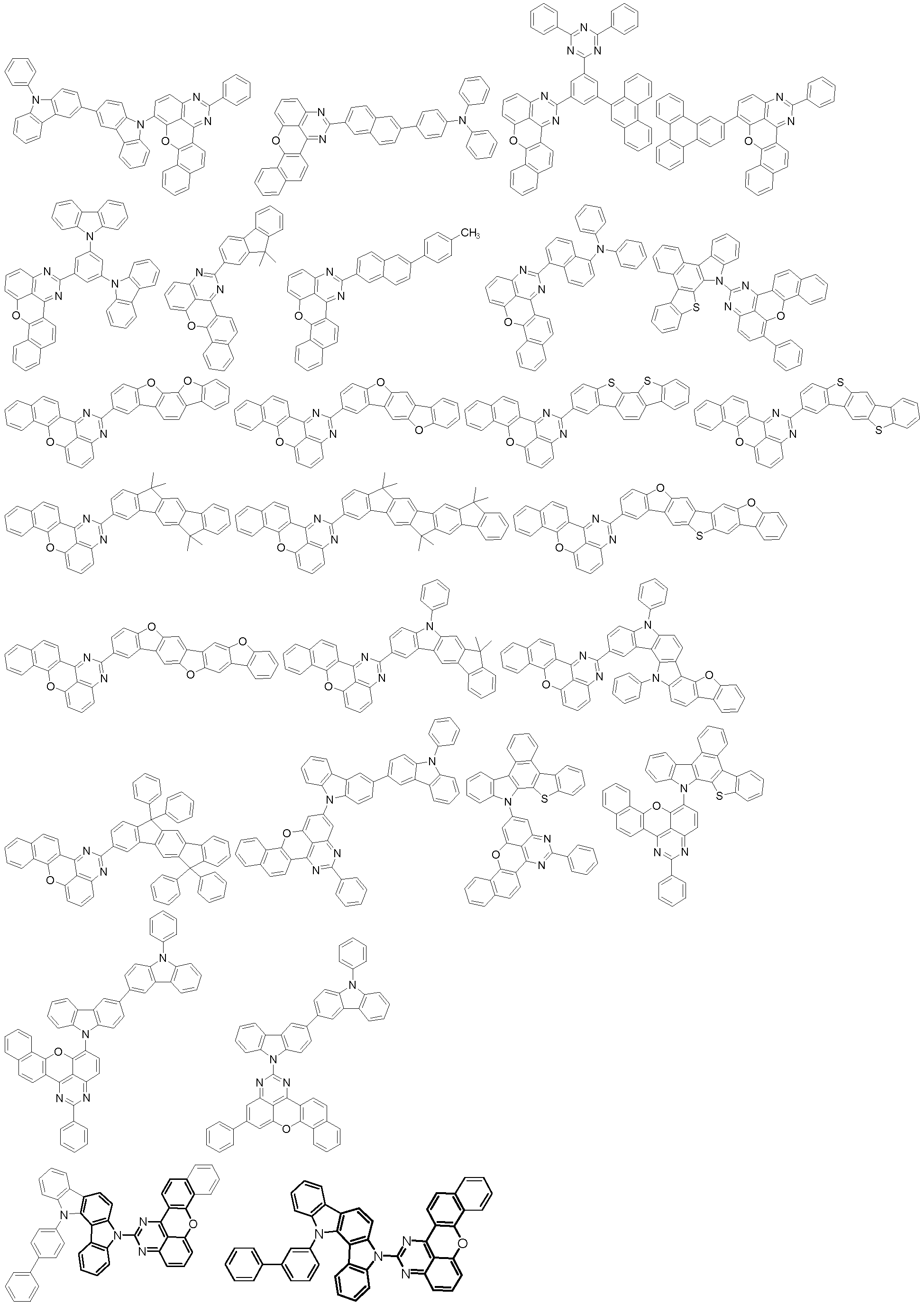

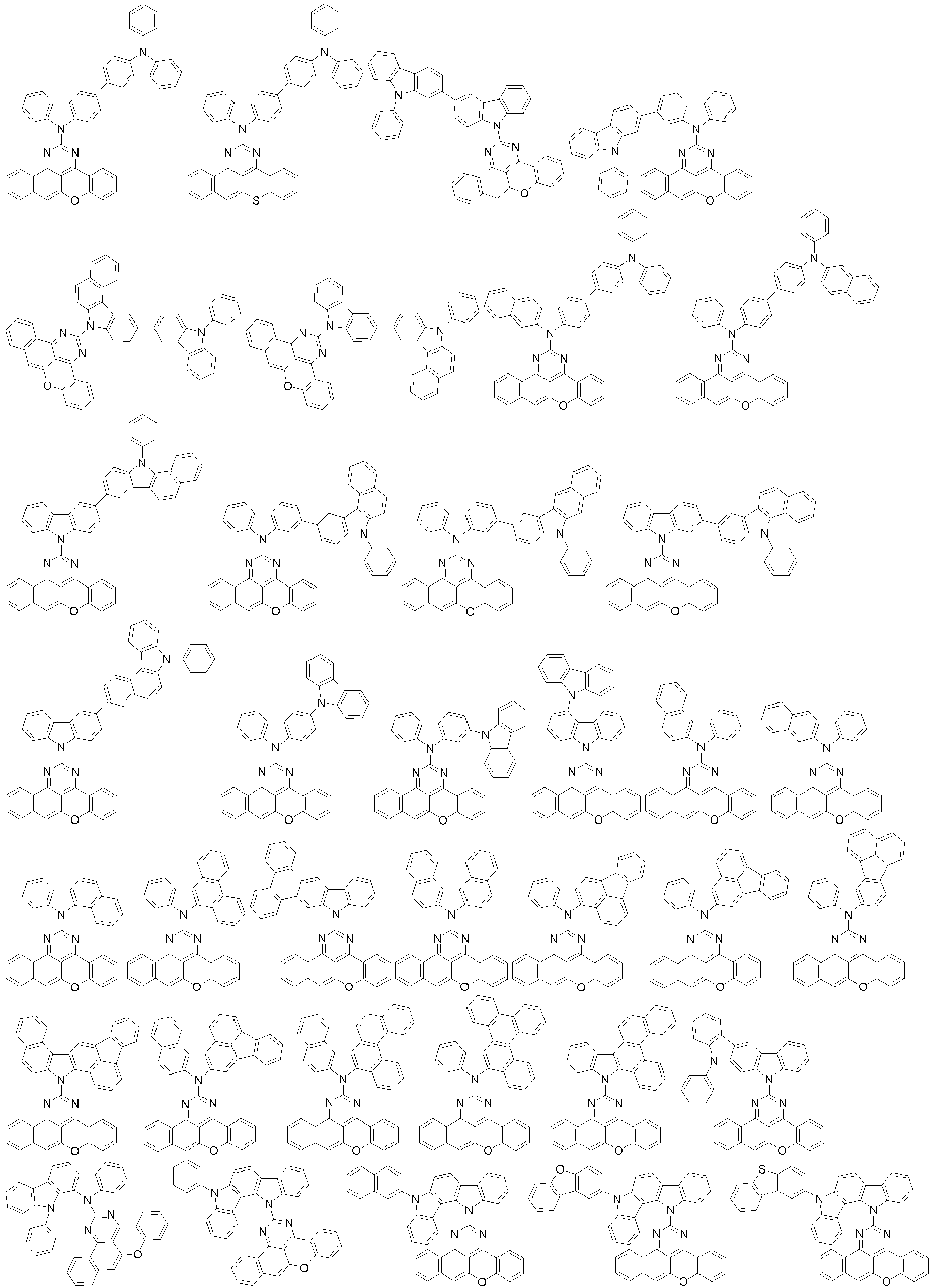

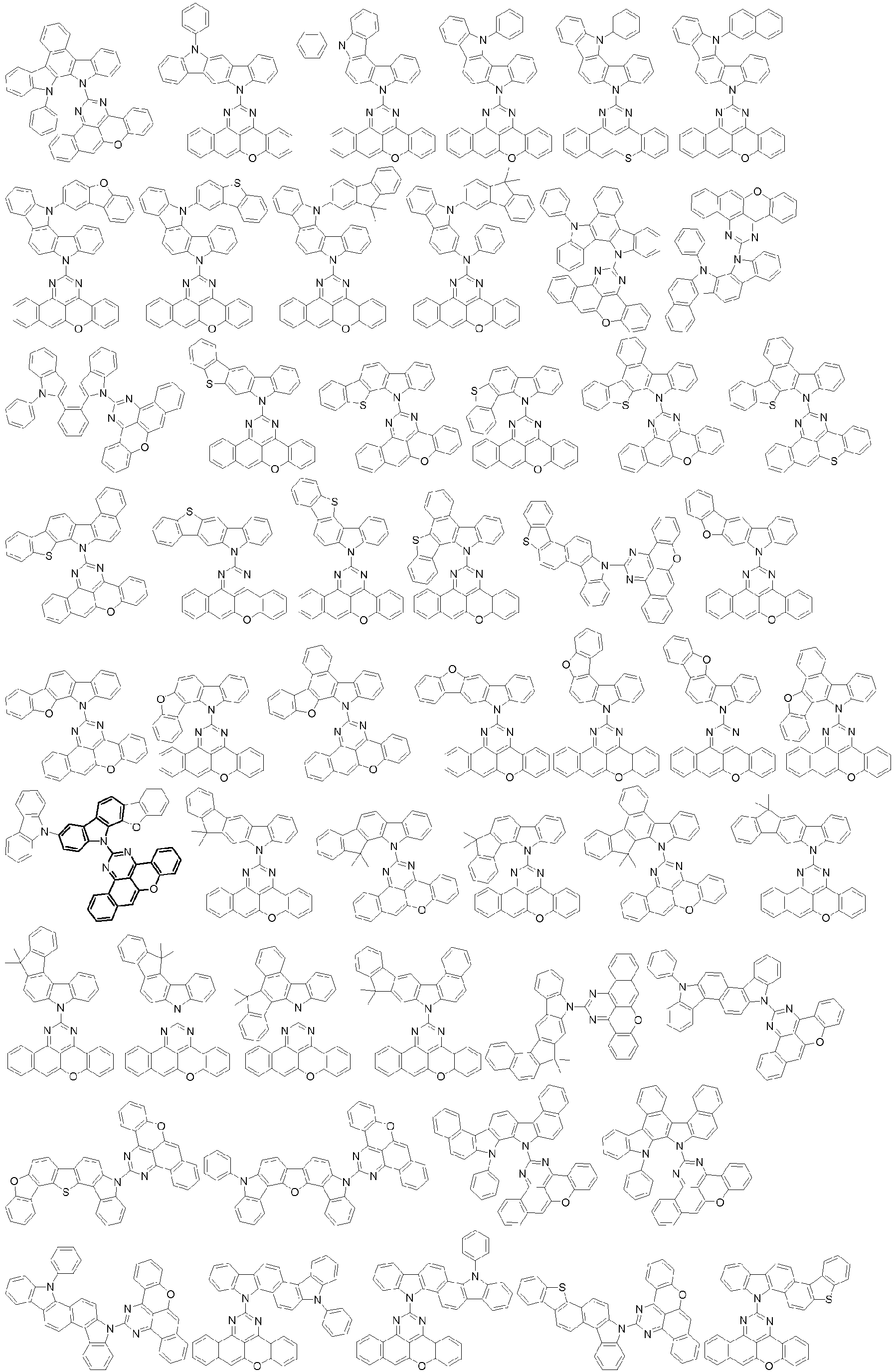

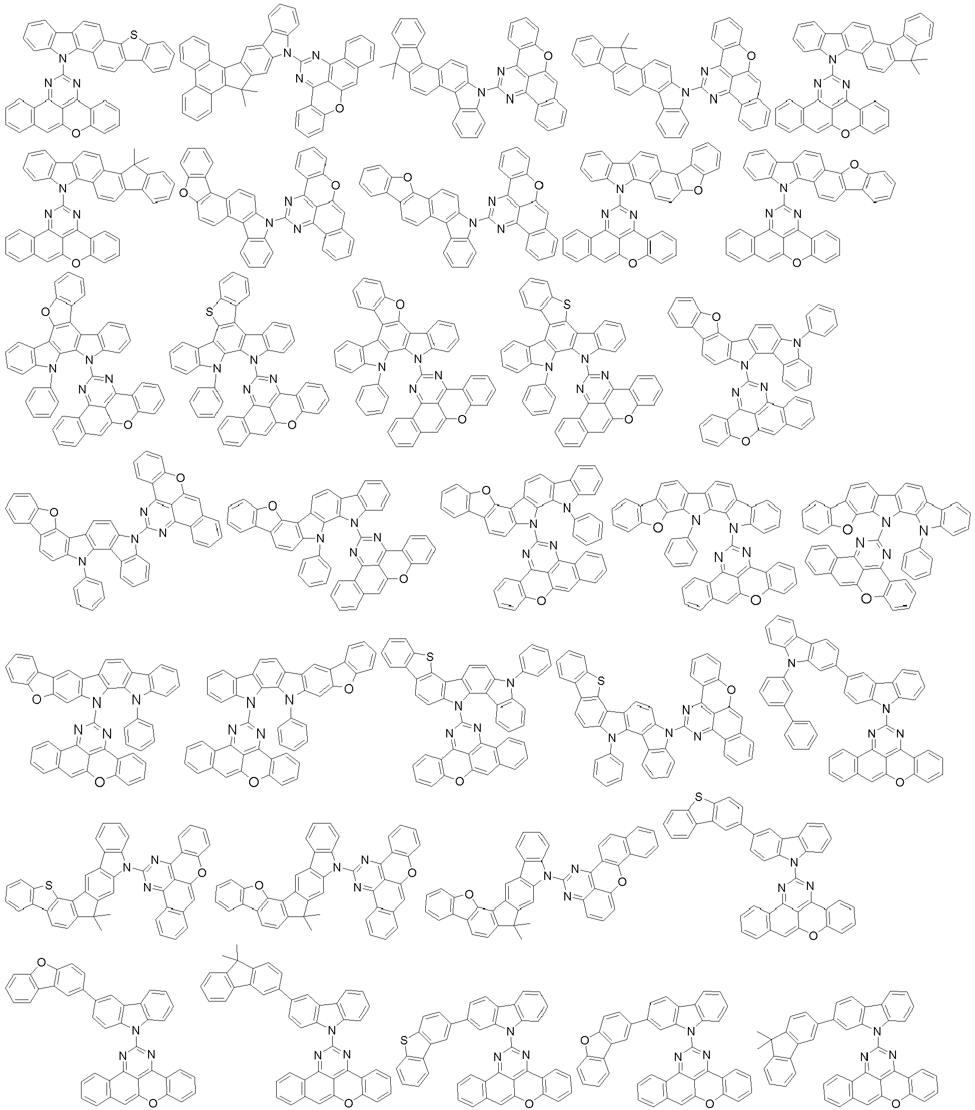

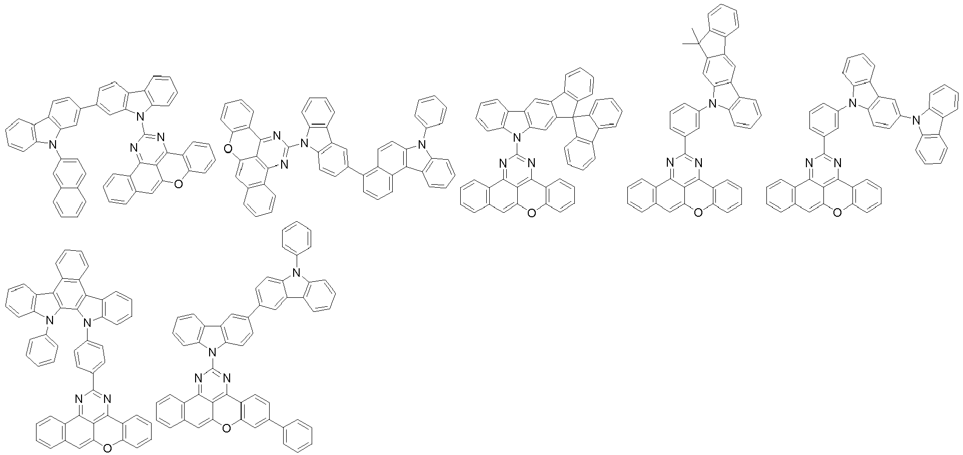

Particularly preferred compounds of formula (I), wherein Y is S:

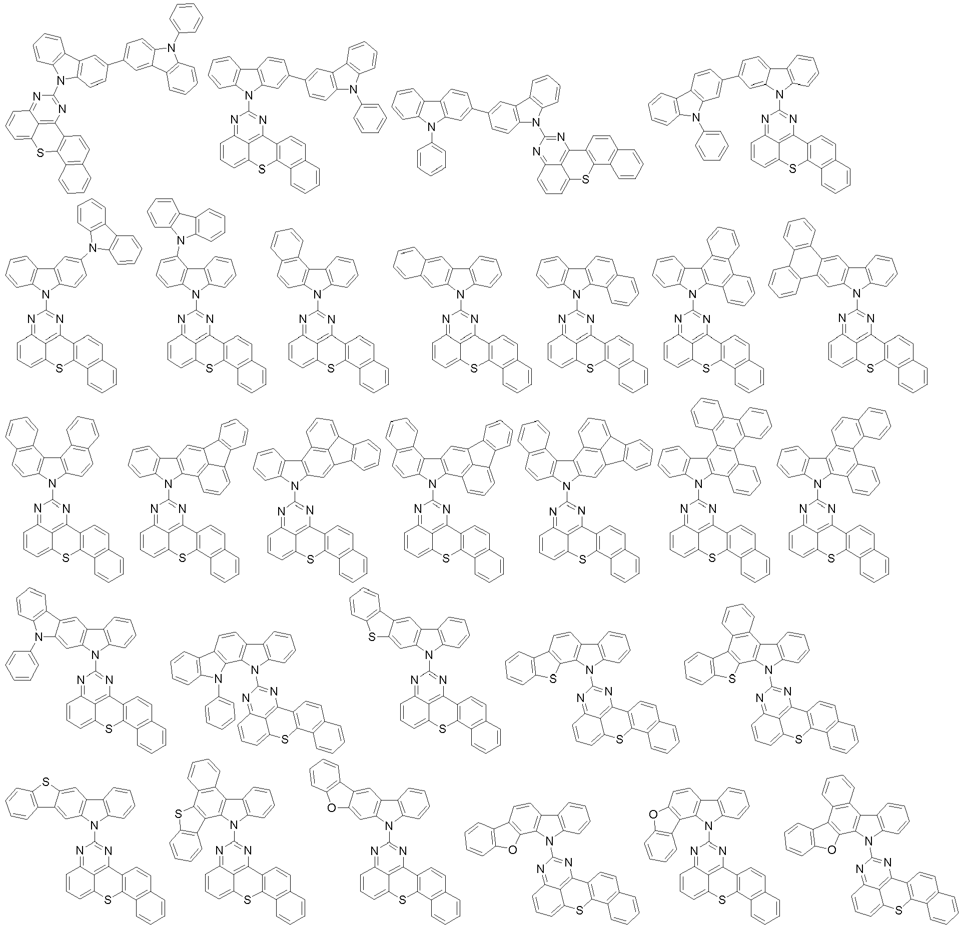

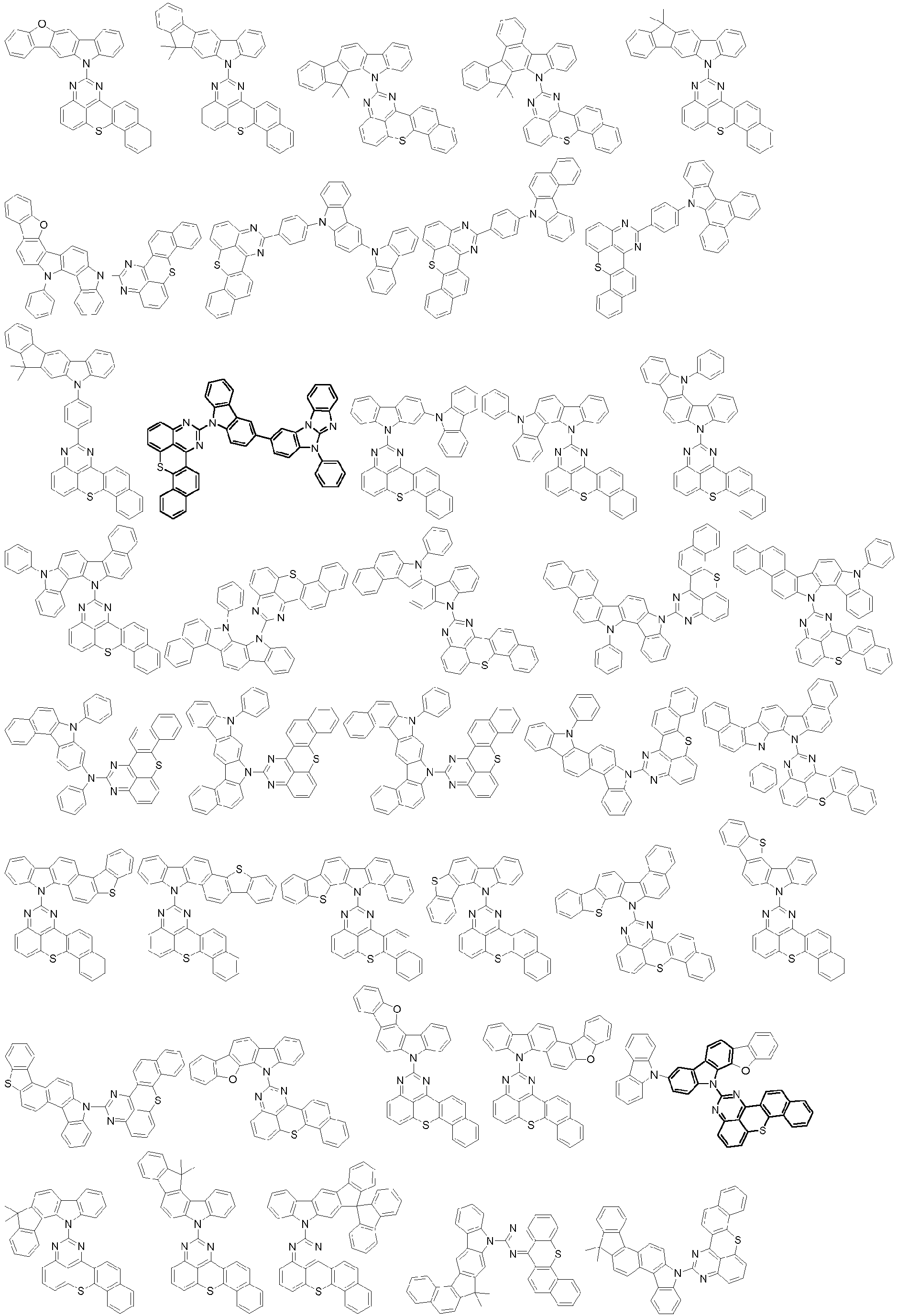

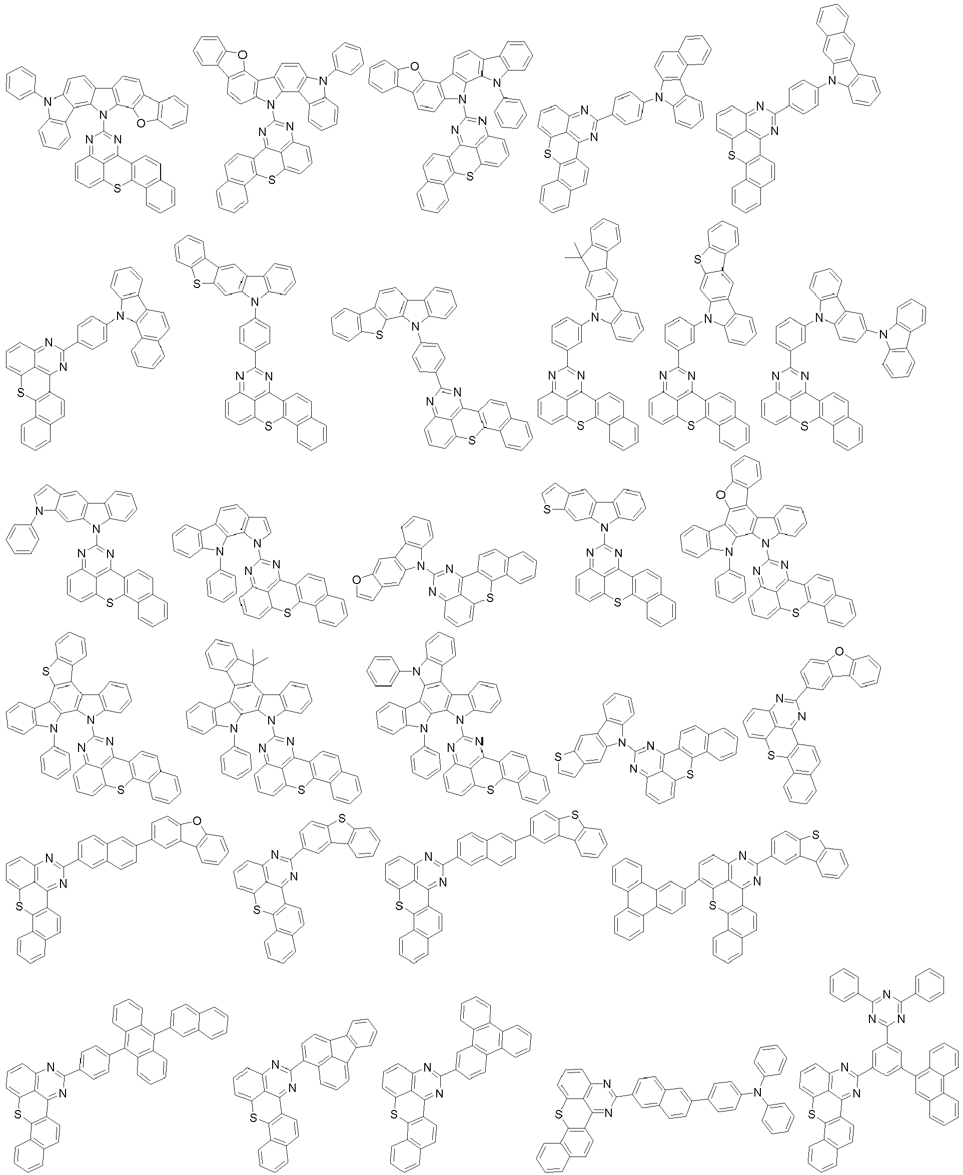

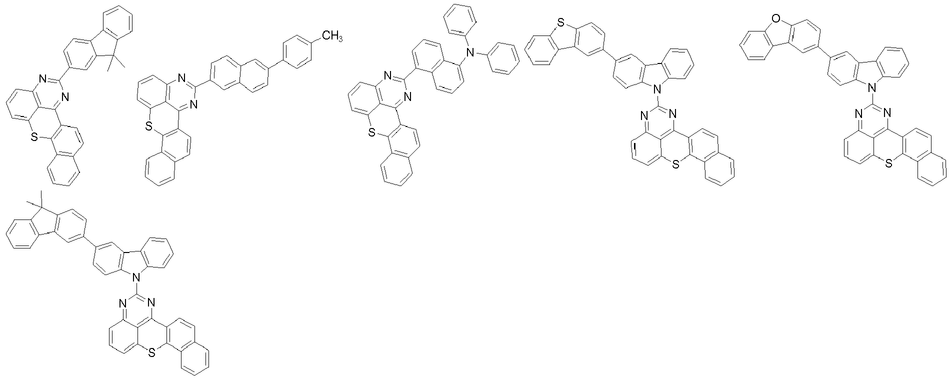

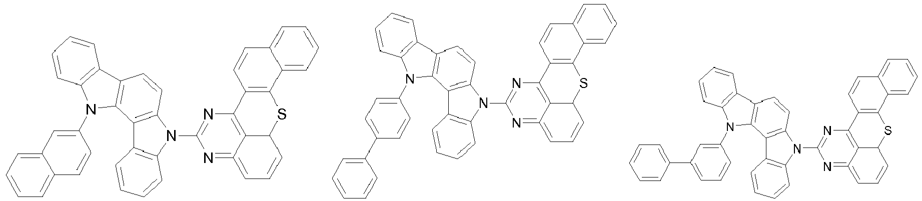

### Preparation of the compounds of formula (I)

The present invention also relates to a process for the preparation of a compound according to general formula (I) as defined above, at least comprising step (A)
(A) coupling of a compound according to general formula (VI') 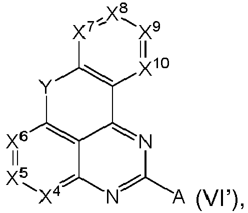
   with a compound of formula R²-H to obtain a compound according to general formula (I), wherein A is a selected from Cl, Br, I, F, OSO₂CH₃, and OSO₂CF₃ or OSO₂C₆H₄CH₃, and
   wherein Y, R², X⁴, X⁵, X⁶, X⁷, X⁸, X⁹ and X¹⁰ have the same meanings as defined above. Preferfably, R² is a N-heteroaryl group according to one of the formulae (XII), (XIII), (XIIIa), (XIIIb), (XIV), (XIVb), (XV), (XVa), (XVb), (XVI), (XVIa), (XVIb), (XVII), (XVIIb), (XXI), (XXIa), (XXIb) or (XXIc).

Step (A) of the process according to the present invention can in general be conducted by any method and under any conditions that are known to provide the desired product by a person having ordinary skill in the art. For example, step (A) of the process according to the present invention can be coupling reactions that are known to a person having ordinary skill in the art, for example reactions using palladium or copper catalysts.

The present invention therefore preferably relates to the process according to the present invention, wherein step (A) is a coupling reaction that is conducted in the presence of a palladium and/or copper catalyst.

According to one preferred embodiment, step (A) of the process according to the present invention can be conducted using the so called Buchwald-Hartwig reaction which is known to the skilled artisan and which is, for example, mentioned in Adv. Synth. Catal., 2006, 23, J. Organomet. Chem., 1999, 125, Chem. Sci., 2011, 27.

According to another preferred embodiment, step (A) of the process according to the present invention can be conducted using the so called Suzuki reaction which is known to the skilled artisan and which is, for example, mentioned in Chem. Soc. Rev., 2014, 3525, Angew. Chem. Int. Ed., 2009, 6954.

According to another preferred embodiment, step (A) of the process according to the present invention can be conducted using the so called Ulmann reaction which is known to the skilled artisan and which is, for example, mentioned in Chem. Rev., 1995, 2457, "Boronic Acids" Wiley-VCH, 2005.

In particular the coupling according to step (A) of the process according to the present invention is conducted in the presence of at least one basic compound, for example selected from the group consisting of alkali metal salts of alcohols having 1 to 6 carbon atoms, in particular sodium tert-butoxide, potassium tert-butoxide, potassium carbonate, cesium carbonate, rubidium carbonate or potassium phosphate.

The reaction is preferably conducted in at least one aprotic, organic solvent. Preferred are aromatic solvents, for example selected from the group consisting of toluene, benzene, xylene, mesitylene and mixtures thereof.

As a catalyst particularly preferably a combination of at least one Lewis acid and of at least one palladium compound is used. Particularly preferably, a combination of at least one boron comprising complex and at least one palladium salt is used, for example a combination of tert-Bu₃P-HBF₄ and Pd₂(dba)₃, wherein dba means dibenzylideneacetone. Other suitable ligands and/or palladium comprising reagents are mentioned in the above mentioned scientific papers.

The reaction is conducted at a temperature that is high enough to obtain the desired compound in high yield and high selectivity, for example at 40 to 160 °C, preferably at 60 to 140 °C, particularly preferably at 70 to 120 °C.

The reaction is conducted for a time that is long enough to obtain the desired compound in high yield and high selectivity, for example for 1 to 6 h, preferably for 2 to 4 h, particularly preferably for 3 h.

After the reaction is completed, the reaction mixture can be worked up according to methods that are known to the skilled artisan, for example extraction, filtration, recrystallization, chromatography etc.

The reaction product can be analyzed, for example, by proton- or carbon-NMR, mass spectrometry etc.

The substrates that are used in step (A) of the process according to the present invention, i.e. compounds according to general formula (VI') and compounds according to general formula R²-H, wherein R² has the meanings as mentioned above, can be made by methods that are known to a person having ordinary skill in the art. The compound according to general formula R²-H, is, for example, commercially available.

A preferred reaction sequence to obtain the compound of formula (VI') is shown in the following:
Step (A01): A compound according to general formula (VII) is transferred to a compound of general formula (VIII). 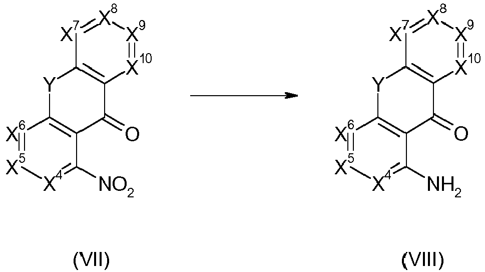 wherein Y, X⁴, X⁵, X⁶, X⁷, X⁸, X⁹ and X¹⁰ have the same meanings as defined above. Y is preferably O.

In particular the reaction according to step (A01) of the process according to the present invention is conducted in the presence of at least one reducing compound, for example selected from the group consisting of HCO₂NH₄ and mixtures thereof.

The reaction is preferably conducted in at least one organic solvent. Preferred are alcohols, for example selected from the group consisting of ethanol, isopropanol and mixtures thereof

Preferably step (A01) is conducted in the presence of a catalyst. As a preferred catalyst palladium is used. Particularly preferably, palladium on carbon is used.

The reaction is conducted at a temperature that is high enough to obtain the desired compound in high yield and high selectivity, for example at 40 to 160 °C, preferably at 60 to 120 °C, particularly preferably at reflux temperature of the solvent.

The reaction is conducted for a time that is long enough to obtain the desired compound in high yield and high selectivity, for example for 0.2 to 6 h, preferably for 0.5 to 3 h.

After the reaction is completed, the reaction mixture can be worked up according to methods that are known to the skilled artisan, for example extraction, filtration, recrystallization, chromatography etc.

Step (A02): The compound according to general formula (VIII) is then transferred to a compound of general formula (IX). 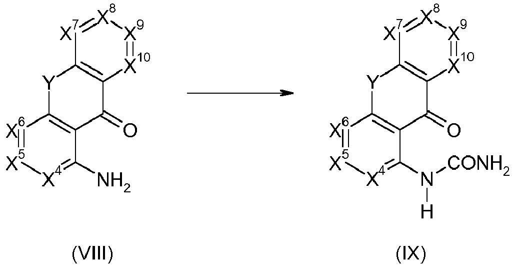 wherein Y, X⁴, X⁵, X⁶, X⁷, X⁸, X⁹ and X¹⁰ have the same meanings as defined above. Y is preferably O.

In particular the reaction according to step (A02) of the process according to the present invention is conducted in the presence of KOCN.

The reaction is preferably conducted in at least one acidic organic solvent. Preferred are carboxylic acids, for example acetic acid.

The reaction is conducted at a temperature that is high enough to obtain the desired compound in high yield and high selectivity, for example at 10 to 40 °C, preferably at room temperature, i.e. 20 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Step (A03): The compound according to general formula (IX) is then transferred to a compound of general formula (X). 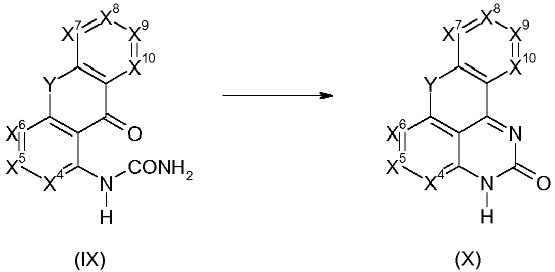 wherein Y, X⁴, X⁵, X⁶, X⁷, X⁸, X⁹ and X¹⁰ have the same meanings as defined above. Y is preferably O.

In particular the reaction according to step (A03) of the process according to the present invention is conducted in the presence of at least one basic compound selected from the group consisting of KOH, NaOH and mixtures thereof.

The reaction is preferably conducted in at least one organic solvent, for example alcohols like ethanol, isopropanol or mixtures thereof

The reaction is conducted at a temperature that is high enough to obtain the desired compound in high yield and high selectivity, for example at 50 to 150 °C, preferably at reflux temperature of the solvent.

The reaction is conducted for a time that is long enough to obtain the desired compound in high yield and high selectivity, for example for 0.1 to 2 h, preferably for 0.3 to 1 h.

After the reaction is completed, the reaction mixture can be worked up according to methods that are known to the skilled artisan, for example extraction, filtration, recrystallization, chromatography etc.

Step (A04): The compound according to general formula (X) is then transferred to a compound of general formula (XI). 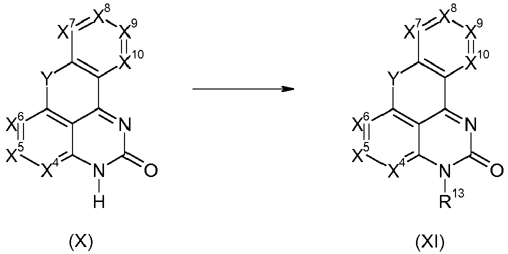 wherein Y, X⁴, X⁵, X⁶, X⁷, X⁸, X⁹ and X¹⁰ have the same meanings as defined above and R¹³ is linear or branched C₁-C₆alkyl group, preferably methyl. Y is preferably O.

In particular the reaction according to step (A04) of the process according to the present invention is conducted in the presence of at least one strongly basic compound, for example NaH.

The reaction according to step (A04) of the process according to the present invention is further conducted in the presence of at least one compound according to formula R¹³-B, wherein R¹³ has the meanings as mentioned above and B is selected from I, Br or Cl.

The reaction is preferably conducted in at least one organic solvent, for example dimethylformamide or mixtures thereof

The reaction is conducted at a temperature that is high enough to obtain the desired compound in high yield and high selectivity, for example at -20 to 20 °C, preferably at -10 to 10 °C, most preferably at 0 °C.

The reaction is conducted for a time that is long enough to obtain the desired compound in high yield and high selectivity, for example for 0.5 to 4 h, preferably for 1 to 3 h.

After the reaction is completed, the reaction mixture can be worked up according to methods that are known to the skilled artisan, for example extraction, filtration, recrystallization, chromatography etc.

Step (A05): The compound according to general formula (XI) is then transferred to a compound of general formula (VI'). 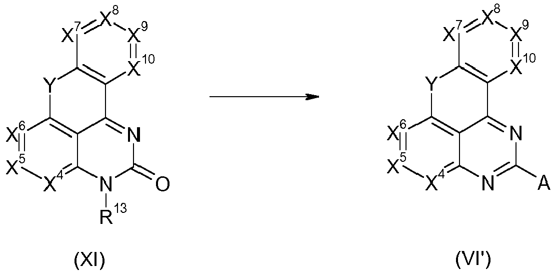 wherein Y, X⁴, X⁵, X⁶, X⁷, X⁸, X⁹, X¹⁰ and R¹³ have the same meanings as defined above. Y is preferably O.

In particular, the reaction according to step (A05) according to the present invention is conducted in the presence of at least one compound, which is able to introduce the moiety A into the molecule. According to the preferred case that A is Cl, chlorination agents are used, for example phosphorous comprising chlorination agents like POCl₃ or PCl₅ in step (A05) of the process according to the present invention

The reaction according to step (A05) is preferably conducted without any solvent, i.e. in substance.

The reaction is conducted at a temperature that is high enough to obtain the desired compound in high yield and high selectivity, for example at 40 to 160 °C, preferably at 60 to 120 °C, particularly preferably at 70 to 90 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Further detailed reaction conditions of this reaction scheme for obtaining the compound of formula (VI') can be taken from Carlos M. Martinez et al., J. Heterocyclic Chem., 44, 1035 (2007).

Particularly preferably, the process according to the present invention for the preparation of compounds according to general formula (I) comprises step (A01), followed by step (A02), followed by step (A03), followed by step (A04), followed by step (A05), followed by step (A).

According to another preferred embodiment of the process according to the present invention, the compound according to general formula (VI') can also be obtained directly from compound of general formula (X) using reaction conditions as mentioned in respect of step (A05). The present invention therefore preferably relates to the process according to the present invention, wherein the compound according to general formula (VI') can also be obtained directly from compound of general formula (X) as shown in the following (step (A05a):

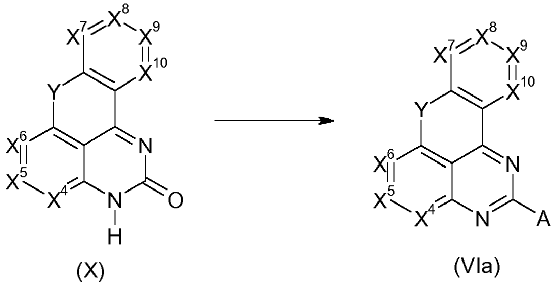

Therefore, particularly preferably, the process according to the present invention for the preparation of compounds according to general formula (I) comprises step (A01), followed by step (A02), followed by step (A03), followed by step (A05a), followed by step (A).

According to the further preferred embodiment that Y is S, the process according to the present invention for the preparation of compounds according to general formula (I) comprises step (A01), followed by step (A02), followed by step (A03), followed by step (A05a), followed by step (A).

Compound (VII) as mentioned above can be prepared in chemical reactions that are known to the skilled artisan. Preferred methods for the preparation of the compound according to general formula (VII) are mentioned in Yamagami, Isao et al., Jpn. Kokai Tokkyo Koh, 2008273906, 2008, Kralj, Ana et al., ChemMedChem, 9(1), 151-168, 2014 and Okabayashi, Ichizo and Iwata, Noriko, Chemical and Pharmaceutical Bulletin, 28(9), 2831-5, 1980.

The compound according to general formula (VI') as mentioned above can also be prepared by further methods that are explained in the following. For example, the compound according to general formula (VI') can be prepared from a compound according to general formula (VI*): 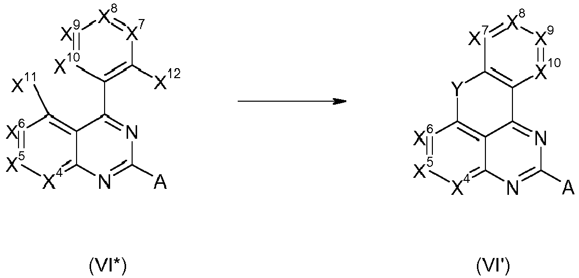 wherein Y, X⁴, X⁵, X⁶, X⁷, X⁸, X⁹, X¹⁰ and A have the same meanings as defined above, X¹¹ and X¹² are independently of each other F or OH, if Y is O, or X¹¹ and X¹² are independently of each other H or S(O)CH₃, if Y is S.

According to the preferred embodiment that Y is O, the following reaction is conducted (step (A06):

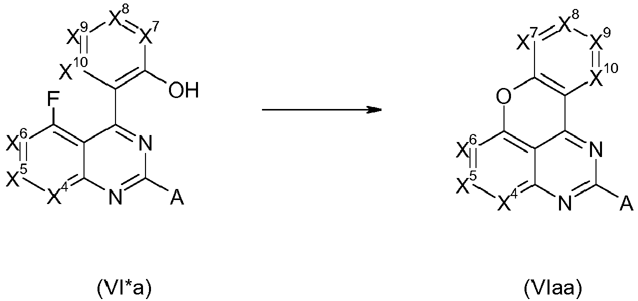

According to this preferred embodiment (step (A06)) the compound according to general formula (VI*a) is transferred into the compound according to general formula (Vlaa), wherein X⁴, X⁵, X⁶, X⁷, X⁸, X⁹, X¹⁰ and A have the same meanings as defined above.

The reaction is preferably conducted in the presence of at least one basic compound, for example selected from the group consisting of K₂CO₃, NaH and mixtures thereof. Further reaction conditions like temperature, solvent, reaction time etc. are known to the person having ordinary skill in the art.

The compound according to general formula (VI*a) can be obtained by any method that is known to the skilled artisan. Preferably the compound according to general formula (VI*a) is obtained according to the method described in Kang Hyun-Ju et al., WO 2014088290 or Welsh Dean M et al., Jpn. Kokai Tokkyo Koh, 2014183315, 2014.

According to the preferred embodiment that Y is S, the following reaction is conducted (step (A07):

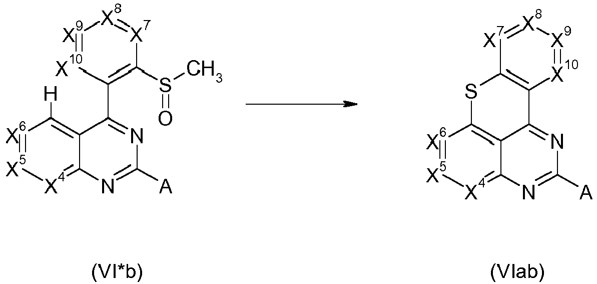

According to this preferred embodiment (step (A07)) the compound according to general formula (VI*b) is transferred into the compound according to general formula (Vlab), wherein X⁴, X⁵, X⁶, X⁷, X⁸, X⁹, X¹⁰ and A have the same meanings as defined above.

The reaction is preferably conducted in the presence of at least one acidic compound, for example selected from the group consisting of (CF₃SO₂)₂, CF₃SO₂OH, and mixtures thereof.

Further reaction conditions like temperature, solvent, reaction time etc. are known to the person having ordinary skill in the art.

### Compounds of formula (I*)

The object of the present invention is also achieved by a compound represented by the general formula (I*) 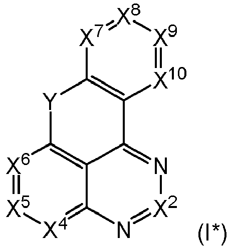 wherein
X² is CR²,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
X¹⁰ is CR¹⁰ or N,
Y is O or S,
R², R⁴, R⁵, R⁶, R⁷, R⁸, R⁹, and R¹⁰ are independently of each other selected from H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
at least two of R⁴, R⁵, R⁶, R⁷, R⁸, R⁹ or R¹⁰, if present at adjacent carbon atoms, together form at least one C₆-C₁₈aryl or C₂-C₁₈heteroaryl ring or ring system,
p is 0 or 1, q is 0 or 1, r is 0 or 1 and s is 0 or 1,
D is independently of each other -CO-, -COO-, -S-, -SO-, -SO₂-, -O-,-CR¹⁵=CR¹⁶-, -NR¹⁷-,-SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN,-SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₂₅alkyl group, which is unsubstituted or substituted by at least one -F,-CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; R¹⁵ and R¹⁶ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²¹ is independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₆-C₁₂aryl or by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₆-C₁₂aryl or by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein one of R², R⁴, R⁵, R⁶, R⁷, R⁸, R⁹, R¹⁰ in the compound of formula (I*) is a N-heteroaryl group according to general formula (XVI), preferably, R² in the compound of formula (I) is a N-heteroaryl group according to general formula (XVI); 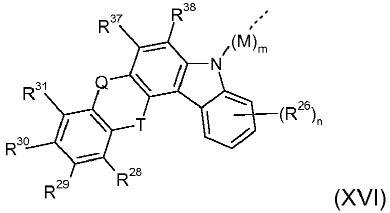
   wherein n is 0, 1, 2, 3 or 4, preferably 0, 1 or 2, more preferably 0 or 1, most preferably 0,
   m is 0, 1, 2, 3 or 4, preferably 0, 1 or 2, more preferably 0 or 1,
   M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
   R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
   or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
   R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl, or
   at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
   wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
   R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
   R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
   wherein the dotted line is a bonding site.

The compounds of formula (I) and the compounds of formula (I*) are suitable as material for electronic devices, preferably OLEDs, especially as a host material, a charge transporting mate-rial, charge and/or exciton blocking material, preferably as a host material, having a good overall performance, especially improved efficiency and/or driving voltage and preferably having a high triplet energy, preferably of > 2.0 eV.

Preferred embodiments of the compounds of formula (I*) are explained in the following.

Y in the general formula and (I*) is O or S, preferably O.

Therefore the present invention preferably relates to compounds according to the following general formulae (I*a) and (I*b), more preferably to compounds according to the following general formula (I*a), 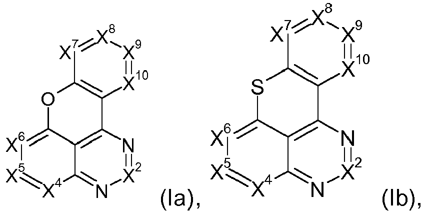 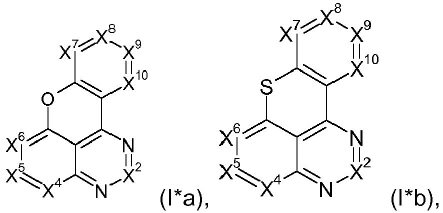 wherein independently of each other X², X⁴, X⁵, X⁶, X⁷, X⁸, X⁹, X¹⁰ and R¹¹ have the same meanings as mentioned above.

Preferably, 0, 1 or 2 of X⁴, X⁵, X⁶, X⁷, X⁸, X⁹, X¹⁰ in the compounds of formula (I*) are N, more preferably, 0 of X⁴, X⁵, X⁶, X⁷, X⁸, X⁹, X¹⁰ are N, i.e. X⁴, X⁵, X⁶, X⁷, X⁸, X⁹, X¹⁰ are CR⁴, CR⁵, CR⁶, CR⁷, CR⁸, CR⁹ and CR¹⁰.

More preferred compounds of formula (I*) are therefore compounds of the following general formulae (I*a1) and (I*b1), preferably (I*a1):

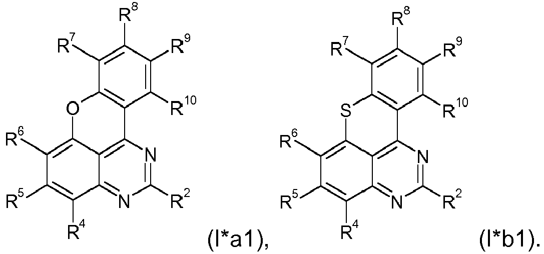

*In formula (I*):*
R2, R⁴, R⁵, R⁶, R⁷, R⁸, R⁹, and R¹⁰ are independently of each other selected from H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
at least two of R⁴, R⁵, R⁶, R⁷, R⁸, R⁹ or R¹⁰, if present at adjacent carbon atoms, together form at least one C₆-C₁₈aryl or C₂-C₁₈heteroaryl ring or ring system,
wherein one of R², R⁴, R⁵, R⁶, R⁷, R⁸, R⁹, R¹⁰ in the compound of formula (I*) is a N-heteroaryl group according to general formula (XVI), preferably, R² in the compound of formula (I*) is a N-heteroaryl group according to general formula (XVI); 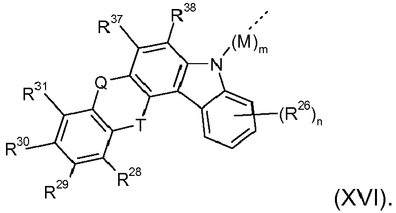 D is -CO-, -COO-, -S-, -SO-, -SO₂-, -O-,-CR¹⁵=CR¹⁶-, -NR¹⁷-, -SiR²²R²³-, -POR²⁵-, -C≡C-, preferably -O-, -NR¹⁷-, -SiR²²R²³-.

E is -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₂₅alkyl group, which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; preferably E is independently of each other selected from -NR¹⁷R¹⁸, -CN, -SiR²²R²³R²⁴, -POR²⁵R^{26'}, a C₁-C₁₈ alkyl group, a C6-C18aryl group which is unsubstituted or substituted by at least one C6-C12aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one C₁-C₁₈alkyl group, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group.

R¹⁵ and R¹⁶ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group which is interrupted by at least one O, preferably H.

R¹⁷ and R¹⁸ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H or a C₁-C₁₈ alkyl group.

R¹⁷ and R¹⁸ together form a five or six membered aliphatic, aromatic or heteroaromatic ring, preferably five or six membered aliphatic ring.

R¹⁹ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²⁰ is H or a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈ alkyl group or a C₁-C₁₈alkyl group, which is interrupted by at least one O, preferably H, a C₆-C₁₈aryl group which is unsubstituted or a C₁-C₁₈alkyl group.

R²¹ is independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²², R²³ and R²⁴ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group.

R²⁵, R^{26'} and R²⁷ are independently of each other H, a C₁-C₁₈alkyl group, a C₆-C₁₈aryl group which is unsubstituted or substituted by at least one C₆-C₁₂aryl or by at least one C₁-C₁₈alkyl group, a C₇-C₂₅aralkyl which is unsubstituted or substituted by at least one group E, a C₅-C₁₂cycloalkyl group which is unsubstituted or substituted by at least one group E, preferably H, a C₆-C₁₈aryl group which is unsubstituted or a C₁-C₁₈alkyl group.

According to the present invention, in the compounds according to general formula (I*), in particular in compounds according to general formulae (I*a) and (I*b), X⁴, X⁵, X⁶, X⁷, X⁸, X⁹ and X¹⁰ can independently of each other be N, CR⁴, CR⁵, CR⁶, CR⁷, CR⁸, CR⁹ or CR¹⁰, wherein R⁴ to R¹⁰ have the meanings as mentioned above. According to a preferred embodiment of the present invention none of X⁴, X⁵, X⁶, X⁷, X⁸, X⁹ and X¹⁰ is N, i.e. X⁴, X⁵, X⁶, X⁷, X⁸, X⁹ and X¹⁰ are CR⁴, CR⁵, CR⁶, CR⁷, CR⁸, CR⁹ respectively CR¹⁰, wherein R⁴ to R¹⁰ have the meanings as mentioned above.

In a further embodiment, the present invention therefore preferably relates to compounds according to formula (I*), preferably (I*a) and (I*b), more preferably (I*a1) and (I*b1) as mentioned above, wherein independently of each other R², R⁴, R⁵, R⁶, R⁷, R⁸, R⁹, R¹⁰ and R¹¹ have the same meanings as mentioned above. Most preferably, R², R⁴, R⁵, R⁶, R⁷, R⁸, R⁹, and R¹⁰ are H, wherein one of R², R⁴, R⁵, R⁶, R⁷, R⁸, R⁹, R¹⁰ in the compound of formula (I*) is a N-heteroaryl group according to the general formula (XVI), (XVIa), (XVIb), (XVII) or (XVIIb). Further most preferably, R⁴, R⁵, R⁶, R⁷, R⁸, R⁹ and R¹⁰ are H and R² is a N-heteroaryl group according to one of the formula (XVI), (XVIa), (XVIb), (XVII) or (XVIIb).

Particularly preferred molecules according to general formula (I*) according to the present invention are shown in the following.

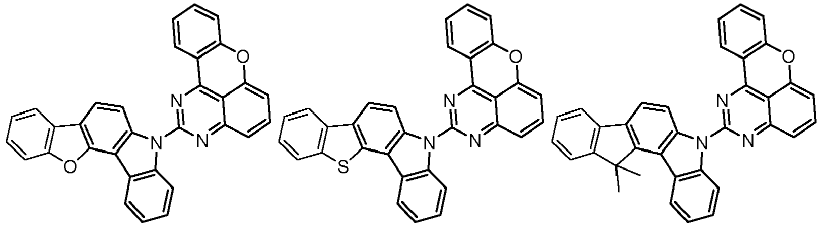

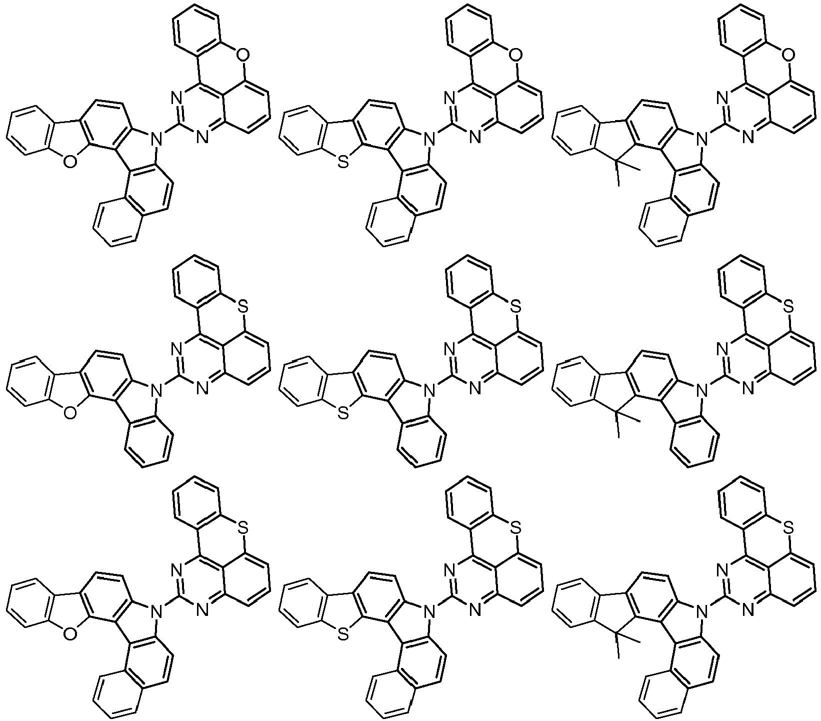

### Preparation of the compounds of formula (I*)

The preparation steps for preparing the compounds of formula (I*) are the same as the preparation steps mentioned above concerning the preparation of the compounds of formula (I).

The present invention therefore also relates to a process for the preparation of a compound according to general formula (I*) as defined above, at least comprising step (A*)
(A*) coupling of a compound according to general formula (VI*) 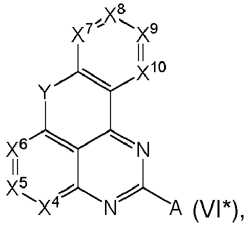
   with a compound of formula R²-H to obtain a compound according to general formula (I*), wherein A is a selected from Cl, Br, I, F, OSO₂CH₃, and OSO₂CF₃ or OSO₂C₆H₄CH₃,
   wherein Y, R², X⁴, X⁵, X⁶, X⁷, X⁸, X⁹ and X¹⁰ have the same meanings as defined above. Preferfably, R² is a N-heteroaryl group according to one of the formulae (XVI), (XVIa), (XVIb), (XVII) or (XVIIb).

The reaction parameters for step (A*) are the same as mentioned for step (A).

Also, the preferred reaction sequence for obtaining the compound of formula (VI*) is the same as mentioned for the compound of formula (VI').

The compounds of formula (I) and of formula (I*) according to the present invention are particularly suitable for use in organic electronics applications, especially OLEDs, especially as a host material, a charge transporting material, charge and/or exciton blocking material, preferably having a good overall performance, especially improved efficiency and/or driving voltage, preferably as host material. The structure of organic electronics applications, especially thr OLEDs is known in the art. Suitable structures are described below.

### Compounds of formula (I) and compounds of formula (I*) according to the present invention in organic electronics applications

In the following applications of the compounds of formula (I) and the compounds of formula (I*) according to the present invention as mentioned above in organic electronic applications will be explained.

It has been found that the compounds of formula (I) and the compounds of formula (I*) according to the present invention are particularly suitable for use in applications in which charge carrier conductivity is required, especially for use in organic electronics applications, more preferably for use in organic light-emitting diodes (OLEDs).

The compounds of formula (I) and the compounds of formula (I*) according to the present invention being particularly suitable in OLEDs for use in a light-emitting layer, wherein the compound of formula (I) respectively the compound of formula (I*) is preferably used as a host material, a charge transporting material, charge and/or exciton blocking material.

In a preferred embodiment, the compounds of formula (I) and the compounds of formula (I*) according to the present invention are present in OLEDs as a host, as a single host or as a host in combination with one or more further hosts.

The present invention therefore relates to an electronic device, preferably an organic electroluminescent device, more preferably an organic light emitting diode (OLED), comprising least one compound of formula (I) or at least one compound of formula (I*) according to the present invention.

The present invention preferably further relates to the electronic device according to the present invention, preferably an organic electroluminescence device, more preferably an organic light emitting diode (OLED), comprising a cathode, an anode, and a plurality of organic thin film layers provided between the cathode and the anode, the plurality of organic thin film layers comprising at least one compound of formula (I) or at least one compound of formula (I*) according to the present invention.

The present invention preferably further relates to the electronic device according to the present invention, preferably an organic electroluminescence device, more preferably an organic light emitting diode (OLED), comprising a cathode, an anode, and a plurality of organic thin film layers provided between the cathode and the anode, the plurality of organic thin film layers comprising at least one emitting layer comprising least one compound of formula (I) or at least one compound of formula (I*) according to the present invention.

The present invention further relates to an electronic equipment comprising the organic electroluminescence device according to the present invention.

The present invention also relates to an emitting layer, preferably present in an electronic device, more preferably in an electroluminescence device, particularly preferably in an organic light emitting diode (OLED), comprising least one compound of formula (I) or at least one compound of formula (I*) according to the present invention.

The present invention preferably further relates to the use of least one compound of formula (I) or at least one compound of formula (I*) according to the present invention in an electronic device, preferably in an electroluminescence device, particularly preferably in an organic light emitting diode (OLED), preferably in an emitting layer.

According to the present application, the terms matrix and host are used interchangeable.

Suitable structures of organic electronic devices, especially organic light-emitting diodes (OLED), are known to those skilled in the art and are specified below.

For example, the electronic device, preferably an organic electroluminescence device, more preferably an organic light emitting diode (OLED), according to the present invention comprises a cathode, an anode, and a plurality of organic thin film layers provided between the cathode and the anode, the organic thin film layers comprising least one compound of formula (I) or at least one compound of formula (I*) according to the present invention.

Preferably, the present invention provides an organic light-emitting diode (OLED) comprising an anode and a cathode and a light-emitting layer arranged between the anode and the cathode, and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the OLED comprises least one compound of formula (I) or at least one compound of formula (I*) according to the present invention.

The present application further relates to a light-emitting layer, preferably present in an electronic device, more preferably in an electroluminescence device, particularly preferably in an organic light emitting diode (OLED), comprising least one compound of formula (I) or at least one compound of formula (I*) according to the present invention. Examples of preferred compounds according to general formulae (I) and (I*) are shown above.

Most preferably, the electronic device according to the present invention is an organic light emitting diode (OLED).

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

The least one compound of formula (I) and the at least one compound of formula (I*) according to the present invention may be present in any layer of the OLED, preferably a host material, a charge transporting material, charge and/or exciton blocking material. More preferably, the least one compound of formula (I) and the at least one compound of formula (I*) according to the present invention are present as host material in the emitting layer of the OLED.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

According to a preferred embodiment the OLED according to the present invention comprises at least one compound according to general formula (I) or their preferred embodiments as a charge transporting material, preferably as a hole transporting layer. In addition to the compounds according to general formula (I) or without these compounds either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 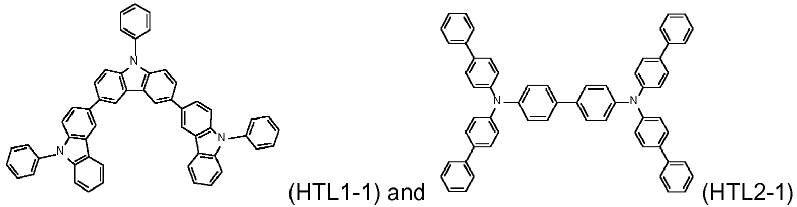 constitute the hole transport layer.

Customarily used hole-transporting molecules are selected from the group consisting of 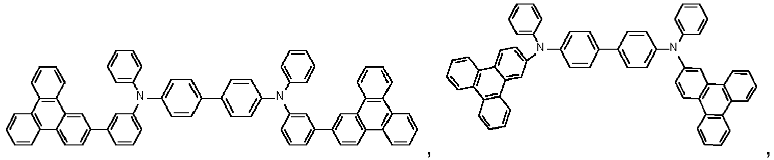 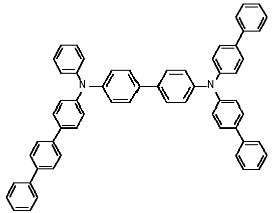 (4-phenyl-N-(4-phenylphenyl)-N-[4-[4-(N-[4-(4-phenyl-phenyl)phenyl]anilino)phenyl]phenyl]aniline), 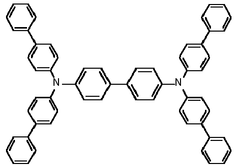 (4-phenyl-N-(4-phenylphenyl)-N-[4-[4-(4-phenyl-N-(4-phenylphenyl)anilino)phenyl]phenyl]aniline), 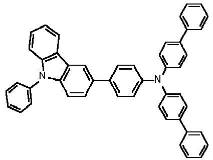 (4-phenyl-N-[4-(9-phenylcarbazol-3-yl)phenyl]-N-(4-phenylphenyl)aniline), 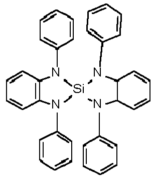 (1,1',3,3'-tetraphenylspiro[1,3,2-benzodiazasilole-2,2'-3a,7a-dihydro-1,3,2-benzodiazasilole]), 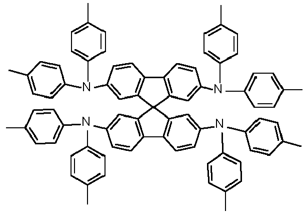 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application.

According to a preferred embodiment of the present invention, at least one compound of general formula (I) or at least one compound of general formula (I*) is present in the exciton blocking layer of the OLED according to the present invention.

### Emitting layer (e)

The light emitting layer is an organic layer having a light emitting function and is formed from one or more layers, wherein one of the layers comprises a host material (first host material), optionally a second host material, and the light emitting material.

According to a preferred embodiment of the present invention, at least one compound of general formula (I) or at least one compound of general formula (I*) is present in the emitting layer of the OLED according to the present invention, preferably as host material.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are used in combination and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one fluorescence or phosphorescence emitter, suitable emitter materials being known to those skilled in the art.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 to 570 nm). In another preferred embodiment, red emitter materials (570 to 680 nm) are preferred.

The phosphorescent dopant (phosphorescent emitter material) is a compound which usually emits light by releasing the energy of excited triplet state.
The compounds according to general formula (I) as well as the compounds according to general formula (I*) are most preferably used as the matrix (=host material) in the light-emitting layer.

Suitable metal complexes for use in the inventive OLEDs, preferably as emitter material, are described, for example, in documents WO 02/60910 A1, US 2001/0015432 A1,

US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Particularly suitable metal complexes are described in US2014048784, US2012223295, US2014367667, US2013234119, US2014001446, US2014231794, US2014008633, WO2012108388 and WO2012108389. The emitters mentioned in US2013234119, paragraph [0222], are exemplified. Selected emitters, especially red emitters, of said emitters mentioned in US2013234119, paragraph [0222], are:

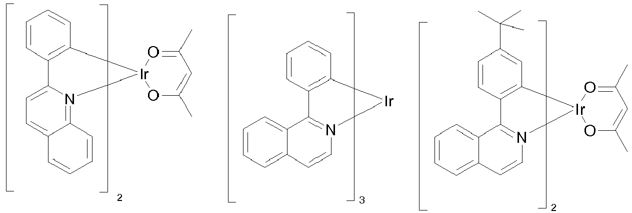

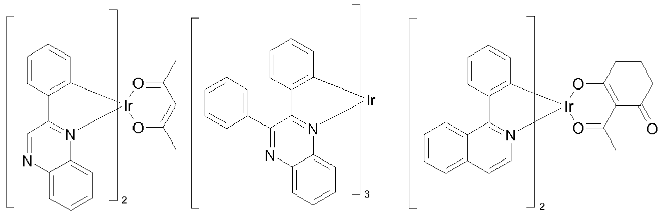

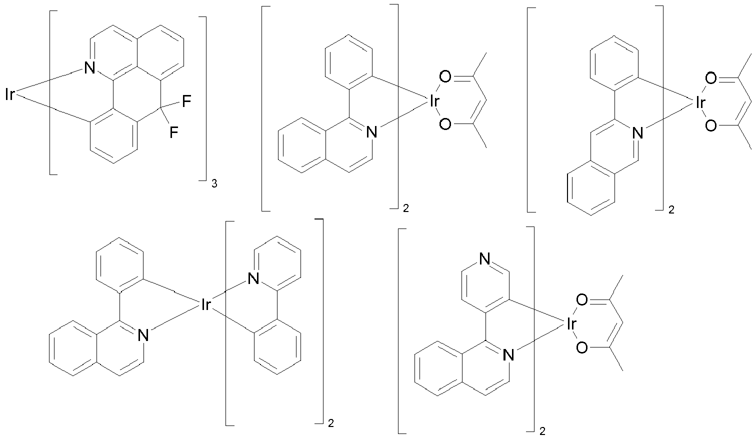

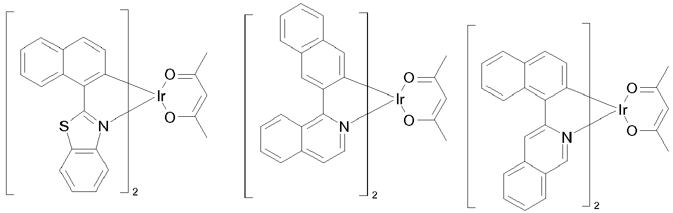

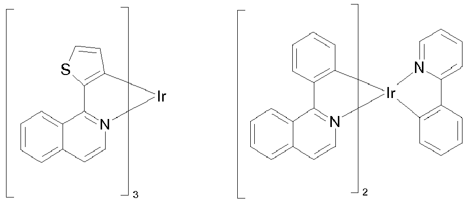

Further suitable Emitters are mentioned in: Mrs Bulletin, 2007, 32, 694:

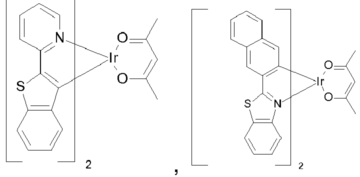

Further suitable Emitters are mentioned in: WO2009100991:

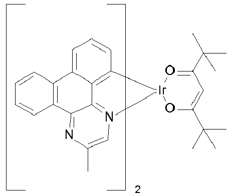

Further suitable Emitters are mentioned in: WO2008101842:

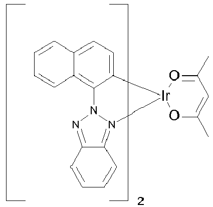

Further suitable Emitters are mentioned in: US 20140048784, especially in paragraph [0159]:

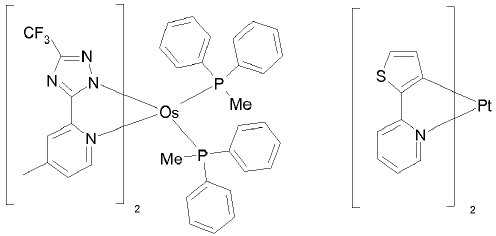

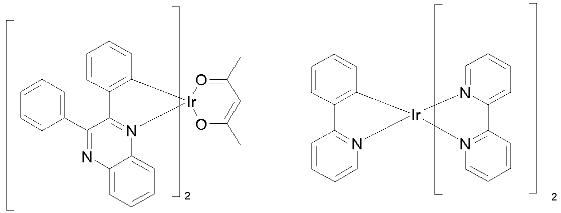

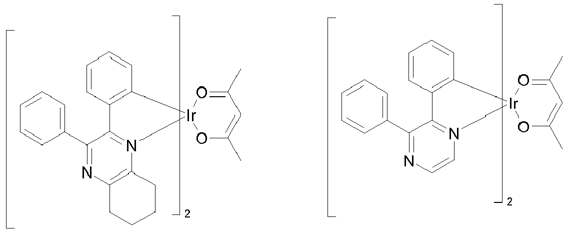

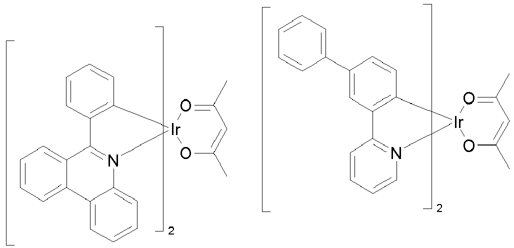

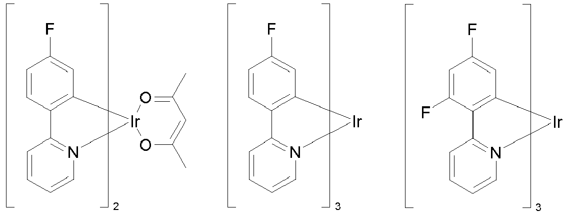

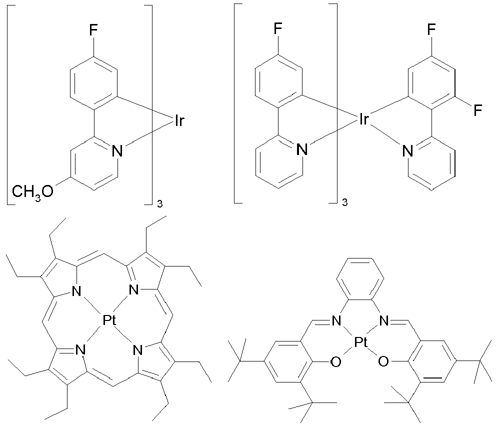

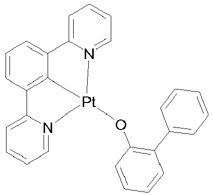

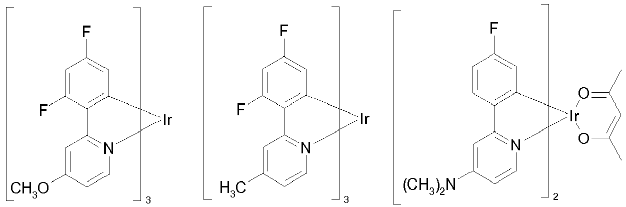

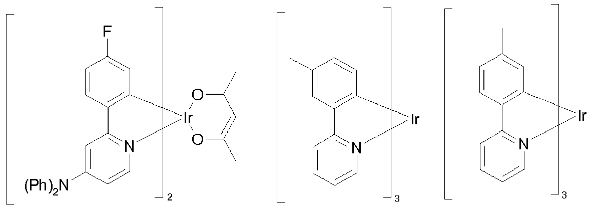

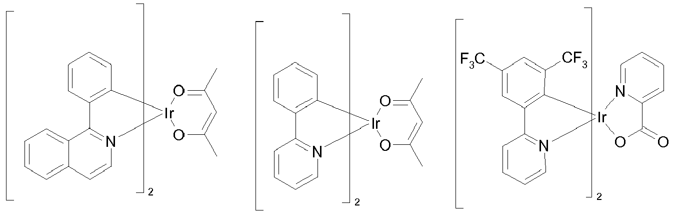

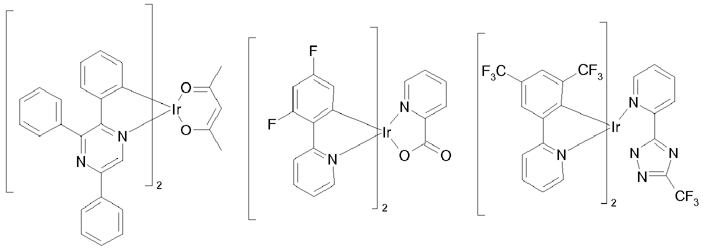

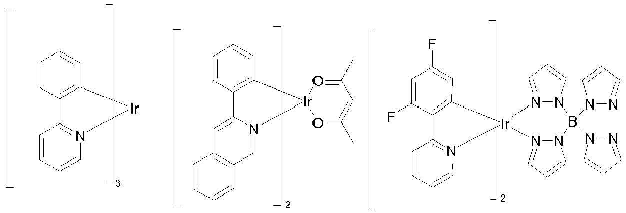

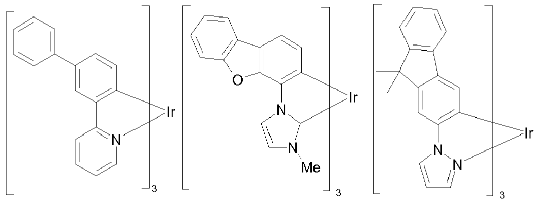

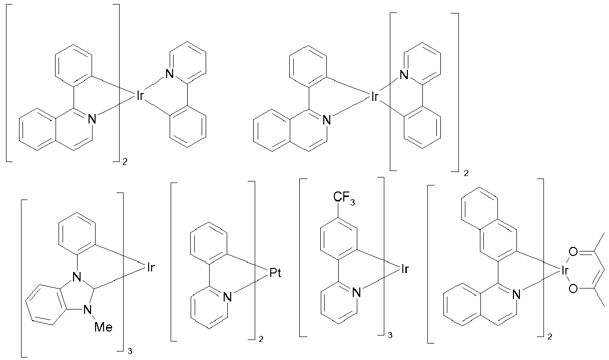

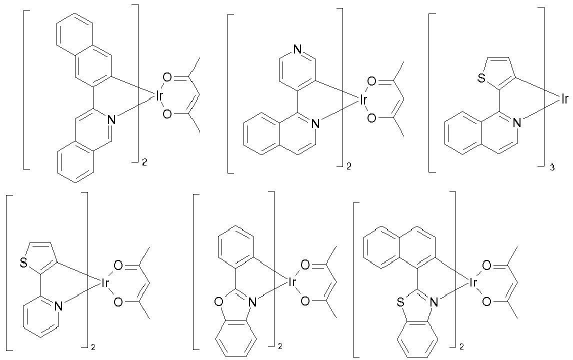

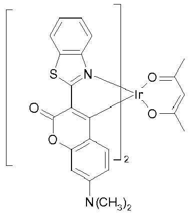

Further suitable red emitters are shown in WO 2008/109824. Preferred red emitters according to this document are the following compounds:

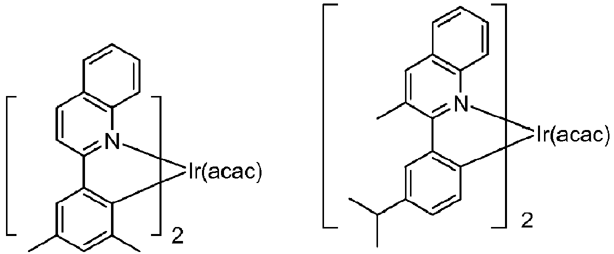

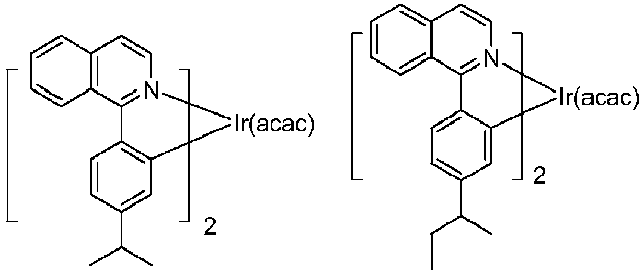

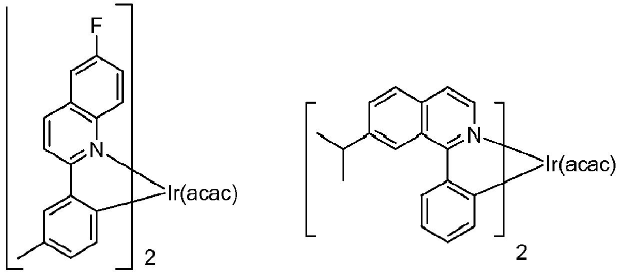

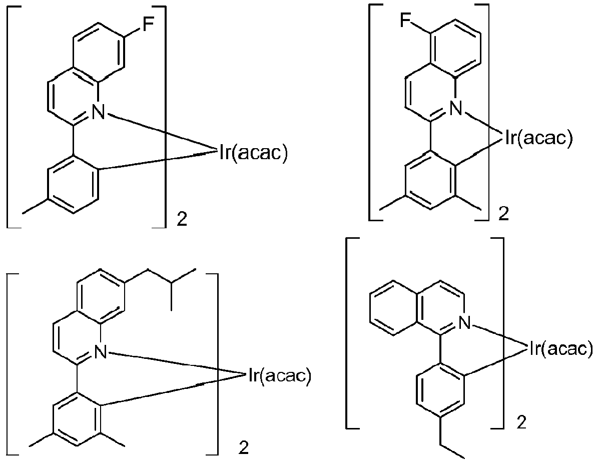

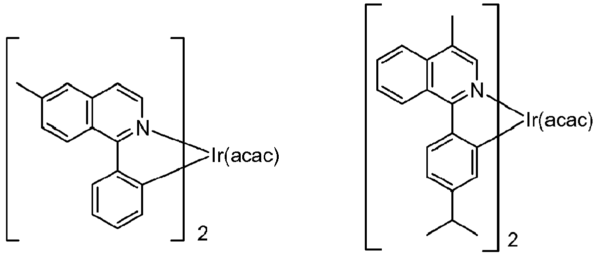

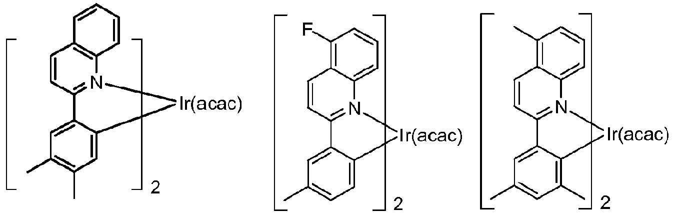

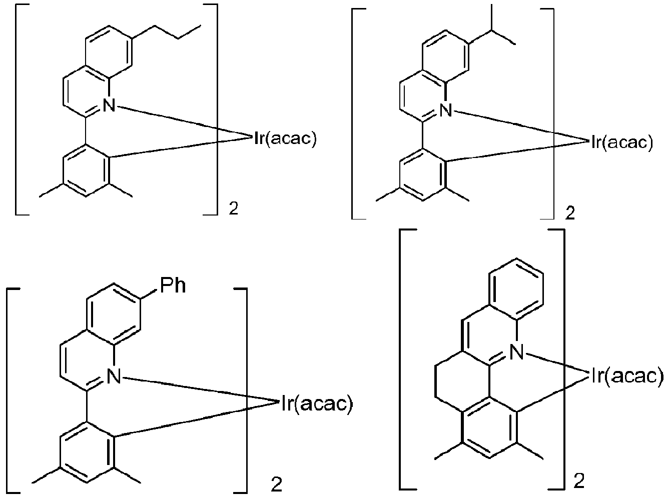

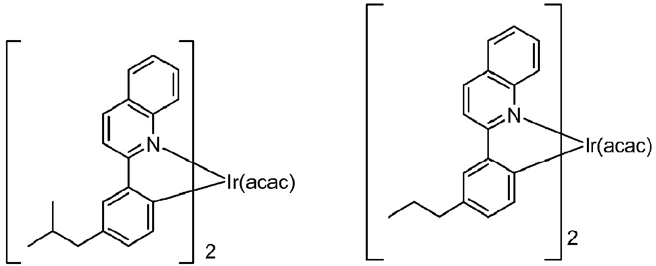

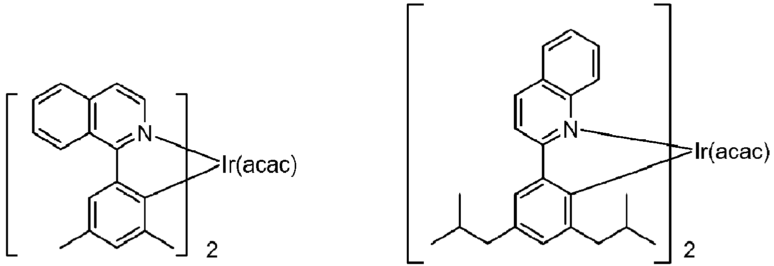

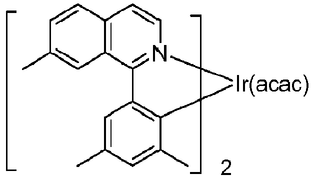

The emitter materials (dopants), preferably the phosphorescent emitter materials, may be used alone or in combination of two or more.

Further red emitters that may be used in the OLEDs according to the present invention are disclosed in US 2013/0299795 and are shown in the following:

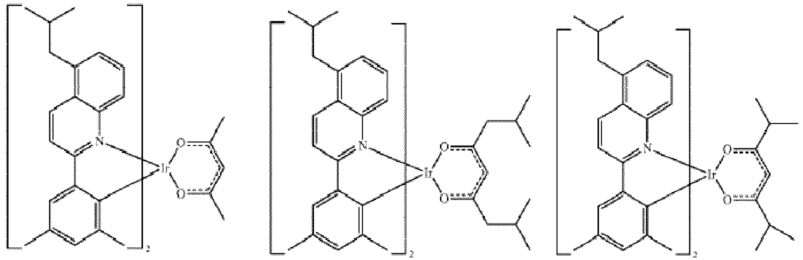

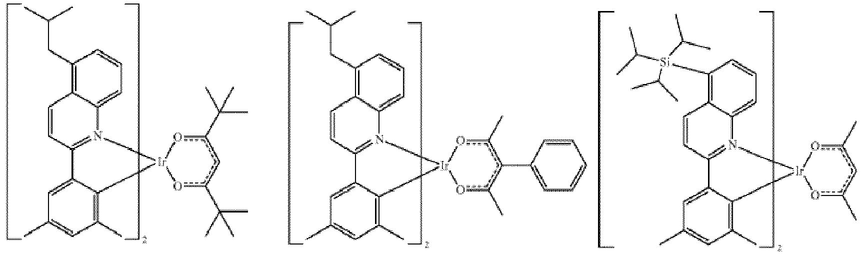

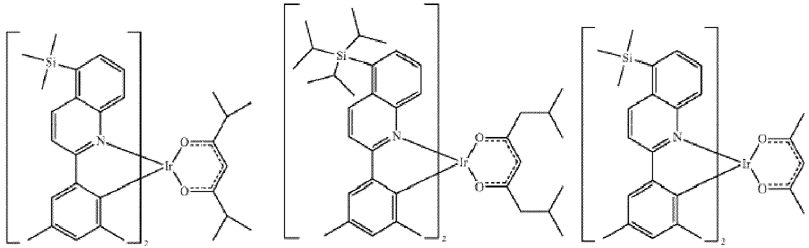

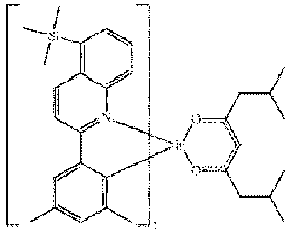

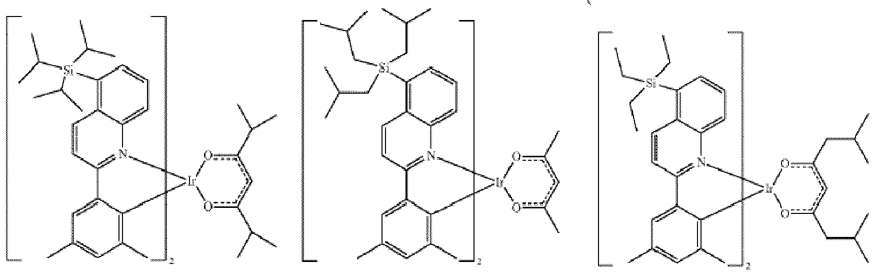

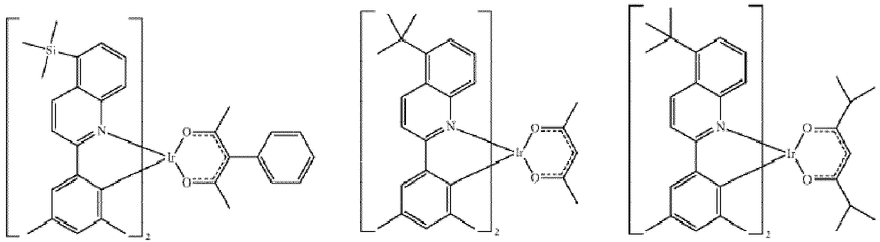

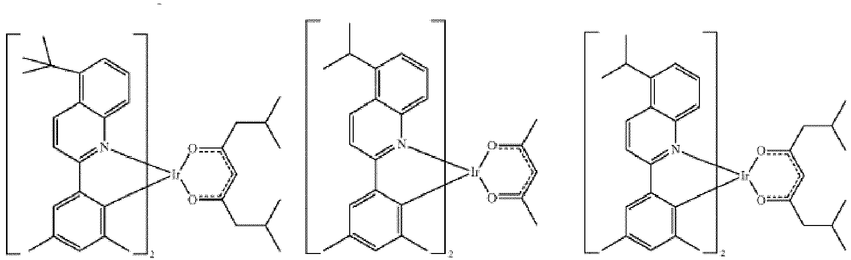

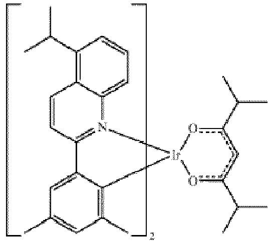

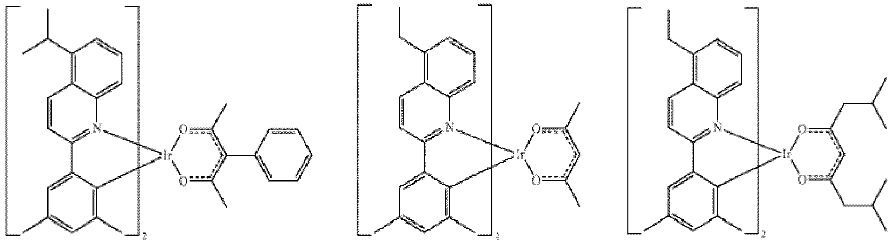

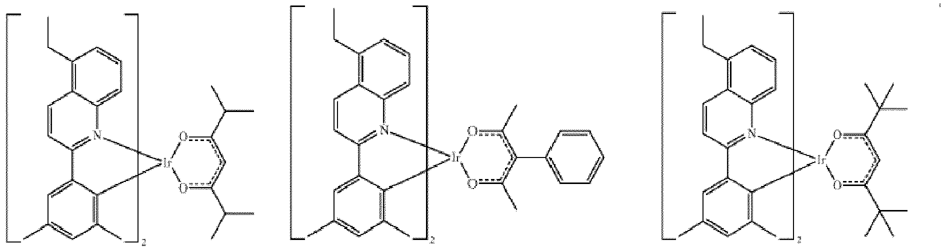

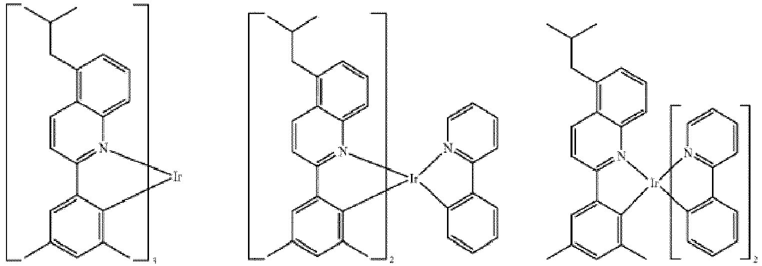

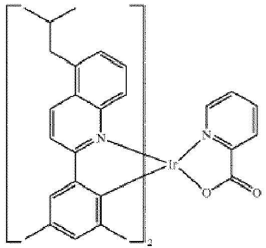

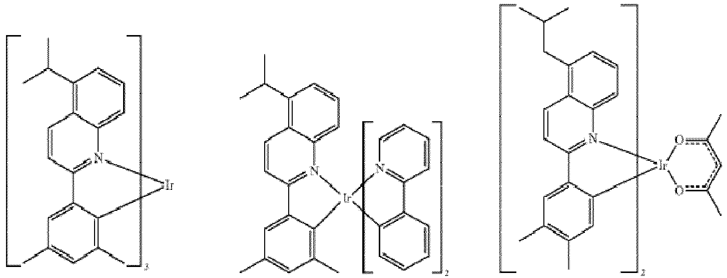

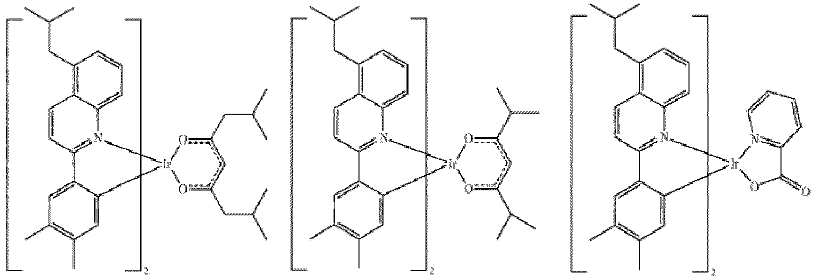

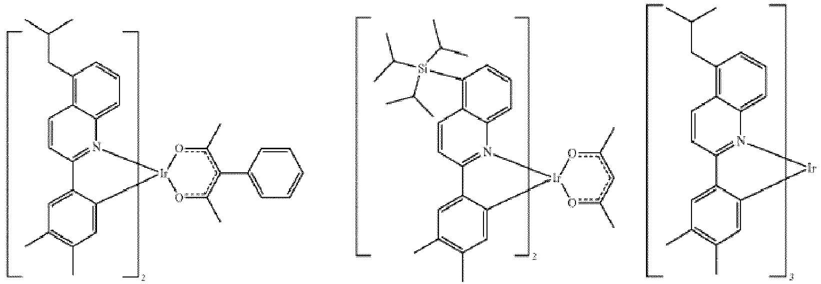

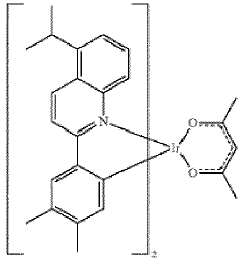

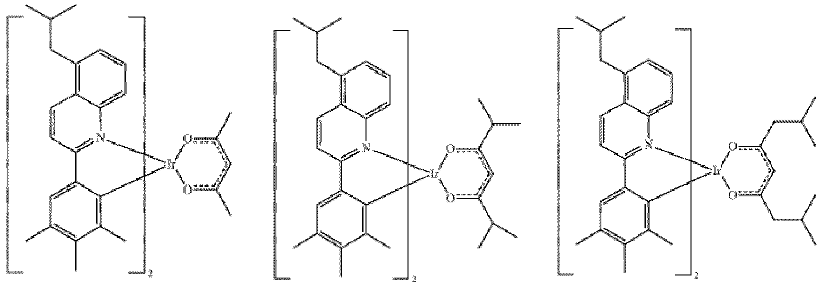

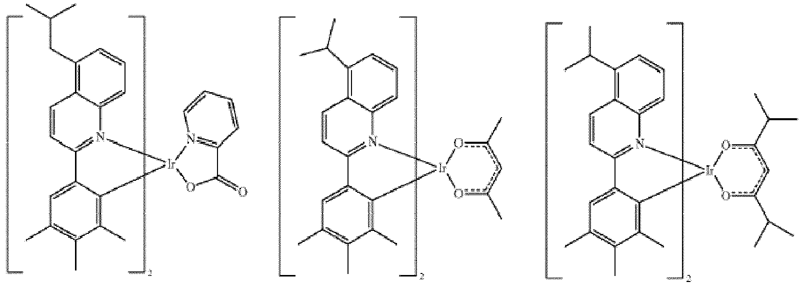

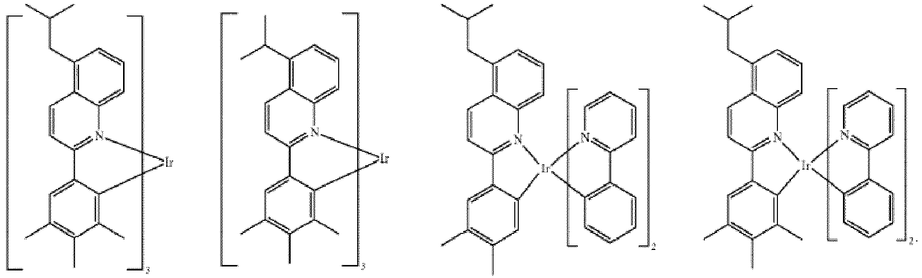

Further red emitters that may be used in the OLEDs according to the present invention are disclosed in US 2013/0146848 and are shown in the following:

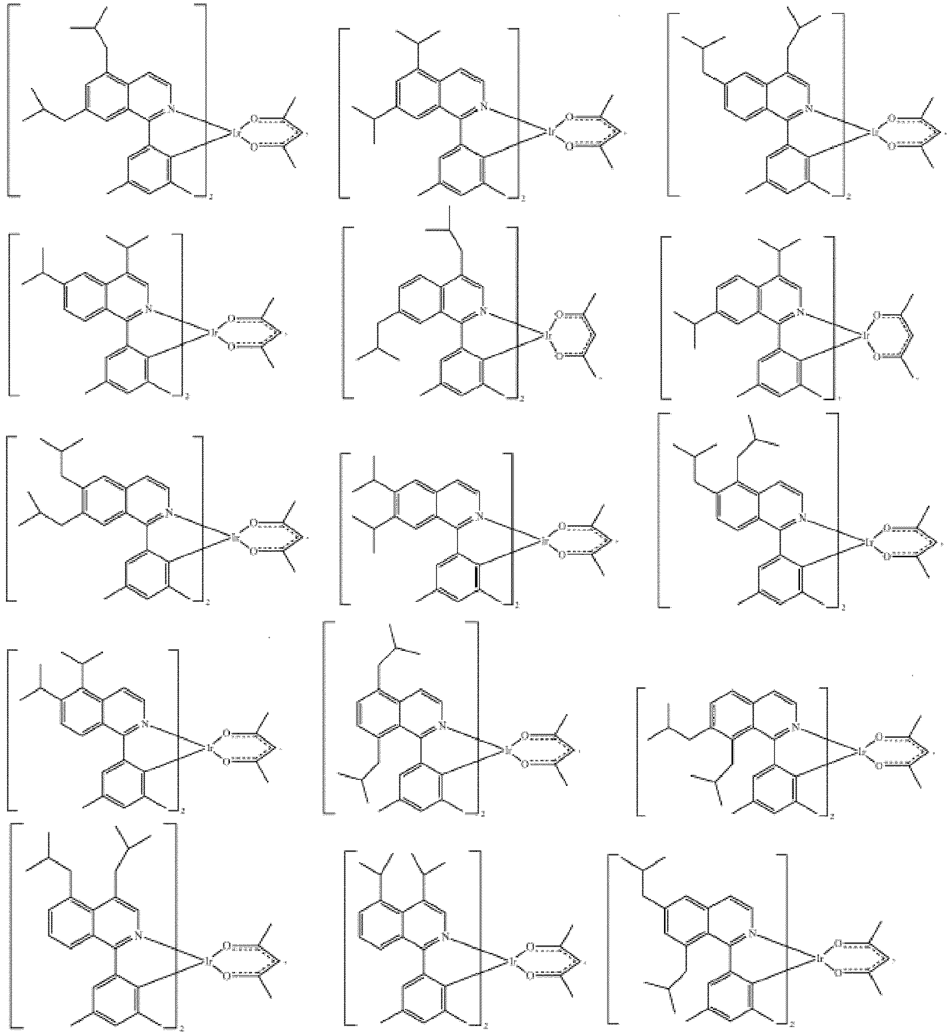

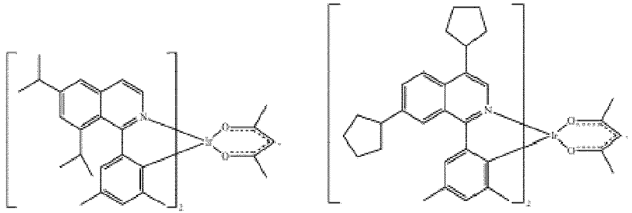

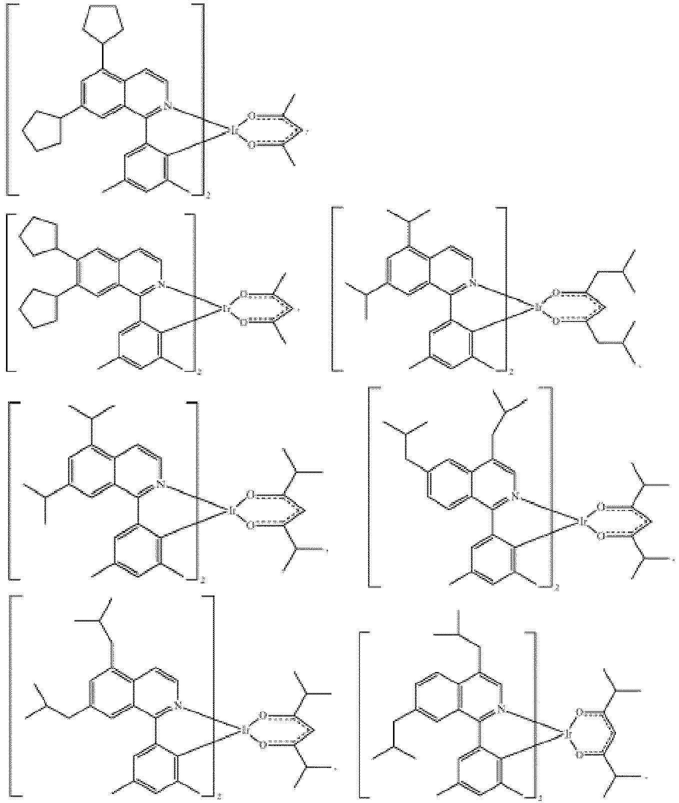

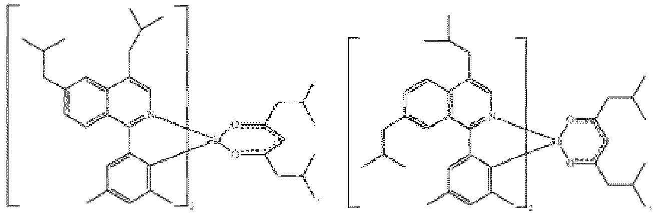

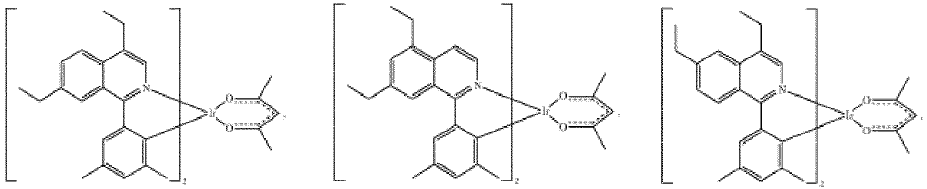

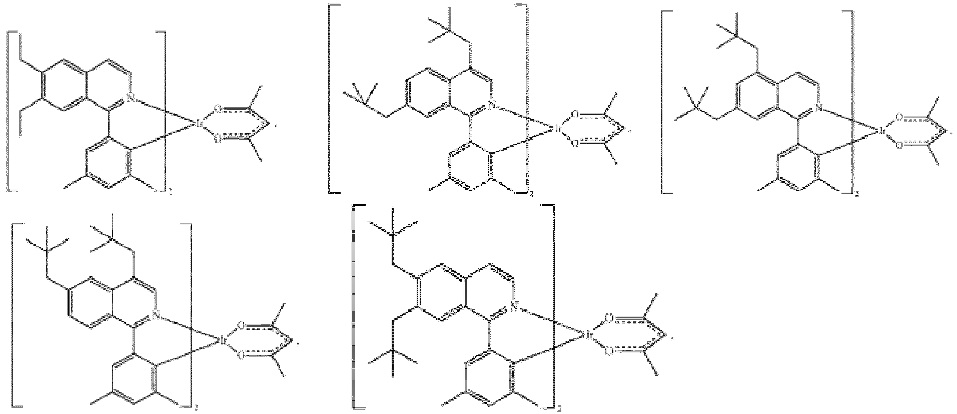

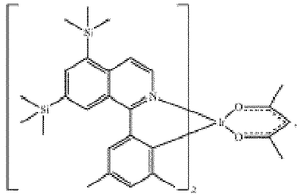

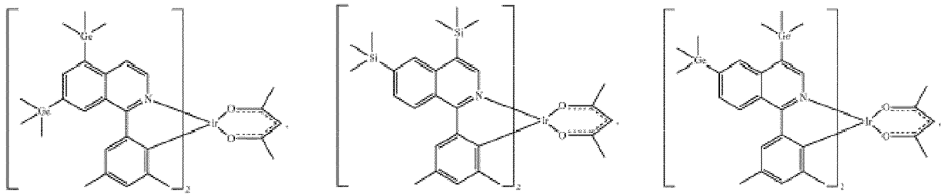

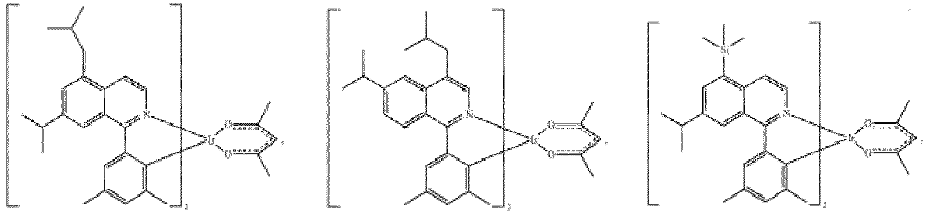

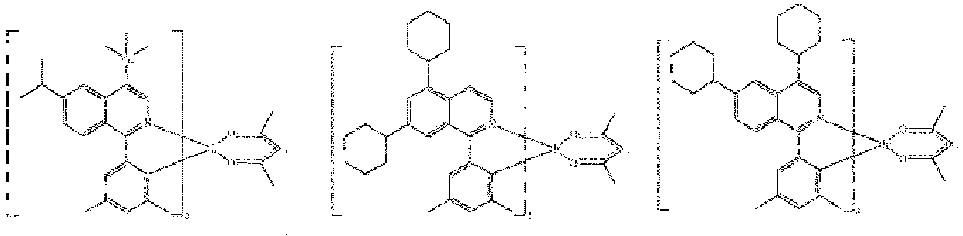

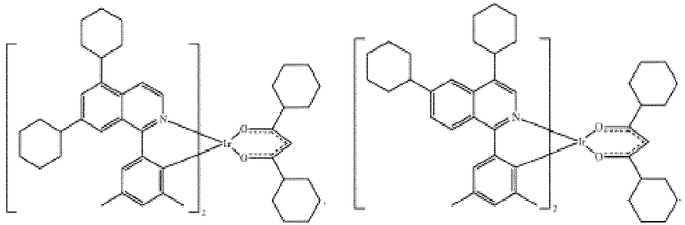

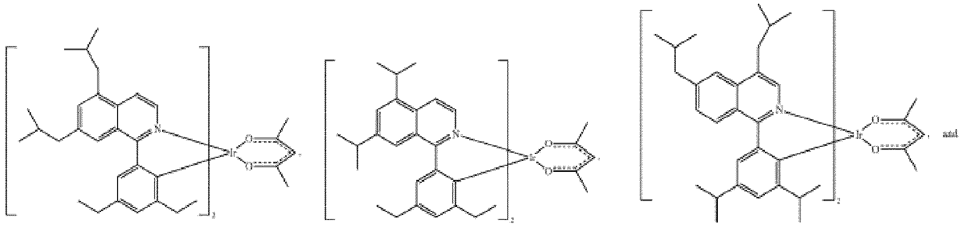

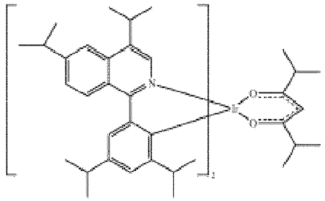

Further suitable red emitters are shown in US 2015/0001472. Preferred red emitters according to this document are the following compounds:

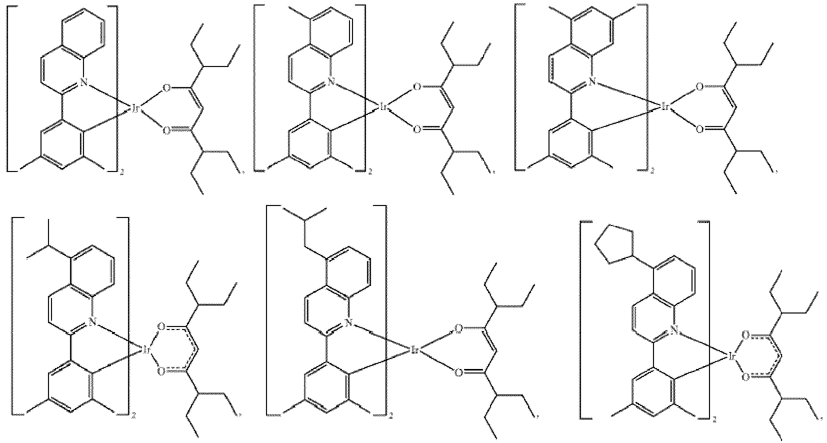

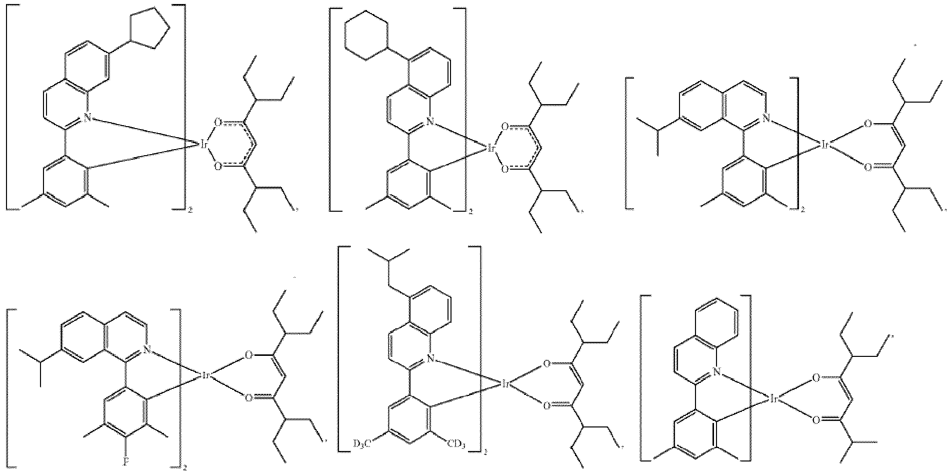

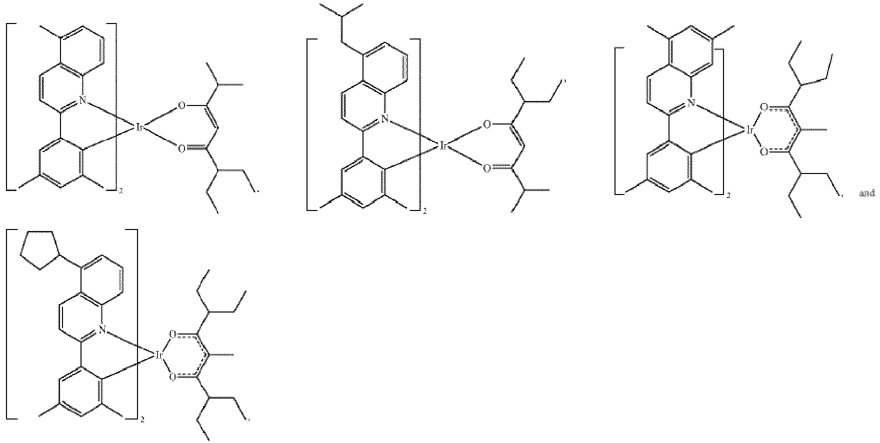

Further suitable red emitters are shown in US 2016/0093808. Preferred red emitters according to this document are the following compounds:

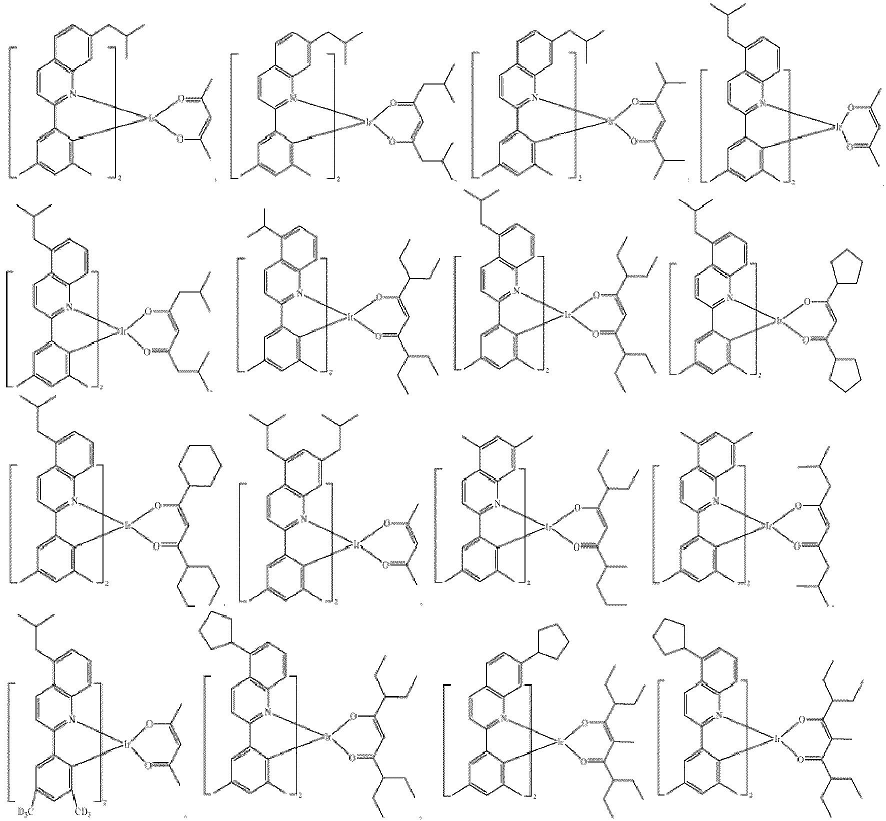

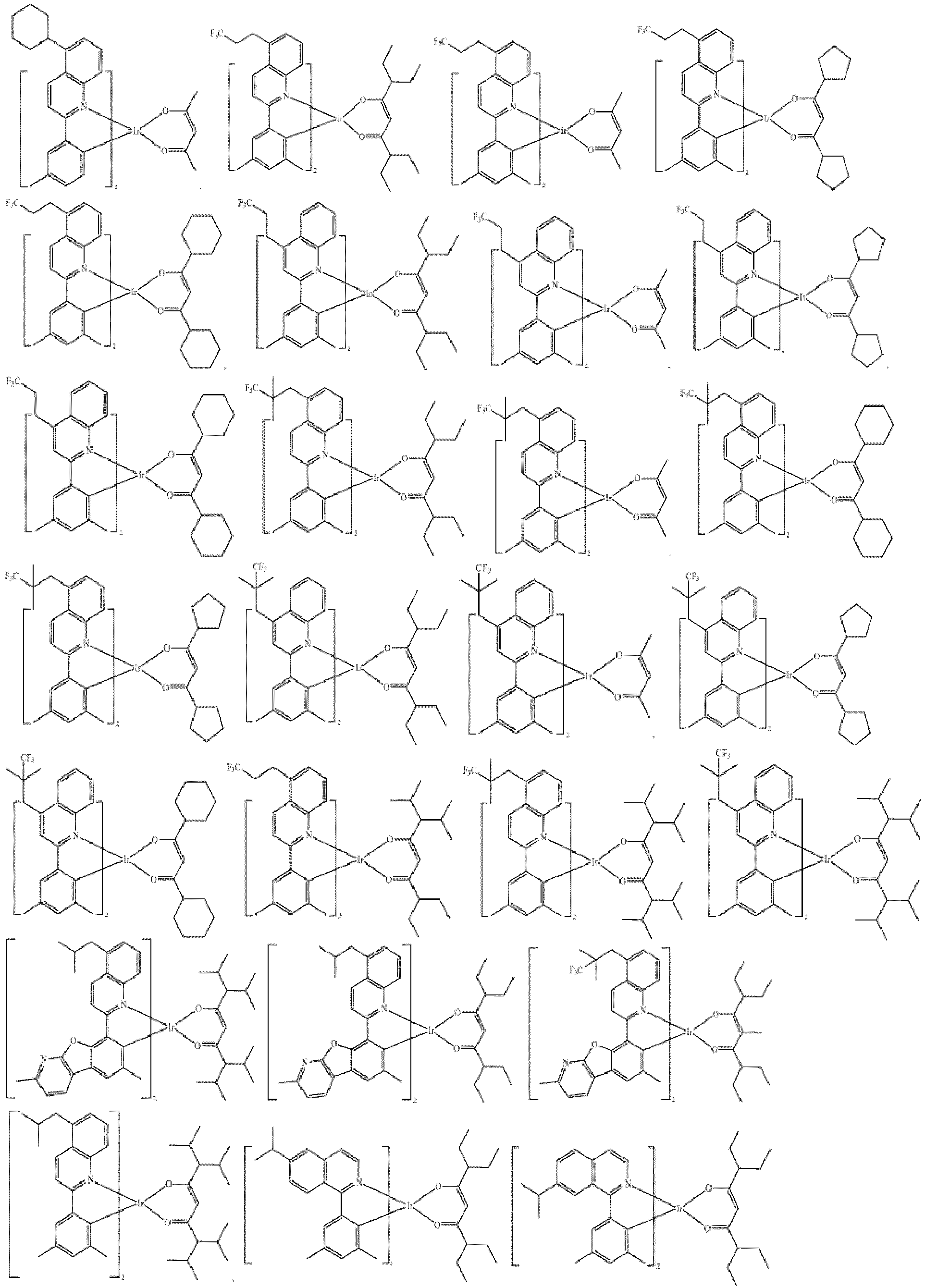

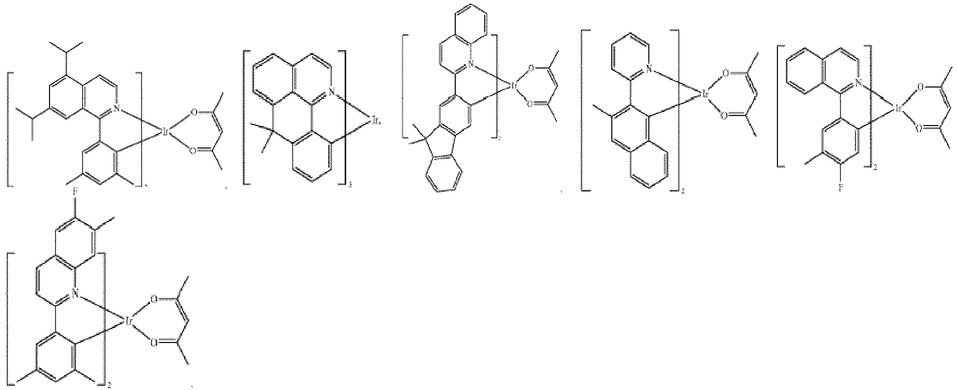

According to a further embodiment of the OLED according to the present invention, the light emitting layer may comprise at least one fluorescent, preferably blue, emitter. Examples of preferred blue dopants that may be present in the light emitting layer of the OLED according to the present invention are polycyclic amine derivatives as mentioned in EP 2924029. Particularly preferred aromatic amine derivatives are selected from compounds according to the following formula (20):

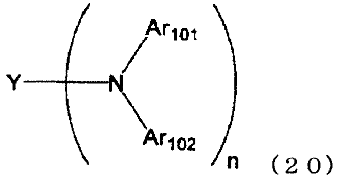

In the formula (20), Y is a substituted or unsubstituted fused aromatic hydrocarbon group including 10 to 50 ring carbon atoms.

Ar₁₀₁, and Ar₁₀₂ are independently a substituted or unsubstituted aryl group including 6 to 50 ring carbon atoms or a substituted or unsubstituted heterocyclic ring group including 5 to 50 ring atoms.

Specific examples of Y include the above-mentioned fused aryl group. Y is preferably a substituted or unsubstituted anthryl group, a substituted or unsubstitued pyrenyl group or a substituted or unsubstituted chrysenyl group.

n is an integer of 1 to 4. It is preferred that n be an integer of 1 to 2.

The above-mentioned formula (20) is preferably one represented by the following formulas (21) to (24).

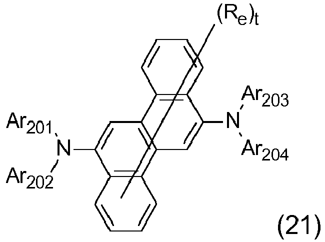

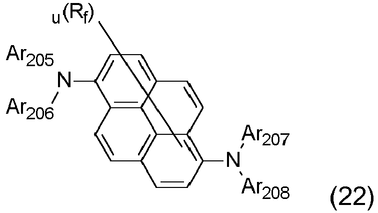

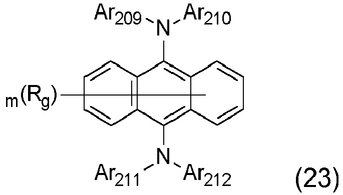

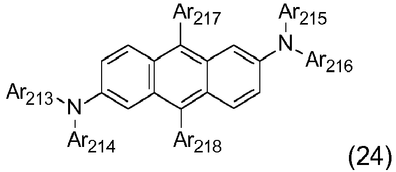

In the formulae (21) to (24), Rₑ, R_{f} and R_{g} are independently a substituted or unsubstituted alkyl group including 1 to 20 carbon atoms, a substituted or unsubstituted alkenyl group including 2 to 50 carbon atoms, a substituted or unsubstituted alkynyl group including 2 to 50 carbon atoms, a substituted or unsubstituted aralykyl group including 1 to 20 carbon atoms, a substituted or unsubstituted cycloalkyl group including 3 to 20 ring carbon atoms, a substituted or unsubstituted alkoxy group including 1 to 20 carbon atoms, a substituted or unsubstituted aryloxy group including 6 to 20 ring carbon atoms, a substituted or unsubstituted aryl group including 6 to 50 ring carbon atoms, a substituted or unsubstituted silyl group, a substituted or unsubstituted alkyl germanium group including 1 to 50 carbon atoms or a substituted or unsubstituted aryl germanium group including 6 to 50 ring carbon atoms. Rₑ, R_{f} and R_{g} may independently be bonded to any of the bonding positions of the benzene rings that constitutes the fused polycyclic skeleton.

As preferable examples of Rₑ, R_{f} and R_{g}, a substituted or unsubstituted aryl group including 6 to 50 ring carbon atoms can be given. More preferably, Rₑ, R_{f} and R_{g} are a substituted or unsubstituted phenyl group, a substituted or unsubstituted naphthyl group, or the like.

t is an integer of 0 to 10. u is an integer of 0 to 8. m is an integer of 0 to 10. Ar₂₀₁ to Ar₂₁₈ are independently an aryl group including 6 to 50 ring carbon atoms or a substituted or unsubstituted heterocyclic group including 5 to 50 ring atoms.

Preferred examples of Ar₂₀₁ to Ar₂₁₈ include a substituted or unsubstituted phenyl group, a substituted or unsubstituted dibenzofuranyl group or the like. As preferable examples of the substituent of Ar₂₀₁, to Ar₂₁₈, an alkyl group, a cyano group and a substituted or unsubstituted silyl group can be given.

In the formulae (21) to (24), as examples of the alkyl group, the alkoxy group, the aryl group, the aryloxy group and the heterocyclic group, those exemplified above can be given.

As the alkenyl group including 2 to 50, preferably 2 to 30, more preferably 2 to 20, and particularly preferably 2 to 10, carbon atoms, a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2 - dimethylallyl group, a 1-phenyl-1-butenyl group, a 3-phenyl-1-butenyl group or the like can be given. Preferred are a styryl group, a 2,2-diphenylvinyl group, a 1,2-diphenylvinyl group or the like.

As the alkynyl group including 2 to 50 (preferably 2 to 30, more preferably 2 to 20, particularly preferably 2 to 10) carbon atoms, a propargyl group, a 3-pentynyl group or the like can be given.

As the alkyl germanium group, a methylhydrogermyl group, a trimethylgermyl group, a triethyl-germyl group, a tripropylgermyl group, a dimethyl-t-butylgermyl group or the like can be given.

As the aryl germanium group, a phenyldihydrogermyl group, a diphenylhydrogermyl group, a triphenylgermyl group, a tritolylgermyl group, a trinaphthylgermyl group or the like can be given.

As the styrylamine compound and the styryldiamine compound, those represented by the following formulas (17) and (18) are preferable.

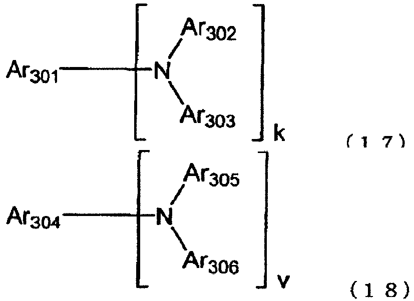

In the formula (17), Ar₃₀₁, is a k-valent group; a k-valent group corresponding to a phenyl group, a naphthyl group, a biphenyl group, a terphenyl group, a stilbene group, a styrylaryl group and a distyrylaryl group. Ar₃₀₂ and Ar₃₀₃ are independently an aryl group including 6 to 20 ring carbon atoms, and Ar₃₀₁, Ar₃₀₂ and Ar₃₀₃ may be substituted.

k is an integer of 1 to 4, with an integer of 1 and 2 being preferable. Any one of Ar₃₀₁ to Ar₃₀₃ is a group including a styryl group. It is further preferred that at least one of Ar₃₀₂ and Ar₃₀₃ be substituted by a styryl group.

As for the aryl group including 6 to 20 ring carbon atoms, the above-mentioned aryl group can be specifically given. Preferable examples include a phenyl group, a naphthyl group, an anthranyl group, a phenanthryl group, a terphenyl group or the like.

In the formula (18), Ar₃₀₄ to Ar₃₀₆ are a v-valent substituted or unsubstituted aryl group including 6 to 40 ring carbon atoms. v is an integer of 1 to 4, with an integer of 1 and 2 being preferable.

Here, as the aryl group including 6 to 40 ring carbon atoms in the formula (18), the above-mentioned aryl group can be specifically given. A naphthyl group, an anthranyl group, a chrysenyl group, a pyrenyl group or an aryl group represented by the formula (20) is preferable.

As preferable substituents that substitute on the aryl group, an alkyl group including 1 to 6 carbon atoms, an alkoxy group including 1 to 6 carbon atoms, an aryl group including 6 to 40 ring carbon atoms, an amino group substituted by an aryl group including 6 to 40 ring carbon atoms, an ester group including an aryl group that includes 5 to 40 ring carbon atoms, an ester group including an alkyl group that includes 1 to 6 carbon atoms, a cyano group, a nitro group, a halogen atom or the like can be given.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

Further possible fluorescent blue emitters that may be present in the emitting layer of the OLED according to the present invention are mentioned in US2012112169.

### Host (matrix) materials

The compound of formula (I) as well as the compound of formula (I*) is preferably employed as host material, more preferably as phosphorescent host material.

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are usually employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials (hosts) mentioned in this application. According to a preferred embodiment, the electronic device according to the present invention, preferably the OLED according to the present invention, comprises at least one compound according to general formula (I) or at least one compound according to general formula (I*) as matrix (host) material.

According to one embodiment, the light-emitting layer comprises at least one emitter material and at least two matrix materials (co-host system), wherein one of the matrix materials is a compound according to general formula (I) or according to the general formula (I*) and the other matrix material(s) is/are used as co-host(s). Suitable other host materials than the compounds of general formula (I) or of general formula (I*) (co-hosts) are known by a person skilled in the art. "Further host materials" means in the sense of the present application, host materials different from the compounds of general formula (I) or of general formula (I*). However, it is also possible to use two or more different compounds of general formula (I) and/or (I*) as host material in the light-emitting layer in an OLED of the present application. This embodiment is preferably realized with emitter materials that emit red light.

According to another embodiment, the light-emitting layer comprises at least one emitter material and a compound according to general formula (I) or according to general formula (I*) as a single matrix material. Examples of preferred compounds of general formula (I) and of general formula (I*) useful as single host material are shown above. This embodiment is preferably realized with emitter materials that emit red light.

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one preferred embodiment at least one compound according to general formula (I) or according to general formula (I*) - where the sum total of the emitter material and of the matrix material adds up to 100% by weight.

In a further more preferred embodiment, the light-emitting layer comprises a compound of general formula (I) or (I*) as matrix material, at least one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound according to general formula (I) or (I*) and the further matrix material, where the sum total of the at least one emitter material, the further matrix material and of the compound of general formula (I) or (I*) adds up to 100% by weight.

The content ratio of the compound according to general formula (I) or (I*) as first host material and the second matrix material as co-host in the light emitting layer is not particularly limited and may be selected accordingly, and the ratio of first host material: second host material is preferably 1:99 to 99:1, more preferably 10:90 to 90:10, each based on mass.

Suitable host materials that may be used in the electronic device according to the present invention as host materials, if the compounds according to the present invention are not used as host material, but for example as charge transporting material, i.e. as electron transporting material or hole transporting material, are also known by a person skilled in the art.

According to the present invention, the compounds according to general formula (I) are preferably be used as host material in the light emitting layer of the electronic device, preferably in a OLED, according to the present invention. The compounds according to general formula (I) can be used (a) as single host materials or can be used (b) in combination with any compounds suitable as host materials as mentioned above.

### Electron transport layer (g):

Electron transport layer may include a material capable of transporting electrons. Electron transport layer may be intrinsic (undoped), or doped. Doping may be used to enhance conductivity.

The compound according to general formula (I) or (I*) according to the present invention is also suitable as electron transport material, either alone or in combination with one or more of the electron transport materials mentioned below. The compound according to general formula (I) or (I*) according to the present invention is preferably suitable as electron transport material, if a blue fluorescent emitter is present in the emitting layer.

Further suitable electron-transporting materials for layer (g) of the inventive OLEDs, which may be used in combination with the compound of general formula (I) or (I*) according to the present invention or in absence of the compound of general formula (I) or (I*) according to the present invention as electron transport material, comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials, which may be used in combination with the compound of general formula (I) or (I*) according to the present invention or in absence of the compound of general formula (I) or (I*) according to the present invention as electron transport material, are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 *(ETM, HTM).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**XVI'**) below, preferably a compound of the formula **(XVI'a)** below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below **(formula XVII').** Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**XVII'**) 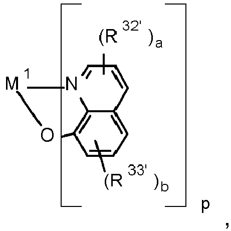 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**XVII'**) is 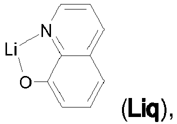 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**XVI'**), 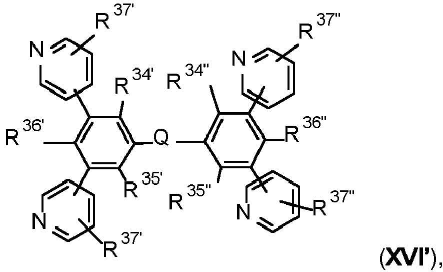 in which
R^{34"}, R^{35"}, R^{36"}, R^{37"}, R^{34'}, R^{35'}, R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-; E' is -OR^{44'}; -SR^{44'}; -NR^{40'}R^{41'}; -COR⁴³; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which
R^{38'} and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula (**XVI'**) are compounds of the formula (**XVI'a**) 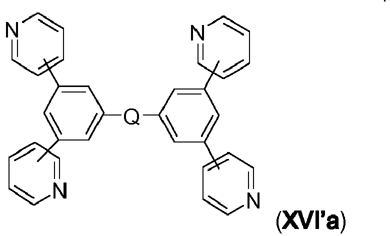 in which Q is: 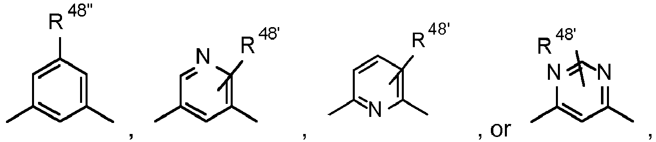 R^{48'} is H or C₁-C₁₈-alkyl and R^{48"} is H, C₁-C₁₈-alkyl or

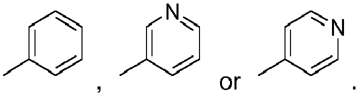

Particular preference is given to a compound of the formula

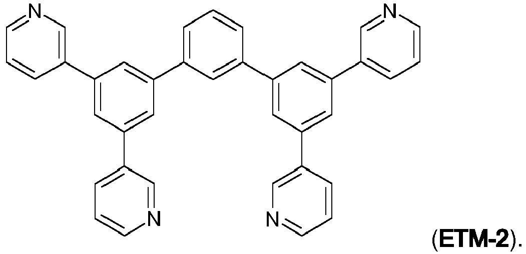

In a further, very particularly preferred embodiment, the electron-transport layer comprises a compound **Liq** and a compound **ETM-2.**

In a preferred embodiment, the electron-transport layer comprises at least one compound of the formula (**XVII'**) in an amount of 99 to 1 % by weight, preferably 75 to 25% by weight, more preferably about 50% by weight, and at least one compound of the formula (**XVI'**) in an amount of 1 to 99% by weight, preferably 25 to 75% by weight, more preferably about 50% by weight, where the amount of the compounds of the formulae (**XVII'**) and the amount of the compounds of the formulae (**XVI'**) adds up to a total of 100% by weight.

The preparation of the compounds of the formula (**XVI'**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in

WO2011/157790 are preferred, wherein dibenzofuran compound 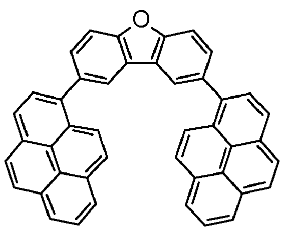 (**A-10; = ETM-1**) is most preferred.

In a preferred embodiment, the electron-transport layer comprises **Liq** in an amount of 99 to 1 % by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 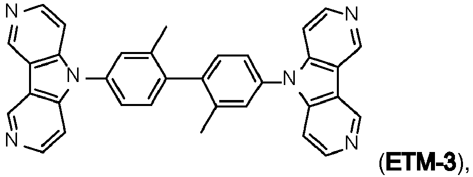 US2012/0261654 , such as, for example, a compound of formula 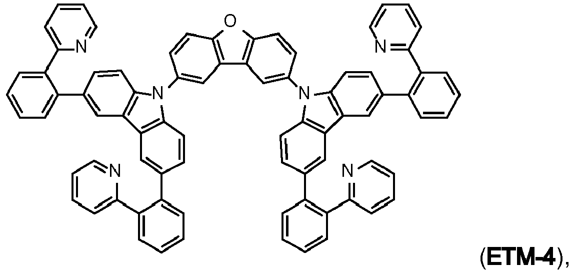 and WO2012/115034, such as for example, such as, for example, a compound of formula

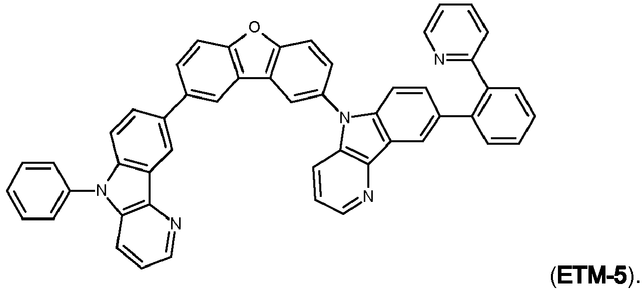

Further preferred embodiments of the electron injection layer of the OLED according to the present invention are mentioned in US 2013306955.

For example, the electron transporting material that may be present in the electron transporting layer of the OLED according to the present invention is an electron transporting material represented by formula (1):

A1(-L1-L2-L3-L4-Ar1)m (1)

wherein:
each of L1, L2, L3, and L4 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar1 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A1 represents an m-valent residue of a ring-containing compound represented by formula (2); and
m represents an integer of 1 or more: 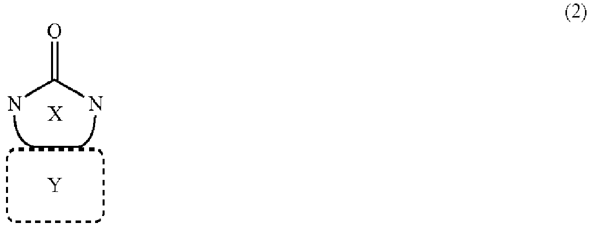 wherein:
   ring X is a substituted or unsubstituted, saturated or unsaturated 5- to 8-membered ring having a ring nitrogen atom and a ring carbon atom;
   the ring X may be fused to one or more rings Y; and
   the ring Y represents a substituted or unsubstituted hydrocarbon ring or a substituted or unsubstituted heteroring;
   The ring Y preferably represents a substituted or unsubstituted non-fused aromatic hydrocarbon ring having 6 to 30 ring carbon atoms, a substituted or unsubstituted fused aromatic hydrocarbon ring having 10 to 30 ring carbon atoms, a substituted or unsubstituted non-fused heteroring having 5 to 30 ring atoms, or a substituted or unsubstituted fused heteroring having 10 to 30 ring atoms.

The electron transporting material of the invention is preferably represented by formula (1-1) or (1-2):

A11(-L11-L21-L31-L41-Ar11)p (1-1)

wherein:
each of L11, L21, L31, and L41 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar11 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A11 represents a p-valent residue of a ring-containing compound represented by formula (2-1); and
p represents an integer of 1 or more: 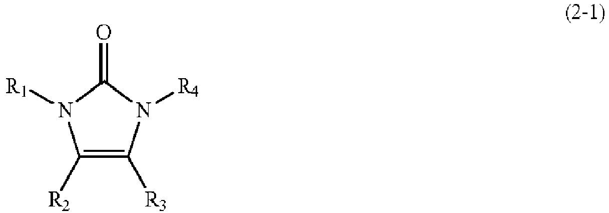 wherein;
   each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, a boryl group, a phosphino group, an amino group, a halogen atom, a cyano group, a nitro group, a hydroxyl group, a carboxyl group, or a valence bonded to L11; or a pair of R1 and R2, R2 and R3, or R3 and R4 are bonded to each other to form a ring Y represented by a substituted or unsubstituted hydrocarbon ring or a substituted or unsubstituted heteroring.

A11 of formula (1-1) preferably represents a p-valent residue of a compound represented by formula (2-1-1), (2-1-2), or (2-1-3): 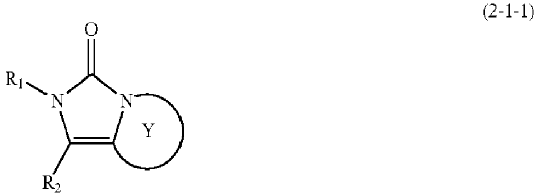 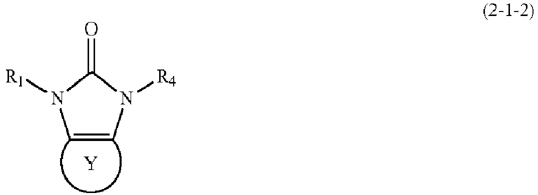 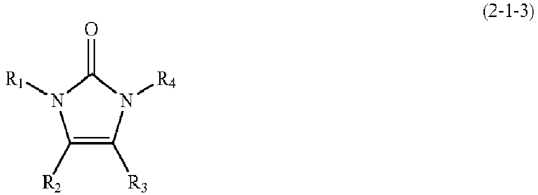 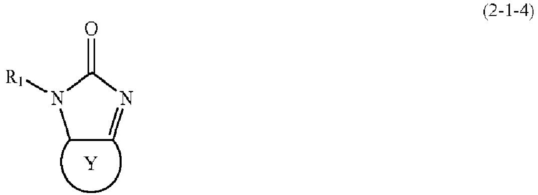 wherein:
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, an amino group substituted by a substituted or unsubstituted alkyl group having 1 to 50 carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; and
Y represents the ring Y.

Further, A11 preferably represents a p-valent residue of a compound represented by formula (2-1-2-1): 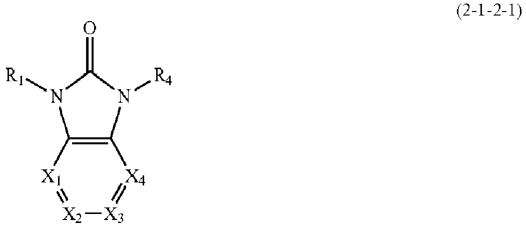 wherein:
each of X1 to X4 independently represents CR5 or N;
each of R1, R4, and R5 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; or R1, R4, and R5 are each bonded to each other to form a ring which forms a part of the ring Y.

In one embodiment, the electron transporting layer of the electronic device, preferably of the OLED according to the present invention, between the light emitting layer and the cathode, preferably comprises at least one compound of the general formula (I) or (I*).

In a preferred embodiment, the electron transporting layer comprising at least one compound of the general fomrula (I) or (I*) further comprises a reducing dopant.

Examples of the reducing dopant include a donating metal, a donating metal compound, and a donating metal complex. The reducing dopant may be used alone or in combination of two or more.

The reducing dopant referred to herein is an electron-donating material. The electron-donating material is a material which generates radical anions by the interaction with a coexisting organic material in the electron transporting layer or an organic material in a layer adjacent to the electron transporting layer, or a material having an electron-donating radical.

The donating metal is a metal having a work function of 3.8 eV or less, preferably an alkali metal, an alkaline earth metal, or a rare earth metal, and more preferably Cs, Li, Na, Sr, K, Mg, Ca, Ba, Yb, Eu, or Ce.

The donating metal compound is a compound comprising the above donating metal, preferably a compound comprising an alkali metal, an alkaline earth metal, or a rare earth metal, and more preferably a halide, an oxide, a carbonate, or a borate of these metals, for example, a compound represented by MOₓ (M: donating metal, x: 0.5 to 1.5), MFₓ (x: 1 to 3), or M(CO₃)ₓ (X: 0.5 to 1.5).

The donating metal complex is a complex comprising the above donating metal, preferably an organic metal complex of an alkali metal, an alkaline earth metal or a rare earth metal, and more preferably an organic metal complex represented by formula (I):

MQₙ (I)

wherein M is a donating metal, Q is a ligand, preferably a carboxylic acid derivative, a diketone derivative, or a quinoline derivative, and n is an integer of 1 to 4.

Examples of the donating metal complex include watermill-shaped tungsten compounds described in JP 2005-72012A and phthalocyanine compounds having an alkali metal or an alkaline earth metal as the central metal, which are described in JP 11-345687A.

The reducing dopant is preferably at least one selected from the group consisting of an alkali metal, an alkaline earth metal, a rare earth metal, an alkali metal oxide, an alkali metal halide, an alkaline earth metal oxide, an alkaline earth metal halide, a rare earth metal oxide, a rare earth metal halide, an organic complex having an alkali metal, an organic complex having an alkaline earth metal, and an organic complex having a rare earth metal, and more preferably a 8-quinolinol complex of an alkali metal.

Examples of the alkali metal includes:
Li (lithium, work function: 2.93 eV),
Na (sodium, work function: 2.36 eV),
K (potassium, work function: 2.3 eV),
Rb (rubidium, work function: 2.16 eV), and
Cs (cesium, work function: 1.95 eV).

The values of work functions are based on Handbook of Chemistry (Pure Chemistry II, 1984, p. 493, edited by The Chemical Society of Japan). The same applies hereafter

Preferred examples of the alkaline earth metals are:
Ca (calcium, work function: 2.9 eV),
Mg (magnesium, work function: 3.66 eV),
Ba (barium, work function: 2.52 eV), and
Sr (strontium, work function: 2.0 to 2.5 eV).

The work function of strontium is based of Physics of Semiconductor Device (N.Y., Wiley, 1969, p. 366).

Preferred examples of the rare earth metal are:
Yb (ytterbium, work function: 2.6 eV),
Eu (europium, work function: 2.5 eV),
Gd (gadolinium, work function: 3.1 eV), and
Er (erbium, work function: 2.5 eV).

Examples of the alkali metal oxide include Li₂O, LiO, and NaO. The alkaline earth metal oxide is preferably CaO, BaO, SrO, BeO, or MgO.

Examples of the alkali metal halide include a fluoride, for example, LiF, NaF, CsF, and KF and a chloride, for example, LiCl, KCI, and NaCl.

The alkaline earth metal halide is preferably a fluoride, such as CaF₂, BaF₂, SrF₂, MgF₂, and BeF₂ and a halide other than fluoride.

An electronic device, preferably an OLED, wherein at least one compound according to general formula (I) or (I*) used in the electron transporting layer is particularly preferred because the driving voltage is reduced or/and the efficiency increases.

The electron transporting layer facilitates the injection of electrons into the light emitting layer and transports the electrons to the light emitting zone, and has a large electron mobility and an electron affinity generally as large as 2.5 eV or more. The electron transporting layer is preferably formed from a material capable of transporting electrons to the light emitting layer at a lower strength of electric field, preferably having an electron mobility of, for example, at least 10⁻⁶ cm²/V·s under an electric field of 10⁴ to 10⁶ V/cm.

The material for forming the electron injecting/transporting layer in combination with the compound according to general formula (I) or (I*) or without the compound according to general formula (I) or (I*) is not particularly limited as long as having the preferred properties mentioned above and may be selected from those commonly used as the electron transporting material in the field of photoconductive materials and those known as the materials for the electron injecting/transporting layer of organic EL devices.

In the present invention, an electron injecting layer including an insulating material or a semiconductor may be disposed between the cathode and the organic layer. By such an electron injecting layer, the leak of electric current is effectively prevented to improve the electron injecting ability. Preferred examples of the insulating material include at least one metal compound selected from the group consisting of an alkali metal chalcogenide, an alkaline earth metal chalcogenide, an alkali metal halide, and an alkaline earth metal halide. An electron injecting layer including the above alkali metal chalcogenide is preferred because the electron injecting property is further improved. Preferred alkali metal chalcogenides include Li₂O, K₂O, Na₂S, Na₂Se, and Na₂O; preferred alkaline earth metal chalcogenides include CaO, BaO, SrO, BeO, BaS, and CaSe; preferred alkali metal halides include LiF, NaF, KF, LiCl, KCI, and NaCl; and preferred alkaline earth metal halides include fluoride such as CaF₂, BaF₂, SrF₂, MgF₂, and BeF₂ and halides other than fluoride.

Examples of the semiconductor for the electron transporting layer include an oxide, a nitride and an oxynitride of at least one element selected from Ba, Ca, Sr, Yb, Al, Ga, In, Li, Na, Cd, Mg, Si, Ta, Sb, and Zn, which are used singly or in combination of two or more. It is preferred that the inorganic compound constituting the electron transporting layer forms a microcrystalline or amorphous insulating thin film. When constituted of the insulating thin film described above, the electron injecting layer is made more uniform to reduce the pixel defect such as dark spots. Examples of such a inorganic compound include the alkali metal chalcogenide, the alkaline earth metal chalcogenide, the alkali metal halide and the alkaline earth metal halide which are described above.

### Electron injection layer (h):

The electron injection layer may be any layer that improves the injection of electrons into an adjacent organic layer.

Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive electronic device, preferably OLED, can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds according to general formula (I) or (I*) in at least one layer of the OLED, preferably in the light-emitting layer, preferably as a host material, a charge transporting material, particularly preferably as a host material and hole or electron transporting material, makes it possible to obtain OLEDs, with high efficiency and with low use and operating voltage. Frequently, the OLEDs obtained by the use of the compounds according to general formula (I) or (I*) additionally have long lifetime. The efficiency of the electronic devices, preferably OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Compounds synthesized

### Compounds synthesized

### Compound 1

### Intermediate 1-1

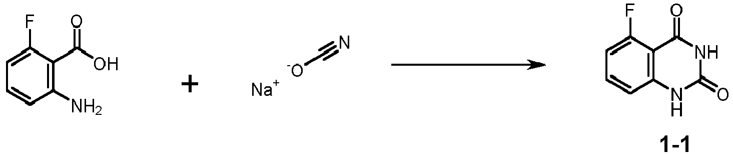

2-Amino-6-fluorobenzoic acid (35 g, 225.6 mmol) was dissolved in 885 ml of water and 26 mL of acetic acid. The mixture was stirred at 35 °C for 15 min. After that, sodium cyanate (36.67 g, 564 mmol) dissolved in 442 mL of water was added dropwise to the suspension, and the mixture was stirred at 35 °C for 30 min. Then, sodium hydroxide (180.49 g / 4.51 mol) was slowly added to the reaction mixture, the mixture was cooled at room temperature. After 374 mL of hydrogen chloride was added there, the precipitate was collected by filtration and washed with water. The solid was dried in a vacuum oven to yield 37.19 (91.5%) of 1-1 as white solid.
LC-MS : 179 [M-H]

### Intermediate 1-2

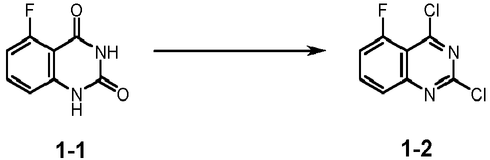

**1-1** (2.6 g, 14.43 mmol) was suspended in 29 mL of toluene and heated to 50 °C. phosphoryl chloride (9.88 mL, 108.25 mmol) was added dropwise, and then DBU (4.31 mL, 28.87 mmol) was added dropwise. The mixture was stirred vigorously at 120 °C for overnight. After the reaction mixture was cooled at room temperature, it was added dropwise to ice-water. The aqueous layer was extracted with ethyl acetate. After it was washed with brine and dried with Na₂SO₄, it was concentrated to give a solid. The crude product was purified by column chromatography eluting with toluene to yield 3.41 g (80.8%) of **1-2** as a white powder.
LC-MS : 215

### Intermediate 1-3

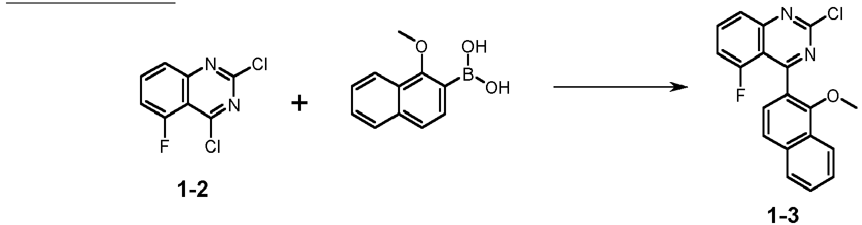

**1-2** (2.15 g, 9.90 mmol) and 1-methoxynaphthalene-2-boronic acid (2.00 g, 9.90 mmol) were dissolved in 45 mL of THF. To the solution was added potassium fluoride (1.73 g, 29.7 mmol) dissolved in 4.5 mL of water, and the mixture was evacuated and purged with Argon gas. Then, ^{t}Bu₃P-HBF₄ (287 mg, 0.99 mmol) and Pd(OAc)₂ (222 mg, 0.99 mmol) were added to the mixture, and the mixture was stirred at room temperature for 14 h. The reaction mixture was filtrated over Celite and washed out with ethyl acetate. The organic layer was collected, dried with MgSO4, and concentrated. The crude was purified by column chromatography on silica gel eluting with a mixed solvent of toluene and ethyl acetate to yield 2.70 g (81%) of **1-3** as a yellow solid.
LC-MS : 339 [M+H]

### Intermediate 1-4

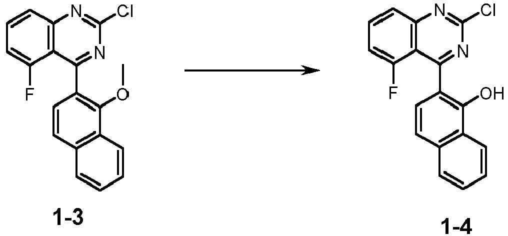

**1-3** (3.60 g, 10.63 mmol) was dissolved in 71 mL of chloroform and the solution was cooled with an ice-bath. To the solution, 63.8 mL of 1 M trichloroboran solution in toluene was added. The reaction mixture was stirred at room temperature for 48 h. The reaction mixture was added to ice water. The aqueous layer was extracted with chloroform, and then the organic layer was washed with brine and dried with Na₂SO₄. After removal of the solvent, the crude was purified by column chromatography on silica gel eluting with a mixed solvent of heptane and chloroform to yield 2.55 g (81 %) of **1-4** as an orange solid.
LC-MS : 325 [M+H]

### Intermediate 1-5

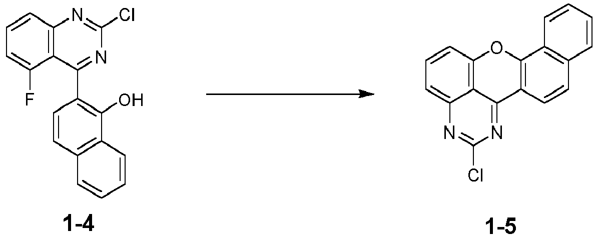

**1-4** (2.55 g, 7.85 mmol) was dissolved in 39 mL of DMF. To the solution, potassium carbonate (0.977 g, 7.07 mmol) was added at room temperature. After the mixture was stirred at room temperature for 5 min, the reaction mixture was cooled with an ice-bath and diluted with 38 mL of water. The yellow solid was collected by filtration, washed with water, and dried in a vacuum oven. The target (**1-5**) was obtained as a yellow solid in 99% yield. Without further purification, it was used for next reaction.
LC-MS : 305 [M+H]

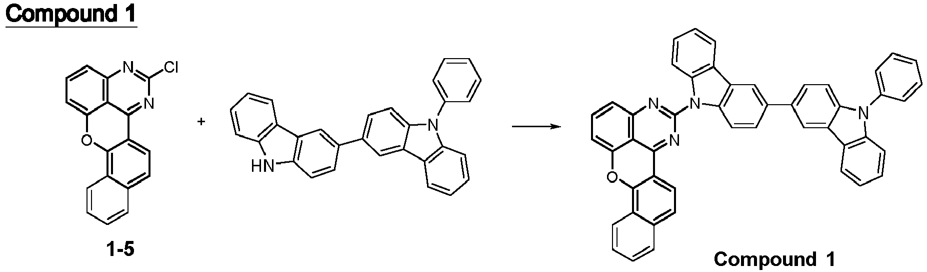

**1-5** (750 mg, 2.46 mmol), 3-(9H-carbazol-3-yl)-9-phenyl-carbazole (1.01 g, 2.46 mmol), and sodium *tert*-butoxide (331 mg, 3.45 mmol) were added to 16 mL of Toluene. The reaction mixture was evacuated and purged with Argon gas three times. Then, ^{t}Bu₃P-HBF₄ (57 mg, 0.20 mmol) and Pd₂(dba)₃ (45 mg, 0.05 mmol) were added to the mixture, and the reaction mixture was stirred at 110°C for 40 h. The precipitate was collected by filtration, and washed with toluene, ethanol, and water. The solid was dried in vacuum at 70 °C. The crude was purified by column chromatography on silica gel eluting with a mixed solvent of toluene and chloroform to yield 1.83 g (91 %) of **Compound 1** as a yellow solid.
LC-MS : 677 [M+H]

### Application Examples

### Application Example 1

A glass substrate with 120 nm-thick indium-tin-oxide (ITO) transparent electrode (manufactured by Geomatec Co., Ltd.) used as an anode was first cleaned with isopropanol in an ultrasonic bath for 10 min. To eliminate any possible organic residues, the substrate was exposed to an ultraviolet light and ozone for further 30 min. This treatment also improves the hole injection properties of the ITO. The cleaned substrate was mounted on a substrate holder and loaded into a vacuum chamber. Thereafter, the organic materials specified below were applied by vapor deposition to the ITO substrate at a rate of approx. 0.2-1 Å/sec at about 10⁻⁶ -10⁻⁸ mbar. As the first layer, 5 nm-thick of electron accepting Compound A was vapor-deposited. Then 210 nm-thick of aromatic amine Compound B was applied as a hole transporting layer and 10 nm-thick of Compound C. Then, a mixture of 2% by weight of an emitter compound (compound D), 98% by weight of a host (Compound 1) was applied to form a 40 nm-thick phosphorescentemitting layer. On the emitting layer, a mixture of 50% by weight of an electron transporting compound (Compound E), 50% by weight of Liq (8-hydroxyquinolate lithium) was applied to form a 30 nm-thick as an electron transport layer. Finally, 1 nm-thick LiF was deposited as an electron injection layer and 80 nm-thick Al was then deposited as a cathode to complete the device. The device was sealed with a glass lid and a getter in an inert nitrogen atmosphere with less than 1 ppm of water and oxygen.

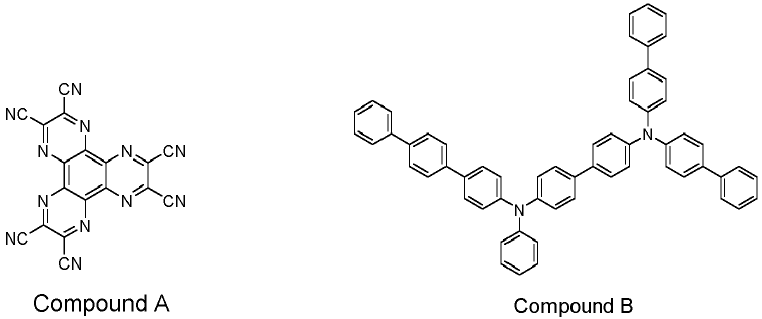

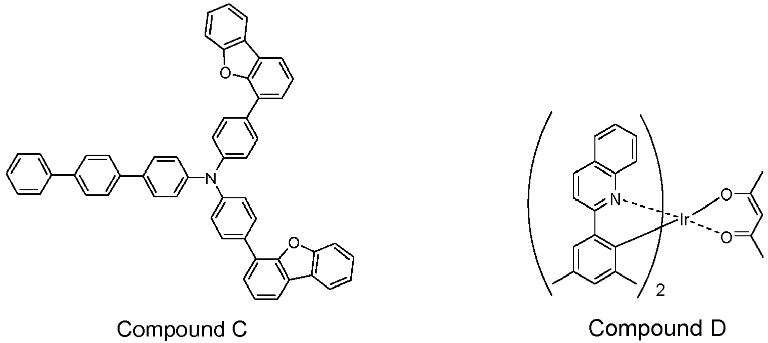

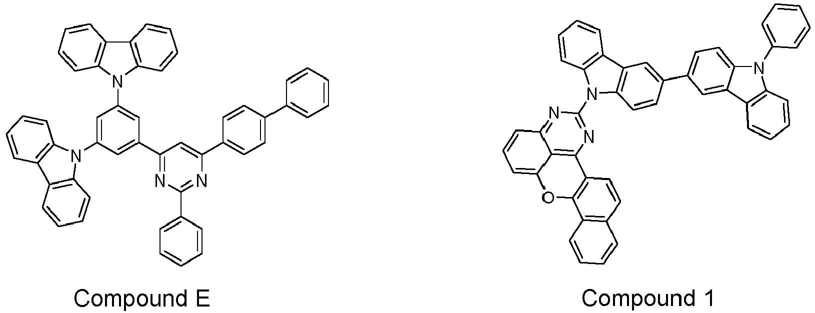

### Application Example 2

Application Example 2 was repeated except that the host (compound 1) was replaced by a comparative compound (Comparative 1). The device results are shown at table 1.

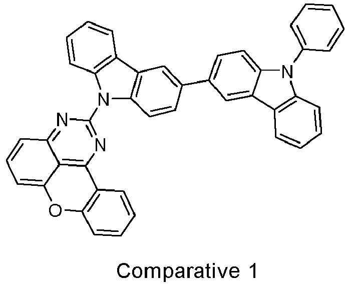

### OLED characterization

To characterize the OLED, electroluminescence spectra were recorded at various currents and voltages. In addition, the current-voltage characteristic was measured in combination with the luminance to determine luminous efficiency and external quantum efficiency (EQE). Driving voltage U, EQE and Commission Internationale de l'Eclairage (CIE) coordinate are given at 10mA/cm² except otherwise stated.

**Table 1**

| **Appl. Ex.** | **Host** | **U (V)** | **EQE (%)** | **CIE x, y** |
|---|---|---|---|---|
| **Appl. Ex. 1** | **Compound 1** | **4.67** | **16.33** | **0.66, 0.34** |
| **Appl. Ex. 2** | **Comparative 1** | **5.22** | **16.27** | **0.66, 0.34** |

The results are shown in Table 1. The CIE values show that the electroluminescence is originated from the red emitter compound (Compound D). The compound 1 can show lower driving voltage and higher EQE than the comparative compound (Comparative 1).

## Claims

1. A compound represented by the general formula (I) 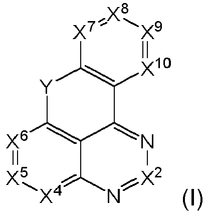 wherein
X² is CR²,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
X¹⁰ is CR¹⁰ or N,
Y is O or S,
wherein at least two adjacent groups X⁴, X⁵, X⁷, X⁸, X⁹ or X¹⁰ are not N, and at least two of R⁴, R⁵, R⁷, R⁸, R⁹ or R¹⁰, if present at adjacent carbon atoms, preferably at least two of R⁷, R⁸, R⁹ or R¹⁰, if present at adjacent carbon atoms, together form a structure according to the general formula (II) 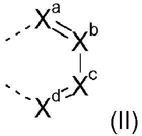 wherein
X^{a} is CR^{a} or N, preferably CR^{a},
X^{b} is CR^{b} or N, preferably CR^{b},
X^{c} is CR^{c} or N, preferably CR^{c},
X^{d} is CR^{d} or N, preferably CR^{d},
the remainder of R², R⁴, R⁵, R⁶, R⁷, R⁸, R⁹, R¹⁰, R^{a}, R^{b}, R^{c} and R^{d}
are independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E,
D is independently of each other -CO-, -COO-, -S-, -SO-, -SO₂-, -O-,-CR¹⁵=CR¹⁶-, -NR¹⁷-, -SiR²²R²³-, -POR²⁵-, -C≡C-,
E is independently of each other -OR²¹,-SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆₀aryl group which is unsubstituted or is substituted by at least one -F,-CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -
CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group;
a C₁-C₂₅alkyl group, which is unsubstituted or substituted by at least one -F, - CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -
CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group;R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₆-C₁₂aryl or at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₆-C₁₂aryl or at least one C₁-C₁₈alkyl group, and
R²⁵ and R²⁷ are independently of each other H, a C₁-C₁₈alkyl group, a C₆-C₁₈aryl group which is unsubstituted or substituted by at least one C₆-C₁₂aryl or at least one C₁-C₁₈alkyl group, a C₇-C₂₅aralkyl which is unsubstituted or substituted by at least one group E, a C₅-C₁₂cycloalkyl group which is unsubstituted or substituted by at least one group E, wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a group of formula -(L¹)ₒ-(L²)ₚ-(L³)_{q}-(L⁴)ᵣ-R¹⁴,
wherein L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E, and R¹⁴ is H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, and
o is independently of each other 1, p is independently of each other 0 or 1, q is independently of each other 0 or 1 and r is independently of each other 0 or 1,
wherein the dotted lines are bonding sites.

2. The compound according to claim 1, wherein X⁴ is CR⁴, X⁵ is CR⁵, X⁶ is CR⁶, X⁷ is CR⁷, X⁸ is CR⁸, X⁹ is CR⁹ and X¹⁰ is CR¹⁰.

3. The compound according to claim 1 or 2, wherein R¹⁴ is H or a C₁-C₂₅alkyl group which unsubstituted or substituted by at least one group E and/or interrupted by at least one group D.

4. The compound according to any one of claims 1 to 3, wherein the compound is represented by the following formulae (III), (IV), (V) or (VI)

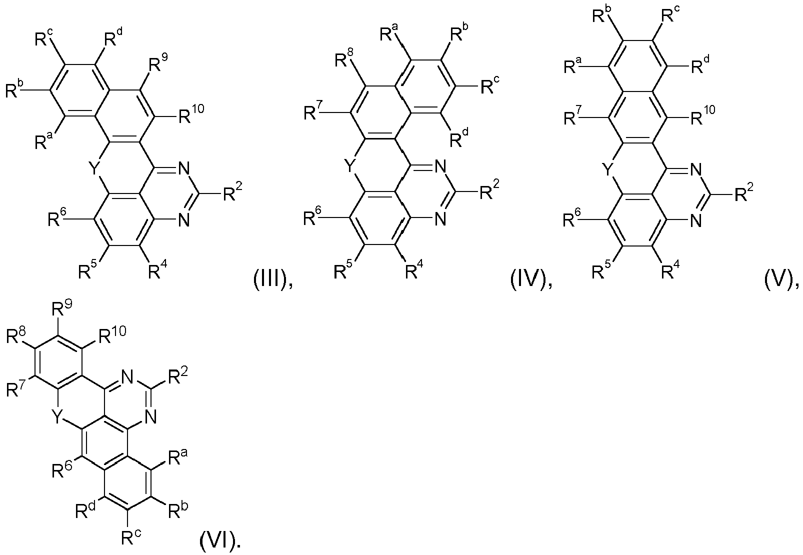

5. The compound according to any one of claims 1 to 4, wherein at least one, preferably one or two, more preferably one of R², R⁴, R⁵, R⁶, R⁷, R⁸, R⁹, R¹⁰, R^{a}, R^{b}, R^{c} and R^{d}, most preferably R², in the compound of formula (I) is a N-heteroaryl group according to general formulae (XII), (XIII), (XV), (XVI) or (XXI); 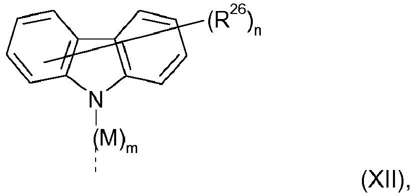 wherein
n is an integer of 0 to 8, preferably 0, 1, 2, 3 or 4, more preferably 0, 1 or 2, most preferably 1,
m is an integer of 0 to 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E,
or
at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
wherein the dotted line is a bonding site;
or 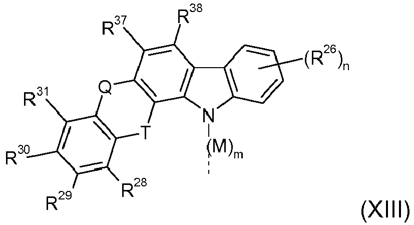
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 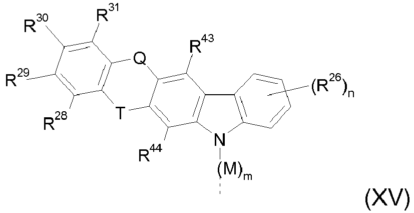
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
or
at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably, R³⁴ and R³⁵ are independently of each other H, ethyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group, for example phenyl,
wherein the dotted line is a bonding site,
or 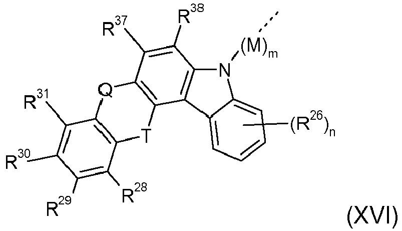
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 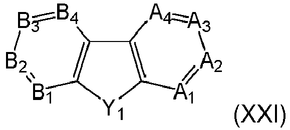 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is NR⁷⁰, CR⁷¹R⁷², O or S;
R⁶², R⁶³, R⁶⁴, R⁶⁵, R⁶⁶, R⁶⁷, R⁶⁸ and R⁶⁹ are independently of each other H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E, or
at least two of R⁶², R⁶³, R⁶⁴ and R⁶⁵ may be directly bonded to the moiety represented by the general formula (XXII) via the two *-locations; 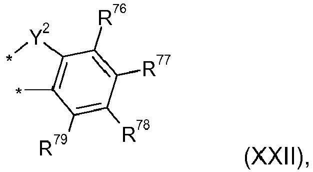
wherein
Y² is NR⁷³, CR⁷⁴R⁷⁵, O or S,
or
at least two of R⁶⁶, R⁶⁷, R⁶⁸ and R⁶⁹ may be directly bonded to the moiety represented by the general formula (XXIII) via the two *-locations. 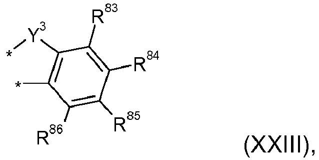
wherein
Y³ is NR⁸⁰, CR⁸¹R⁸², O or S,
R⁷⁰, R⁷³ and R⁸⁰ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E, R⁷¹, R⁷², R⁷⁴, R⁷⁵, R⁸¹ and R⁸² are, independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached, R⁷⁶, R⁷⁷, R⁷⁸, R⁷⁹, R⁸³, R⁸⁴, R⁸⁵ and R⁸⁶ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
R⁷⁶ and R⁷⁷, R⁷⁷ and R⁷⁸ and/or R⁷⁸ and R⁷⁹ together may form at least one C₆-C₁₈aryl or C₂-C₁₈heteroaryl ring or ring system, and/or
R⁸³ and R⁸⁴, R⁸⁴ and R⁸⁵ and/or R⁸⁵ and R⁸⁶ together form at least one C₆-C₁₈aryl or C₂-C₁₈heteroaryl ring or ring system,
wherein the heterocyclic group according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, wherein this respective residue R⁷⁰, R⁷³, R⁸⁰, R⁶², R⁶³, R⁶⁴, R⁶⁵, R⁶⁶, P⁶⁷ R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
m is 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

6. The compound according to claim 5, wherein the N-heteroaryl groups according to general formulae (XIII) and (XVI) are represented by the general formulae (XIV) or (XVII) 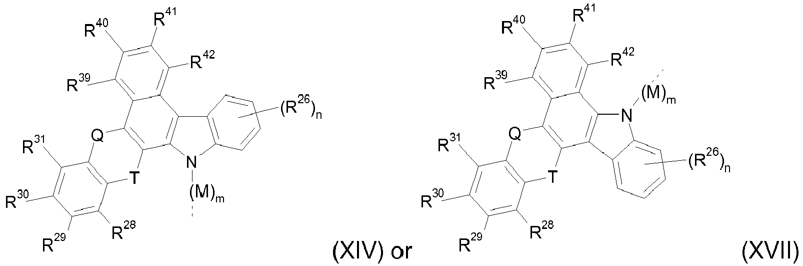 wherein
R³⁹, R⁴⁰, R⁴¹, R⁴² are independently of each other selected from H, a C₆-C₁₈aryl group which is unsubstituted or substituted by at least one C₆-C₁₂aryl or at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, more preferably R³⁹, R⁴⁰, R⁴¹ and R⁴² are H, wherein the dotted line is a bonding site.

7. The compound according to claim 5, wherein the heterocyclic group according to the general formula (XXI) is represented by any one of the general formulae (XXIa), (XXIb) or (XXIc)

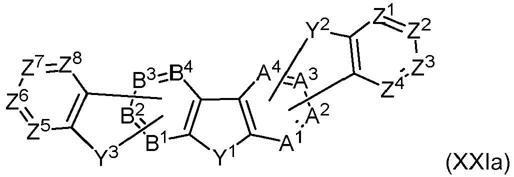

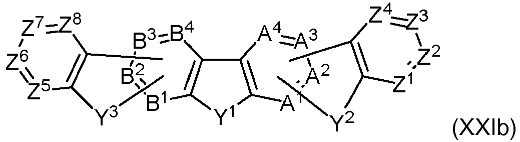

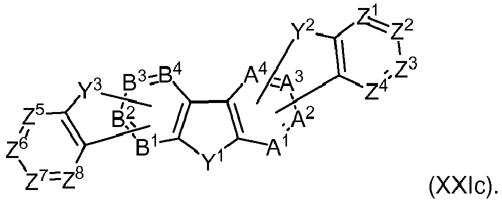

8. The compound according to claim 5 or 7 wherein at least one of Y¹, Y² and Y³ is NR⁷⁰, NR⁷³ or NR⁸⁰ respectively.

9. The compound according to claim 8, wherein two of Y¹, Y² and Y³ are NR⁷⁰, NR⁷³ or NR⁸⁰ respectively.

10. The compound according to any one of claims 1 to 5, 7, 8 and 9 wherein the substituent according to general formula (XXI) is connected to the compound according to general formula (I) via R⁷⁰, R⁷³ or R⁸⁰, wherein this respective residue R⁷⁰, R⁷³ or R⁸⁰ is a direct bond, optionally interrupted by a group of formula -(M)ₘ-,
wherein in this case,
m is 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

11. The compound according to claim 5, wherein the N-heteroaryl group according to general formula (XII) is represented by a group according to the general formula (XX) 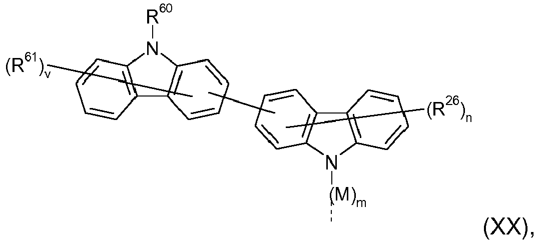 wherein
m, M, R²⁶ have the same meanings as defined in claim 5,
n is 0 to 7, preferably 0, 1, 2 or 3, more preferably 0 or 1, most preferably 0,
v is 0 to 7, preferably 0, 1, 2 or 3, more preferably 0 or 1,
R⁶⁰ is independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E,
R⁶¹ is independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E,
or at least two of R⁶¹, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused, and
the dotted line is a bonding site.

12. The compound according to any one of claims 1 to 6, wherein
formulae (XIII), (XIV). (XV), (XVI) and (XVII) are represented by

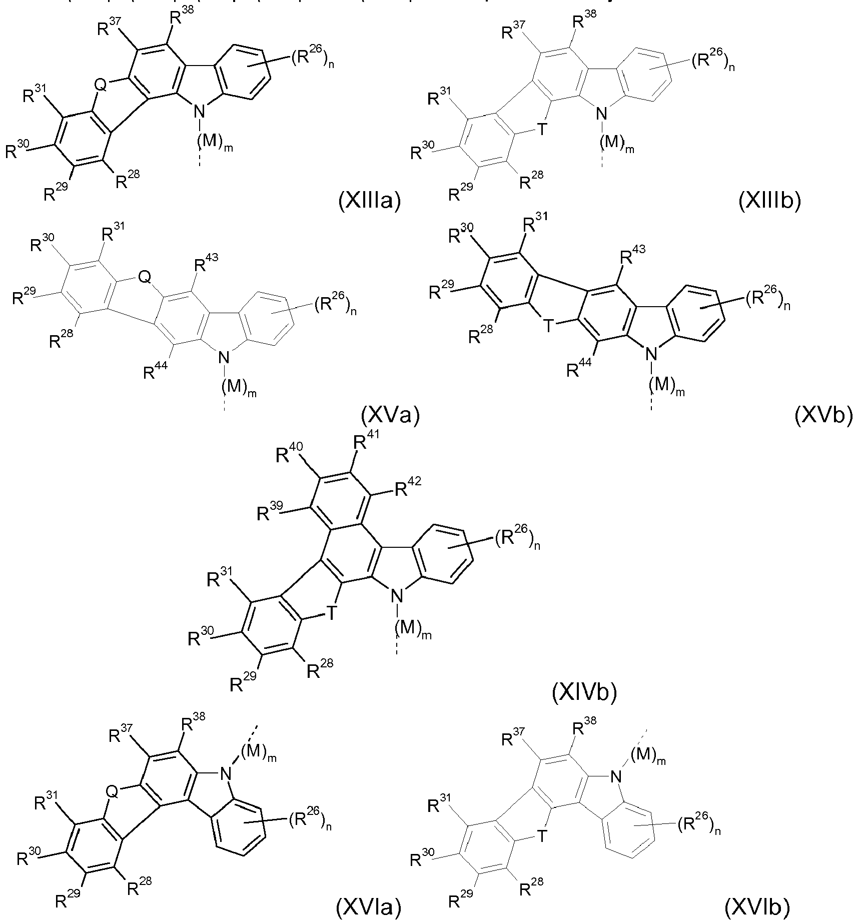

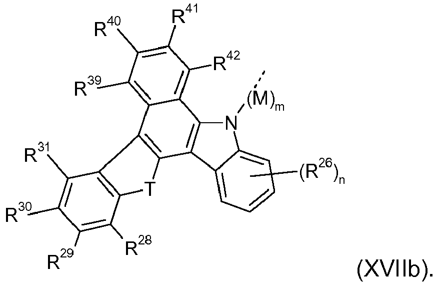

13. The compound according to any one of claims 1 to 12, wherein R⁴, R⁵, R⁶, R⁷, R⁸, R⁹ and R¹⁰ are H and R² is a N-heteroaryl group according to one of the formulae (XII), (XIII), (Xllla), (XIIIb), (XIV), (XIVb), (XV), (XVa), (XVb), (XVI), (XVIa), (XVIb), (XVII), (XVIIb), (XX), (XXI), (XXIa), (XXIb) or (XXIc).

14. A process for the preparation of a compound according to general formula (I) as defined in any of claims 1 to 13, at least comprising step (A)
(A) coupling of a compound according to general formula (VI') 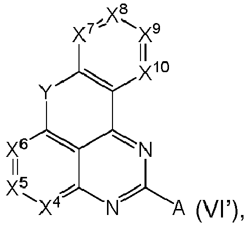 with a compound of formula R²-H to obtain a compound according to general formula (I), wherein A is a selected from Cl, Br, I, F, OSO₂CH₃, and OSO₂CF₃ or OSO₂C₆H₄CH₃.

15. An electronic device comprising at least one compound as defined in any of claims 1 to 13.

16. The electronic device, preferably an organic electroluminescence device, more preferably an organic light emitting diode (OLED), according to claim 15, comprising a cathode, an anode, and a plurality of organic thin film layers provided between the cathode and the anode, the organic thin film layers comprising an emitting layer comprising the at least one compound of general formula (I), preferably as a host material, a charge transporting material, charge and/or exciton blocking material, particularly preferably as a host material or an electron transporting material.

17. The electronic device, preferably an organic electroluminescence device, more preferably an organic light emitting diode (OLED), according to claim 15 or 16, wherein the emitting layer comprises a phosphorescent material, which is an ortho-metallated complex comprising a metal atom selected from iridium (Ir), osmium (Os) and platinum (Pt).

18. An electronic equipment comprising the organic electroluminescence device according to claim 16 or 17.

19. An emitting layer, preferably present in an electronic device, more preferably in an electroluminescence device, particularly preferably in an organic light emitting diode (OLED), comprising at least one compound of general formula (I) as defined in any of claims 1 to 13.

20. Use of a compound according to general formula (I) as defined in any of claims 1 to 13 in an electronic device, preferably in an electroluminescence device, particularly preferably in an organic light emitting diode (OLED), preferably in an emitting layer, as a host material, a charge transporting material, charge and/or exciton blocking material, preferably as a host material or an electron transporting material.

21. A material for an organic electroluminescence device comprising at least one compound according to any one of claims 1 to 13.
